(19) **Europäisches Patentamt**
**European Patent Office**
**Office européen des brevets**

(11) **EP 3 341 062 B1**

(12) **EUROPEAN PATENT SPECIFICATION**

(45) Date of publication and mention
of the grant of the patent:
**04.11.2020 Bulletin 2020/45**

(51) Int Cl.:
*A61M 16/00* *(2006.01)*     *A61M 16/08* *(2006.01)*
*A61M 16/10* *(2006.01)*     *A61M 16/12* *(2006.01)*

(21) Application number: **17742120.3**

(22) Date of filing: **23.01.2017**

(86) International application number:
**PCT/US2017/014597**

(87) International publication number:
**WO 2017/127823 (27.07.2017 Gazette 2017/30)**

(54) **MODULAR VENTILATION SYSTEM**

MODULARES BELÜFTUNGSSYSTEM

SYSTÈME DE VENTILATION MODULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.01.2016 US 201662281415 P**
**20.01.2017 US 201715411665**

(43) Date of publication of application:
**04.07.2018 Bulletin 2018/27**

(60) Divisional application:
**20182231.9**

(73) Proprietor: **Breathe Technologies, Inc.**
**Irvine, CA 92618 (US)**

(72) Inventors:
• **AHMAD, Samir S.**
**Irvine, California 92618 (US)**
• **BRAMBILLA, Enrico**
**Irvine, California 92618 (US)**

• **NIKKHAH, Ali**
**Irvine, California 92618 (US)**
• **MASTROVICH, Lawrence A.**
**Irvine, California 92618 (US)**
• **BERMAN, Gary**
**Irvine, California 92618 (US)**
• **VAHIDI, Masoud**
**Irvine, California 92618 (US)**

(74) Representative: **Pallini Gervasi, Diego et al**
**Notarbartolo & Gervasi GmbH**
**Bavariaring 21**
**80336 Munich (DE)**

(56) References cited:
WO-A1-2006/136878     CN-A- 105 169 537
US-A- 5 335 651       US-A- 6 095 138
US-A- 6 095 138       US-A- 6 158 430
US-A1- 2002 053 286   US-A1- 2002 053 286
US-A1- 2005 051 168   US-A1- 2008 202 508
US-A1- 2012 298 099   US-A1- 2013 206 140
US-A1- 2014 137 737   US-A1- 2014 373 842

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND

1. Technical Field

The present disclosure relates generally to ventilators and, more particularly, to a modular ventilation system which is adapted to provide continuous or intermittent ventilatory support for the care of individuals who require mechanical ventilation.

2. Related Art

A wide range of clinical conditions may require some form of ventilation therapy. These conditions may include hypoxemia, various forms of respiratory insufficiency, and airway disorders. There are also non-respiratory and non-airway diseases that require ventilation therapy, such as congestive heart failure and neuromuscular diseases.

The Chinese Patent No. CN 105 169 537 A discloses a breathing machine and a using method thereof. The breathing machine comprises a breathing machine mainframe and a breathing machine running module. The breathing machine mainframe is detachably connected with the breathing machine running module and provided with a first air supply interface. The breathing machine running module is provided with a second air supply interface, a third air supply interface and a patient breathing interface which are correspondingly matched with the first air supply interface. An ICU treatment breathing machine and a first-aid transfer breathing machine are integrated.

The United States Patent No. US 5 335 651 A1 discloses A hospital bed supported on a wheeled base, and a ventilator supported on a wheeled cart and docked to the base of the bed, the combination of ventilator and bed capable of being rolled as a single unit. The ventilator cart includes a wheeled base, and supports connected to the base for supporting a ventilator, with the supports providing for selective raising and lowering of the ventilator.

The United States Patent No. US 6 158 430 A discloses a ventilator system that has at least one ventilator unit and a number of docking stations. Each docking station is adapted to receive the ventilator unit and comprises a communication interface adapted to be connected to a matching interface on the ventilator unit when the ventilator unit is docked in the docking station.

The US Patent No. US 6 095 138 A discloses a portable respiration apparatus which is dimensioned to convey a flow of gas, with an upper limit on pressure, from an inlet to an outlet connected to a patient. The inlet of the portable respiration apparatus can be connected to a stationary gas unit when a higher pressure is desired. The stationary gas unit can generate a gas pressure that is applied to the inlet of the portable respiration apparatus, so that a higher gas pressure can be generated and delivered to the patient. To improve the quality of life of many patients who require long-term ventilation therapy, various types of ventilation systems have been developed in the prior art. Some of these prior art systems are compact, lightweight and portable, whereas others are substantially more robust and not well suited for portability or to be wearable by the patient. However, the known prior art is deficient in providing a ventilation system which is uniquely adapted for use in any one of several different configurations, and to provide any one of several different types of ventilatory support corresponding to those configurations. These deficiencies are addressed by the modular ventilation system of the present disclosure, as described in more detail below.

BRIEF SUMMARY

To solve these and other problems, a novel modular ventilation system capable of transitioning between a stationary configuration, an extended range configuration, and a stand-alone configuration, and methods of use thereof for providing continuous or intermittent ventilatory support for the care of individuals who require mechanical ventilation, is contemplated. According to the invention, there is provided a ventilatory support apparatus according to claim 1, and a method according to claim 12. The dependent claims define preferred embodiments of the invention.

BRIEF DESCRIPTION OF THE DRAWINGS

These and other features and advantages of the various embodiments disclosed herein are better understood with respect to the following descriptions and drawings, in which:

FIG. 1 is a front perspective view of a ventilatory support apparatus in accordance with an embodiment of the disclosed modular ventilation system;
FIG. 2 is a front elevational view of a ventilator of the ventilatory support apparatus;
FIG. 3 is a side elevational view of the ventilator;

FIG. 4 is a top plan view of the ventilator;

FIG. 5 is a bottom plan view of the ventilator;

FIG. 6 is a front perspective view of a compressor unit of the ventilatory support apparatus;

FIG. 7 is a rear perspective view of the compressor unit;

FIG. 8 is a schematic view of the ventilatory support apparatus in the stationary configuration;

FIGS. 9A-9D are front elevational views (FIGS. 9A and 9D) and enlarged cut-away front perspective views (FIGS. 9B and 9C) of the ventilatory support apparatus transitioning to the stationary configuration in an exemplary process having four steps;

FIG. 10 is a front perspective view of the ventilatory support apparatus in the stationary configuration, including a patient interface;

FIG. 11 is an enlarged cut-away rear perspective view of the ventilatory support apparatus in the stationary configuration, along with oxygen connecting tubing;

FIG. 12 is a schematic view of the ventilatory support apparatus in the extended range configuration;

FIGS. 13A and 13B are a front elevational view and an enlarged cut-away front perspective view, respectively, of the ventilatory support apparatus transitioning from the stationary configuration by an exemplary process having two steps;

FIG. 14 is a schematic view of the connection of the ventilator to the compressor unit via the compressed gas hose in the extended range configuration;

FIG. 15 is a cut-away front perspective view of the ventilator, showing the bottom of the ventilator, including the patient interface;

FIG. 16 is a schematic view of the connection of the ventilator to a ventilator battery charger via a ventilator battery charger cord;

FIGS. 17A and 17B are perspective views showing the ventilator being attached to a belt clip in an exemplary process having two steps;

FIG. 18 is a schematic view of the ventilatory support apparatus in the stand-alone configuration;

FIGS. 19A-19C are perspective views showing the ventilator being secured to a pole via the belt clip and a pole mount in an exemplary process having three steps; and

FIGS. 20A-20C are perspective views (FIGS. 20A and 20B) and a top plan view (FIG. 20C) of the ventilatory support apparatus transitioning to the stand-alone configuration by connection of the ventilator to an external compressed gas source in an exemplary process having three steps. Fig 21 - 103 are schematic views of exemplary embodiments according to the example of the present invention.

Common reference numerals are used throughout the drawings and the detailed description to indicate the same elements.

DETAILED DESCRIPTION

According to various aspects of the present disclosure, a modular ventilatory support system which may be transitioned between a stationary, an extended range, and a stand-alone configuration, and methods of performing such transitions are contemplated. The modular components of the modular ventilatory support system are contemplated to include at least a compressor unit, a ventilator which may dock with the compressor unit, and a patient interface which may be connected to either the compressor unit or the ventilator unit. Due to their modular nature, these components may be rearranged into at least three different configurations, with such configurations having differing attributes relating to the mobility and potential duration of use. As such, it may be seen that through use of the modular ventilatory support systems and methods contemplated herein, a user may select the most appropriate configuration for the modular ventilatory support system to fit their present needs at any given time, and then may transition the modular ventilatory support system to that configuration, achieving flexibility benefits without requiring the use of multiple different ventilatory support systems. For example, when transitioned to the stationary configuration, mobility is restrictive, but duration of use is maximized. When transitioned to the extended range configuration, mobility is enhanced, with duration of use limited by the battery power of the ventilator. When transitioned to the stand-alone configuration, mobility is maximized, with duration of use limited by the battery power of the ventilator and the quantity of an external gas supply. As such, it may be seen that substantial improvements in a patient's quality of life may be achieved through the presently disclosed modular ventilation system.

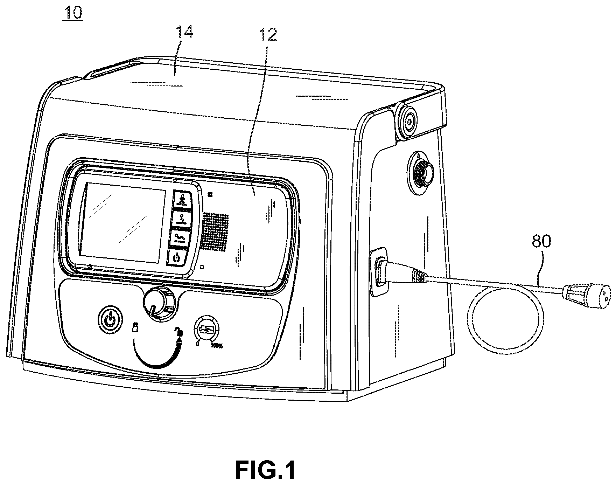

Turning now to FIG. 1, a front perspective view of a ventilatory support apparatus 10 in accordance with an embodiment of the disclosed modular ventilation system is shown. The ventilatory support apparatus 10 may be, for example, in the exemplary embodiment, one or more components of the Breathe Technologies Life2000TM Ventilation System described in the Example on pages 21-130 of this specification. However, it may be seen that the ventilatory support apparatus 10 may be any ventilation system having the herein described components and/or operating according to the herein disclosed methods. In the exemplary embodiment, the ventilatory support apparatus 10 is transitionable between a stationary configuration, an extended range configuration, and a stand-alone configuration. The ventilatory support apparatus 10 includes at least a ventilator 12, a compressor unit 14, and a patient interface 80. In the configuration shown in FIG. 1, i.e. the stationary configuration, the ventilator 12, the compressor unit 14, and the patient interface 80 are used together. However, in other contemplated configurations to which the ventilation system may be transitioned, as described below, the ventilator 12 and the patient interface 80 of the ventilatory support apparatus 10 may be used independently from the compressor unit 14.

The ventilator 12 operates to provide ventilation gas and may be, in the exemplary embodiment, the Breathe Technologies Life2000TM Ventilator described in the Example, which can be used with the Breathe Technologies Life2000TM Compressor also described in the Example, or with an external compressed gas source, which may be, in some embodiments, a 50-PSI pressure source. The ventilation gas may be any gas breathable by a patient using the ventilatory support apparatus 10, e.g. oxygen or air.

The ventilator 12 may operate according to known methods of receiving compressed gas, generating ventilation gas, and providing that ventilation gas to a patient having need of medical ventilation. However, it is contemplated that, in the exemplary embodiment, the ventilator 12 may be configured and/or operate according to certain known configurations of ventilators and/or methods of regulating and delivering ventilation gas to provide therapeutic respiratory support, which may include, for example, the methods disclosed in Applicant's U.S. Patent No. 7,533,670 entitled SYSTEMS, METHODS, AND APPARATUS FOR RESPIRATORY SUPPORT OF A PATIENT,; Applicant's U.S. Patent No. 8,381,729 entitled METHODS AND DEVICES FOR MINIMALLY INVASIVE RESPIRATORY SUPPORT,; Applicant's U.S. Patent No. 8,418,694 entitled SYSTEMS, METHODS, AND APPARATUS FOR RESPIRATORY SUPPORT OF A PATIENT; Applicant's U.S. Patent No. 8,567,399 entitled METHODS AND DEVICES FOR PROVIDING INSPIRATORY AND EXPIRATORY FLOW RELIEF DURING VENTILATION THERAPY,; Applicant's U.S. Patent No. 8,770,193 entitled METHODS AND DEVICES FOR CONTROLLING VENTILATOR FUNCTIONS,; Applicant's U.S. Patent No. 8,776,793 entitled METHODS AND DEVICES FOR SENSING RESPIRATION AND CONTROLLING VENTILATOR FUNCTIONS,; Applicant's U.S. Patent No. 8,895,108 entitled MECHANICAL VENTILATION MASK FIT STATUS INDICATION,; Applicant's U.S. Patent No. 9,399,109 entitled CONTINUOUS POSITIVE AIRWAY PRESSURE (CPAP) THERAPY USING MEASUREMENTS OF SPEED AND PRESSURE,; Applicant's co-pending U.S. Application Ser. No. 13/524,983 (corresponding to U.S. Patent Application Pub. No. 2013/0333702) entitled METHOD AND SYSTEM FOR OPERATING A PATIENT VENTILATION DEVICE,; Applicant's co-pending U.S. Application Ser. No. 13/566,902 (corresponding to U.S. Patent Application Pub. No. 2014/0034055) entitled SELECTIVE RAMPING OF THERAPEUTIC PRESSURE IN A PATIENT BREATHING APPARATUS,; Applicant's co-pending U.S. Application Ser. No. 13/841,189 (corresponding to U.S. Patent Application Pub. No. 2014/0261426) entitled DUAL PRESSURE SENSOR PATIENT VENTILATOR,; Applicant's co-pending U.S. Application Ser. No. 13/849,443 (corresponding to U.S. Patent Application Pub. No. 2014/0283834) entitled PORTABLE VENTILATOR SECRETION MANAGEMENT SYSTEM,; Applicant's co-pending U.S. Application Ser. No. 13/927,016 (corresponding to U.S. Patent Application Pub. No. 2014/0373842) entitled VENTILATOR WITH INTEGRATED COOLING SYSTEM,; Applicant's co-pending U.S. Application Ser. No. 13/935,362 (corresponding to U.S. Patent Application Pub. No. 2015/0011905) entitled RESPIRATORY CYCLE PATIENT VENTILATION FLOW LIMITATION DETECTION,; Applicant's co-pending U.S. Application Ser. No. 14/020,729 (corresponding to U.S. Patent Application Pub. No. 2015/0073291) entitled APNEA AND HYPOPNEA DETECTION,; Applicant's co-pending U.S. Application Ser. No. 14/104,842 (corresponding to U.S. Patent Application Pub. No. 2015/0165143) entitled CONTINUOUS POSITIVE AIRWAY PRESSURE THERAPY AUTO-TITRATION,; Applicant's co-pending U.S. Application Ser. No. 14/181,431 (corresponding to U.S. Patent Application Pub. No. 2015/0231349) entitled SLEEP DETECTION FOR CONTROLLING CONTINUOUS POSITIVE AIRWAY PRESSURE THERAPY,; and Applicant's co-pending U.S. Application Ser. No. 14/181,435 (corresponding to U.S. Patent Application Pub. No. 2015/0231350) entitled DETECTION OF PATIENT INTERFACE DISCONNECT FOR CONTROLLING CONTINUOUS POSITIVE AIRWAY PRESURE THERAPY,; Applicant's co-pending U.S. Application Ser. No. 14/482,444 (corresponding to U.S. Patent Application Pub. No. 2015/0068528) entitled CONTINUOUS POSITIVE AIRWAY PRESSURE THERAPY TARGET PRESSURE COMFORT SIGNATURE,; and Applicant's co-pending U.S. Application Ser. No. 14/482,445 (corresponding to U.S. Patent Application Pub. No. 2015/0068529) entitled ZERO PRESSURE START CONTINUOUS POSITIVE AIRWAY PRESSURE THERAPY,.

The compressor unit 14 may be, in the exemplary embodiment, the Breathe Technologies Life2000TM Compressor described in the Example, which is an electropneumatic power unit that may provide the ventilator 12 with a continuous source of compressed gas and additionally may serve as a charging station for the ventilator 12. It is contemplated that the ventilator 12 and the compressor unit 14 may be configured such that the ventilator 12 may be docked with the compressor unit 14, e.g. by insertion of the ventilator 12 into the compressor unit 14 as shown in FIG. 1. However, it may be seen that the ventilator 12 may be docked with the compressor unit 14 in other fashions other than insertions, and the exact method of docking is not critical, so long as the docking establishes the necessary fluid connections between the ventilator 12 and the compressor unit 14, with such fluid connections being discussed in detail further below.

However, those of ordinary skill in the art will recognize that assuming the use of a docking modality involving the insertion of the ventilator 12 into the compressor unit 14, it is contemplated that the shape or form factor of the receptacle or other opening in the compressor unit 14 which accommodates the ventilator 12 will be complimentary to that of the ventilator 12 itself as shown in FIG. 1, thus providing both the functional and visual effects of a smooth, somewhat seamless integration between these two structural features.

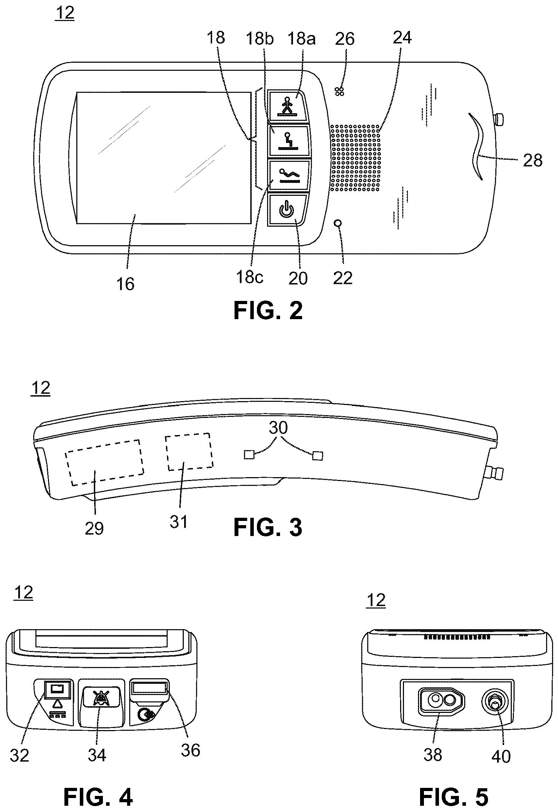

Turning now to FIG. 2, a front elevational view of a ventilator 12 according to the exemplary embodiment is shown. As shown in the example of FIG. 2, the ventilator 12 may include a user interface 18 comprising, for example, a display 16, a ventilator power button 20, a ventilator power indicator light 22, an alarm speaker 24, a backup alarm speaker 26, and a breath indicator light 28. The user interface 18 may include prescription settings buttons, for example, a high activity button 18a, a medium activity button 18b, and a low activity button 18c, and may further include other buttons, dials, sliders, switches, etc. The display 16 may be a touch screen, in which case the user interface 18 may further include a touch screen functionality of the display 16. It may therefore be seen that the user interface 18 of the ventilator may be configured to receive a user input.

Turning now to FIG. 3, a side elevational view of the exemplary embodiment of a ventilator 12 is shown. As shown in the embodiment illustrated in FIG. 3, the ventilator 12 may further include other aspects such as belt clip sockets 30 for attachment to a belt clip or other attachment features, in order to support attachment of the ventilator 12 to persons or objects when not docked with the compressor unit 14. The belt clip sockets 30 may be included on both sides of the ventilator 12 (only one side shown in FIG. 3). The ventilator may also include internally a rechargeable battery 29 and a wireless transmitter 31.

Turning now to FIG. 4, a top plan view of the exemplary embodiment of a ventilator 12 is shown. As shown in the example of FIG. 4, the ventilator 12 may further include a ventilator battery charger connection port 32, a ventilator-side silence alarm button 34, and an additional port 36. The ventilator-side silence alarm button 34 may be used to silence alarms (e.g. as described in the Example). The additional port 36 may be used by the manufacturer to interface with the ventilator 12, for example, to send and receive data such as firmware updates, predefined operative modes, or error logs. In the exemplary embodiment, the additional port 36 is a USB port. However, in other embodiments, it may be seen that the additional port may be any port known or future developed in the art for interfacing between devices, or may be omitted entirely.

Turning now to FIG. 5, a bottom plan view of the exemplary embodiment of a ventilator 12 is shown. As shown in the example of FIG. 5, the ventilator 12 may further include a ventilator ventilation gas output port 38 and a ventilator compressed gas inlet port 40. The ventilator ventilation gas output port 38 may, in the exemplary embodiment, be configured to accept a multi-lumen patient interface gas inlet port 81 of a patient interface 80, such as, for example, the various adapters described in Applicant's co-pending U.S. Application Ser. No. 14/020,032 (corresponding to U.S. Patent Application Pub. No. 2015/0068519) entitled JET PUMP ADAPTOR FOR VENTILATION SYSTEM,. Furthermore, the patient interfaces may be, for example, those interfaces described in, for example, Applicant's U.S. Patent Nos. 8,839,791; 8,844,533; 9,038,634; 9,038,635; 9,132,250; 9,180,270; 9,227,034; and 9,327,092,. However, it may be seen that, in other embodiments, the ventilator ventilation gas output port 38 may be configured in other configurations so as to accept or otherwise fluidly connect to a patient interface gas inlet port 81 of a patient interface 80 for delivering breathing gas to a patient, with possible patient interface 80 including but not being limited to nasal interfaces, nasal masks, respiratory masks, or-nasal masks, or intubation devices.

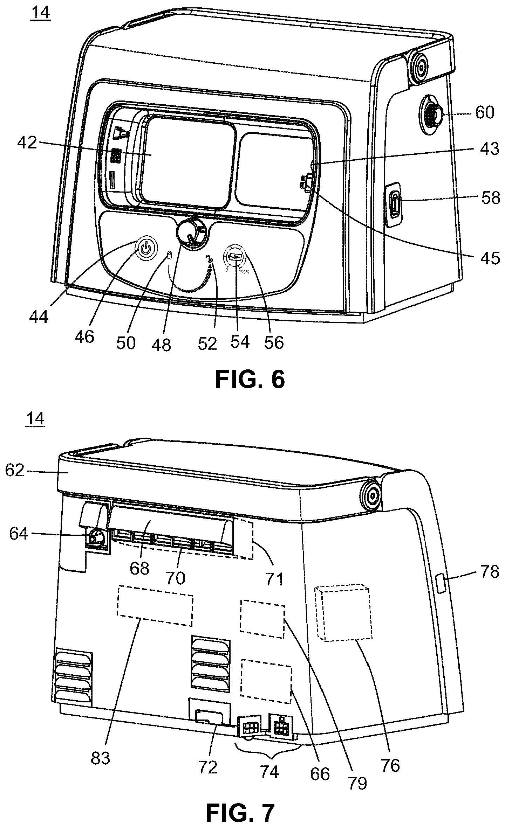

Turning now to FIG. 6, a front perspective view of the compressor unit 14 of the exemplary embodiment of the ventilatory support apparatus 10 is shown. As shown in the example of FIG. 6, the compressor unit 14 may include a ventilator dock 42, a compressor power source indicator light 44, a compressor power button 46, a locking knob 48, a locked icon 50, an unlocked icon 52, a battery charge status button 54, a battery charge indicator 56, a compressor unit compressed gas output port 58, and a compressor unit ventilation gas output port 60. The ventilator dock 42 may have a ventilator dock compressed gas output port 43 and a ventilator dock ventilation gas inlet port 45 and may further be configured to provide electrical power for powering the ventilator 12 and for recharging the rechargeable battery 29 of the ventilator 12 when the ventilator 12 is docked at the ventilator dock 42. The ventilator dock compressed gas output port 43 may be configured to interface with the ventilator compressed gas inlet port 40 when the ventilator 12 is docked at the docking port 42 so as to form a generally sealed fluid connection between the two ports. Likewise, the ventilator dock ventilation gas inlet port 45 may be configured to interface with the ventilator ventilation gas output port 38 when the ventilator 12 is docked at the docking port 42 so as to form a generally sealed fluid connection between the two ports. Further, it may be seen that the docking port 42 may additionally include a power conduit for interfacing with the ventilator battery charger connection port 32 of the ventilator 12 so as to provide electrical power to the ventilator 12 when docked at the docking port 42 for powering the ventilator 12 and for charging the rechargeable battery 29. The power conduit may, in certain embodiments, be configured to be spring-loaded or otherwise movable to permit retraction and subsequent insertion or other form of connection to ventilator battery charger connection port 32, such as when the locking knob 48 is actuated, as it may be seen that doing so may more easily permit the insertion of the ventilator 12 into the docking port 42. Those of ordinary skill in the art will recognize that the complimentary, generally quadrangular (i.e., rectangular) configurations of the ventilator 12 as shown in FIGS. 2-5, and it corresponding docking port 42 within the compressor unit 14 are exemplary only, and may be substituted with alternative complementary shapes.

The locked icon 46 may additionally function as a ventilator charging indicator light. The compressor unit compressed gas output port 58 may, in the exemplary embodiment, be a Diameter Index Safety System (DISS) coupling and may be, e.g., a DISS 1240 output connection port. However, it may be seen that in other embodiments, the compressed gas output port 58 may be any port that is suitable for outputting compressed gas.

Turning now to FIG. 7, a rear perspective view of the exemplary embodiment of the compressor unit 14 is shown. In the exemplary embodiment, the compressor unit 14 further includes a handle 62, a low flow gas (e.g. oxygen) input port 64, internal battery 66, an ambient air filter cover 68, one or more ambient air apertures 70, a water tray 72, a power supply connection port 74, an alarm speaker 76 (internal), a compressor-side silence alarm button 78, and a wireless receiver 79 (internal), as well as a compressor 83 (internal) for providing compressed gas. The handle 62 may be arranged to ensure that the compressor unit 14 remains in an upright position when carried. The one or more ambient air apertures 70 may include an ambient air filter 71 for preventing particulate matter from entering into the compressor unit. The power supply connection port 74 may include a removable or displaceable cover. The compressor-side silence alarm button 78 may be used to silence alarms (e.g., as described in the Example). AC power may be supplied to the compressor unit 14 by connecting an external power supply to the power supply connection port 74 using an AC power cord (e.g., as described in the Example).

The compressor 83 may be configured to compress ambient air and/or low-pressure gas and to produce compressed gas for subsequently delivery to the ventilator 12. In the exemplary embodiment, the one or more ambient air apertures 70 may permit introduction of ambient air to the compressor 83 through the ambient air filter 71 to be pressurized by the compressor 83 (e.g., as described in the Example). It may also be seen, for example, that low-pressure gas may be introduced via low flow gas input port 64 to the compressor 83, and that such low-pressure gas may substitute for or supplement ambient air.

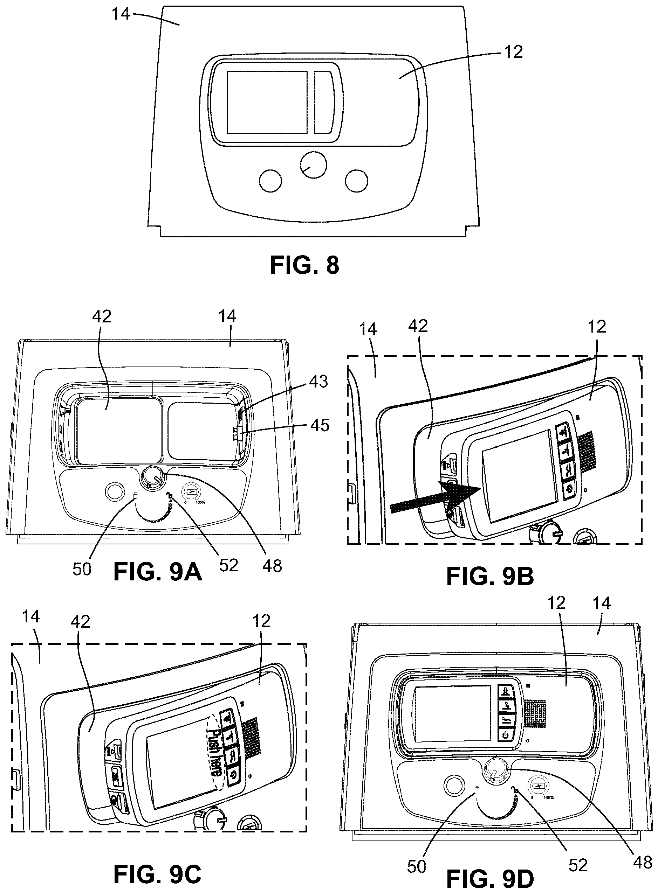

Turning now to FIG. 8, a schematic view of the ventilatory support apparatus 10 in the stationary configuration is shown. When the ventilatory support apparatus 10 is in the stationary configuration, the ventilator 12 is docked with the compressor unit 14 while the compressor unit 14 is preferably positioned upright on a flat, level surface.

Turning now to FIGS. 9A-9D, front elevational views (FIGS. 9A and 9D) and enlarged cut-away front perspective views (FIGS. 9B and 9C) of the ventilatory support apparatus 10 transitioning to the stationary configuration in the exemplary embodiment are shown in a process having four steps. However, it may be seen that in other embodiments, the process of docking the ventilator 12 at the docking port 42 may differ. According to the exemplary embodiment of the docking process, first, after it is ensured that the ventilator 12 is powered off (the ventilator 12 may be powered off using the ventilator power button 20), it is confirmed that the locking knob 48 on the compressor unit 14 is in the unlocked position as shown in FIG. 9A, e.g., with an appropriate indicator mark on the locking knob 48 facing the unlocked icon 52. Second, as shown in FIG. 9B, the ventilator 12 is positioned in the ventilator dock 42 of the compressor unit 14 with one end (e.g. the bottom end) inserted first as shown, and the ventilator 12 is pushed in the direction of the one end (e.g. the direction of the arrow in FIG. 9B) until the ventilator 12 clicks into place, indicating that the ventilator compressed gas inlet port 40 has been interfaced to form a generally sealed fluid connection with the ventilator dock compressed gas output port 43, and that the ventilator ventilation gas output port 38 has been interfaced to form a generally sealed fluid connection with the ventilator dock ventilation gas inlet port 45. Third, as shown in FIG. 9C, the center of the ventilator 12 is pushed in the direction of the compressor unit 14 (e.g., pushed at "Push here" in FIG. 9C) until the front of the ventilator 12 is flush with the front of the compressor unit 14 and the ventilator 12 clicks into place. Fourth, and finally, as shown in FIG. 9D, the locking knob 48 on the compressor unit 14 is turned to the locked position, e.g. with an appropriate indicator mark on the locking knob 48 facing the locked icon 50. In this way, the ventilator 12 may be docked with the compressor unit 14. As noted above, the locked icon 50 may function as a ventilator charging indicator light. Thus, the locked icon 50 may light up when the compressor unit 14 is powered on and the ventilator 12 is properly docked to indicate that the ventilator 12 is being charged by the compressor unit 14.

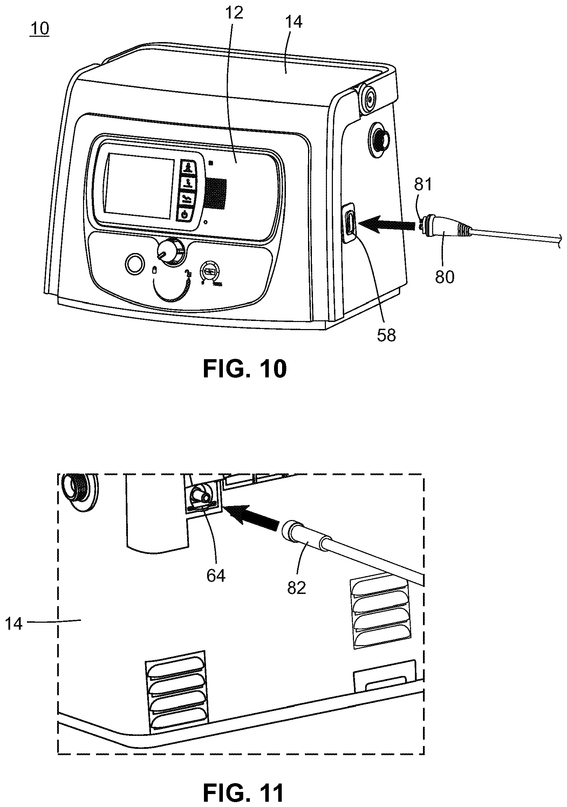

Turning now to FIG. 10, a front perspective view of the ventilatory support apparatus 10 in the stationary configuration, including the patient interface 80, is shown. The patient interface 80 is for receiving ventilation gas and delivering ventilation gas to a patient and may be any dedicated or universal interface used to connect a non-invasive mask (e.g., full face, nasal, pillows) or tracheostomy tube to the ventilatory support apparatus 10 or ventilator 12. The patient interface 80 may be, for example, in the exemplary embodiment, the Breathe Technologies Universal Circuit™ interface. The patient interface 80 has a patient interface gas inlet port 81 transitionable between placement in fluid communication with the compressor unit compressed gas output port 58 of the compressor unit 14 and placement in fluid communication with the ventilator ventilation gas output port 38 of the ventilator 12. As shown in FIG. 10, when the ventilatory support apparatus 10 is in the stationary configuration, the patient interface 80 may be plugged into the compressor unit compressed gas output port 58.

Turning now to FIG. 11, an enlarged cut-away rear perspective view of the ventilatory support apparatus 10 in the stationary configuration, along with low-flow gas tubing 82, is shown. When using a prescription setting that uses oxygen as the source gas, a low flow supplemental oxygen source (not shown), such as a stationary oxygen concentrator, may be connected to the compressor unit 14. As shown in FIG. 11, one end of the oxygen connecting tubing 82 may be attached to the low flow gas input port 64, e.g., by pushing and turning the oxygen connecting tubing 82 until it is completely and securely attached. The other end of the oxygen connecting tubing 82 may be connected to the low flow supplemental oxygen source, which may then be turned on.

With the ventilatory support apparatus 10 in the stationary configuration, the compressor unit 14 may be powered on by pressing the compressor power button 46, upon which the compressor power source indicator light 44 may illuminate to indicate the power source. For example, green illumination of the compressor power source indicator light 44 may indicate that the compressor is connected to AC power (e.g., by connection to an external power supply via the power supply connection port 74 and an AC power cord), while orange illumination of the compressor power source indicator light 44 may indicate that the compressor is using internal battery power as described below. According to the powering on sequence of the exemplary embodiment, the ventilator 12 is powered on by pressing the ventilator power button 20, upon which the ventilator power indicator light 22 may illuminate. After power on of the compressor unit 14 and ventilator 12, various tests (e.g., a test of the alarm speaker) may be performed and startup screens may be displayed on the display 16, with the display 16 eventually displaying a "Home" screen (e.g., as described in the Example).

The compressor unit 14 may include an internal battery 66 for alleviating temporary power disruptions. The internal battery 66 of the compressor unit 14 may charge when the compressor unit 14 is connected to AC power (e.g., by connection to an external power supply via the power supply connection port 74 and an AC power cord). The internal battery 66 of the compressor unit 14 may have a maximum charge, e.g., two hours. The battery charge indicator 56 may include a series of indicator lights arranged as a battery charge scale, e.g., surrounding the battery charge status button 54, the battery charge scale indicating the current battery charge level of the compressor unit 14 (e.g., as described in the Example). The battery charge status button 54 may be used, for example to illuminate the battery charge indicator 56 when the compressor unit 14 is powered off.

When the ventilatory support apparatus 10 is transitioned to the stationary configuration, the ventilator 12 is docked at the ventilator dock 42 of the compressor unit 14, the ventilator compressed gas inlet port 40 is in fluid communication with the ventilator dock compressed gas output port 43, the ventilator ventilation gas output port 38 is in fluid communication with the ventilator dock ventilation gas inlet port 45, and the patient interface gas inlet port 81 of the patient interface 80 is in fluid communication with the compressor unit ventilation gas output port 60, such that compressed gas is provided by the compressor unit 14 to the ventilator 12 and ventilation gas is returned to the compressor unit 14 for subsequent output from the compressor unit 14 to the patient interface 80, e.g., via the compressor unit ventilation gas output port 60.

With the ventilatory support apparatus 10 in the stationary configuration, the compressor unit 14 may be powered off by pressing the compressor power button 46. According to the preferred powering off sequence, the ventilator 12 may then be powered off by using the ventilator power button 20, e.g., by pressing the ventilator power button 20 for three seconds and confirming power off using the display 16 (e.g., as described in the Example).

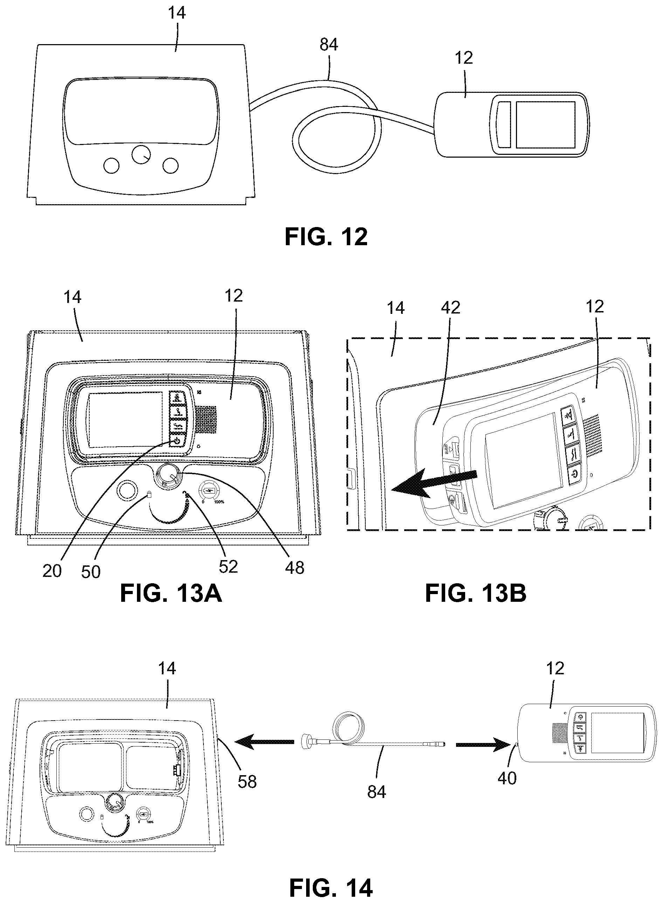

Turning now to FIG. 12, a schematic view of the ventilatory support apparatus 10 in the extended range configuration is shown. As noted above, the ventilatory support apparatus 10 may be used in different configurations of operation as the patient's needs change. In the extended range configuration, the ventilator 12 is connected to the compressor unit 14 with a compressed gas hose 84 to enable the activities of daily living. The compressed gas hose 84 may be, for example, a high-pressure hose of any length, e.g., six feet or fifty feet. In the exemplary embodiment, the compressed gas hose 84 connects to the compressor unit compressed gas output port 58 of the compressor unit 14 and to the ventilator compressed gas inlet port 40 of the ventilator 12 via DISS fittings. However, it may be seen that in other embodiments of a ventilatory support apparatus 10 in the extended range configuration, the compressed gas hose 84 may connect the compressor unit compressed gas output port 58 to the ventilator compressed gas inlet port 40 according to any known or future developed method.

Turning now to FIGS. 13A and 13B, a front elevational view and an enlarged cut-away front perspective view of the ventilatory support apparatus 10 transitioning away from the stationary configuration according to the exemplary embodiment is shown in two steps. First, after it is ensured that the ventilator 12 is powered off (the ventilator 12 may be powered off using the ventilator power button 20), the locking knob 48 on the compressor unit 14 is turned to the unlocked position as shown in FIG. 13A, e.g., with an appropriate indicator mark on the locking knob 48 facing the unlocked icon 52, at which point the compressor unit 14 will eject the ventilator 12. Second, and finally, as shown in FIG. 13B, the ventilator 12 is pulled in a direction to remove the remaining inserted end of the ventilator 12 (e.g., the bottom end) from the ventilator dock 42 of the compressor unit 14 (e.g., pulled in the direction of the arrow in FIG. 13B) until the ventilator 12 is separated from the ventilator dock 42 of the compressor unit 14. In this way, the ventilator 12 may be undocked from the compressor unit 14.

Turning now to FIG. 14, a schematic view of the connection of the ventilator 12 to the compressor unit 14 via the compressed gas hose 84 in the extended range configuration according to the exemplary embodiment is shown. Connecting the ventilator 12 to the compressor unit 14 via the compressed gas hose 84 allows use of the ventilator 12 without the ventilator 12 having to be docked with the compressor unit 14. According to the preferred connecting sequence, it is first ensured that the ventilator 12 is powered off, whereas the compressor unit 14 may be powered off or powered on. The ventilator 12 is then undocked from the compressor unit 14, e.g., by the example method described with respect to FIG. 13. The compressed gas hose 84 is then attached to the compressor unit compressed gas output port 58. For example, in the exemplary embodiment where the compressor unit compressed gas output port 58 is a DISS 1240 output connection port, the compressed gas hose may be connected to the compressor unit compressed gas output port 58 via a DISS connection on the compressed gas hose 84. Lastly, the other end of the compressed gas hose 84 is connected to the ventilator compressed gas inlet port 40 of the ventilator 12, e.g., by pushing a small quick connect end onto the ventilator compressed gas inlet port 40 until it clicks into place.

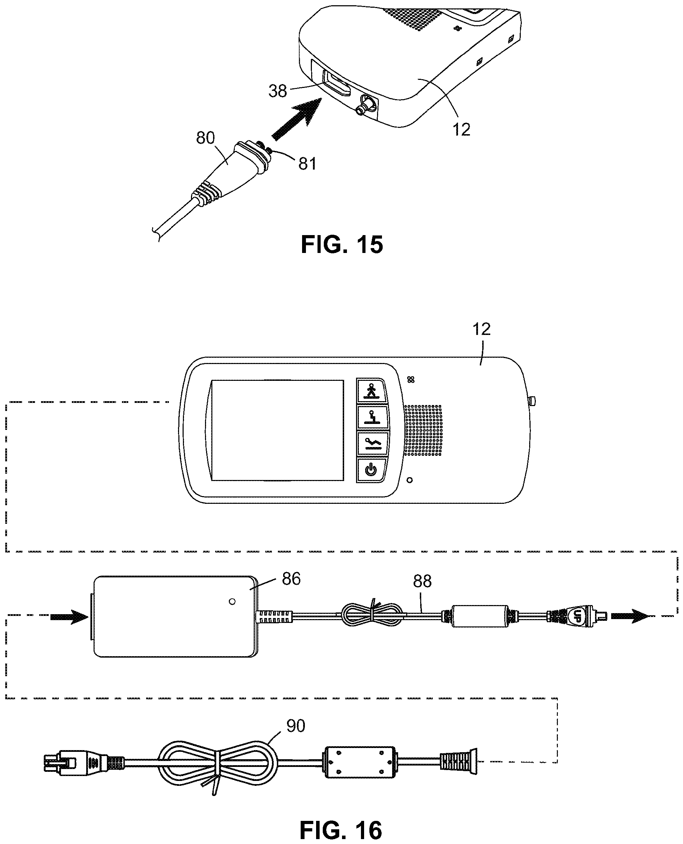

Turning now to FIG. 15, a cut-away front perspective view of the ventilator 12, showing the bottom of the ventilator 12, along with the patient interface 80, is shown. As shown in FIG. 15, in the extended range configuration, the patient interface 80 may be plugged into the ventilator ventilation gas output port 38.

In the exemplary embodiment, when the ventilatory support apparatus 10 is in the extended range configuration, the compressor unit 14 may be powered on by pressing the compressor power button 46, upon which the compressor power source indicator light 44 may illuminate to indicate the power source in the same way as when the ventilatory support apparatus 10 is in the stationary configuration. For example, green illumination of the compressor power source indicator light 44 may indicate that the compressor is connected to AC power (e.g., by connection to an external power supply via the power supply connection port 74 and an AC power cord), while orange illumination of the compressor power source indicator light 44 may indicate that the compressor is using internal battery power as described below. Continuing the exemplary powering on sequence, the ventilator 12 may then be powered on by pressing the ventilator power button 20 in the same way as when the ventilatory support apparatus 10 is in the stationary configuration, upon which the ventilator power indicator light 22 may illuminate. After power on of the compressor unit 14 and ventilator 12, various tests (e.g., a test of the alarm speaker) may be performed and startup screens may be displayed on the display 16, with the display 16 eventually displaying a "Home" screen (e.g. as described in the Example). As noted above, the compressor unit 14 may include an internal battery 66 for temporary power disruptions. The behavior of the internal battery 66 of the compressor unit 14, battery charge indicator 56, and battery charge status button 54 may be the same in the extended range configuration as in the stationary configuration. However, it may be seen that the compressor unit 14 may be powered on or off in alternative ways, such as, for example, by user input at the user interface 18 of the ventilator 12, wherein a wireless transmitter 31 of the ventilator 12 may communicate with the wireless receiver 79 of the compressor unit 14.

The ventilator 12 may also include a rechargeable battery 29 for use while undocked from the compressor unit 14, e.g., while the ventilatory support apparatus 10 is in the extended range configuration. The rechargeable battery 29 of the ventilator 12 may charge while the ventilator 12 is docked with the compressor unit 14, which may function as a charging station for the ventilator 12 as noted above. The rechargeable battery 29 of the ventilator 12 may also be charged in other ways, such as via a ventilator battery charger 86 connecting the ventilator battery charger connection port 32 to a power source such as a wall outlet or a generator. The rechargeable battery 29 of the ventilator 12 may have a maximum charge, e.g., four hours, and may take approximately three to four hours to fully recharge whether the ventilator 12 is off or on. When the ventilator 12 is powered on (e.g., via the ventilator power button 20, the ventilator power indicator light 22 being illuminated), a ventilator battery charge icon on the display 16 may show the current battery charge level of the rechargeable battery 29 of the ventilator 12 (e.g. as described in the Example).

Turning now to FIG. 16, a schematic view of the connection of the ventilator 12 to the ventilator battery charger 86 via the ventilator battery charger cord 88 is shown. The ventilator battery charger cord 88 may be integrally or removably connected to the ventilator battery charger 86. Connecting the ventilator 12 to the ventilator battery charger 86 allows charging of the internal battery of the ventilator 12 without the ventilator 12 having to be docked with the compressor unit 14. According to the preferred connecting sequence, a ventilator AC power cord 90 is first connected to the ventilator battery charger 86 and thereafter plugged into an AC power source. Then, the ventilator battery charger cord 88 (which is already connected to the ventilator battery charger 86) is connected to the ventilator battery charger connection port 32 of the ventilator 12 (e.g., as described in the Example).

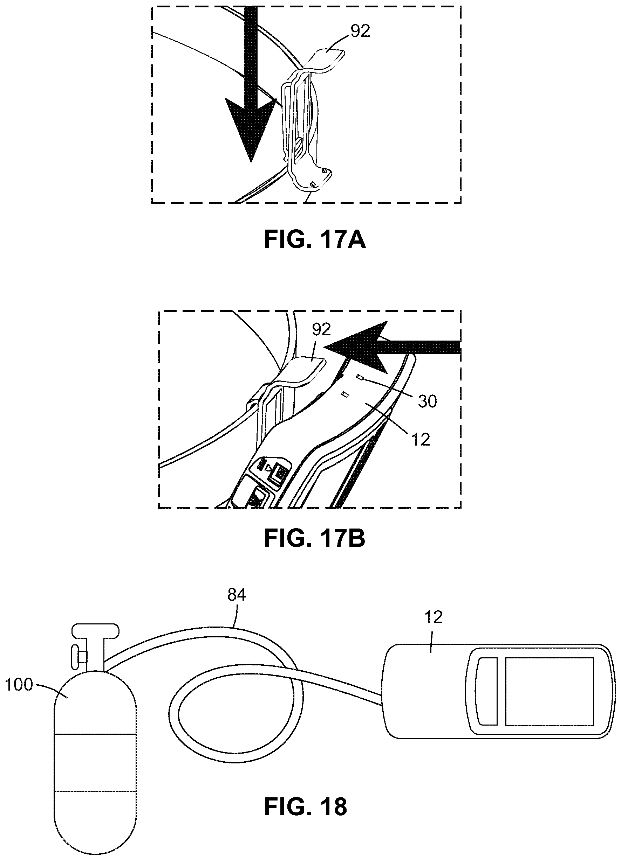

Turning now to FIGS. 17A and 17B, perspective views showing the ventilator 12 being attached to the belt clip 92 in an example process having the two steps is shown. The belt clip 92 may be used to secure the ventilator 12 so as to be wearable on a belt or waistband and may, for example, include protrusions corresponding to the belt clip sockets 30 on the ventilator 12. First, as shown in FIG. 17A, the belt clip 92 is securely fastened to a belt or waistband by positioning the belt clip 92 over the belt or waistband and pushing down (e.g. the direction of the arrow in FIG. 17A) until the belt clip 92 is secure. Second, and lastly, as shown in FIG. 17B, the belt clip 92 may be lined up with the belt clip sockets 30 on the ventilator 12, and the ventilator 12 may be pushed toward the belt clip 92 until a connection is made, e.g., until the protrusions on the belt clip 92 enter the belt clip sockets 30 on the ventilator 12, causing an audible click. In this way, the ventilator 12 may be wearable while the ventilatory support apparatus 10 is in the extended range configuration. Alternatively, the ventilator 12 may, in the extended range configuration, be mounted to a pole mount 94 as described below with respect to the stand-alone configuration. However, it may be seen that there may be a multitude of schemes for mounting or otherwise using the ventilator 12 in the configurations in which it is not docked with the compressor unit 14, and as such the specific illustrated methods should not be construed as limiting the scope of the present disclosure.

When the ventilatory support apparatus 10 is transitioned to the extended range configuration, the ventilator 12 is not docked at the ventilator dock 42, the ventilator compressed gas inlet port 40 is in fluid communication with the compressor unit compressed gas output port 58, and the patient interface gas inlet port 81 of the patient interface 80 is in fluid communication with the ventilator ventilation gas output port 38, such that compressed gas is provided by the compressor unit 14 to the ventilator 12 and ventilation gas is provided by the ventilator 12 to the patient interface 80 without being returned to the compressor unit 14.

With the ventilatory support apparatus 10 in the extended range configuration, the ventilator 12 of the exemplary embodiment may be powered off by pressing the ventilator power button 20, which in the exemplary embodiment may be performed by pressing the ventilator power button 20 for three seconds and confirming power off using the display 16 (e.g., as described in the Example). According to the exemplary powering off sequence, the compressor unit 14 may then be powered off by using the compressor power button 46. With the ventilator 12 powered off, the extended range configuration can be terminated by disconnecting the compressed gas hose 84 from the ventilator 12 and the compressor unit 14 (e.g., as described in the Example).

Turning now to FIG. 18, a schematic view of the ventilatory support apparatus 10 in the stand-alone configuration is shown. As noted above, the ventilatory support apparatus 10 may be used in different configurations of operation as the patient's needs change. In the exemplary embodiment of the stand-alone configuration, the ventilator 12 is connected via the compressed gas hose 84 to an external compressed gas source 100, e.g. an air or oxygen gas cylinder (50-PSI and/or ≤ 40 LPM at 41 PSI) or wall connection. However, it may be seen that in other embodiments, the external compressed gas source 100 may include any source of compressed gas suitable for use with a ventilator 12. In the exemplary embodiment, the ventilator 12 may be compatible with medical grade compressed air or oxygen. However, it may be seen that in certain embodiments, the ventilator 12 may be only suitable for use with one or the other, or with other compressed gases or blends of compressed gases.

When the ventilatory support apparatus 10 is transitioned from the stationary configuration to the stand-alone configuration, the ventilator 12 may be undocked from the compressor unit 14 in the same way as described above with respect to FIGS. 13A and 13B regarding transitioning to the extended range configuration. In the exemplary embodiment of the stand-alone configuration, after the ventilator 12 is undocked in this way, the compressor unit 14 may be powered off using the compressor power button 46.

With the ventilatory support apparatus 10 in the stand-alone configuration, the ventilator 12, in the exemplary embodiment, may then be powered on by pressing the ventilator power button 20 in the same way as when the exemplary ventilatory support apparatus 10 is in the stationary configuration or the extended range configuration, upon which the ventilator power indicator light 22 may illuminate. After power on of the compressor unit 14 and ventilator 12, various tests (e.g., a test of the alarm speaker) may be performed and startup screens may be displayed on the display 16, with the display 16 eventually displaying a "Home" screen (e.g., as described in the Example). As noted above, the ventilator 12 may include an internal battery 66 for use while undocked from the compressor unit 14, e.g. while the ventilatory support apparatus 10 is in the extended range configuration or the stand-alone configuration. The behavior of the internal battery 66 of the ventilator 12 and ventilator battery charge icon on the display 16, as well as the use of the ventilator battery charger connection port 32, ventilator battery charger 86, ventilator battery charger cord 88, and ventilator AC power cord 90, may be the same in the stand-alone configuration as in the extended range configuration (e.g., as described in the Example). Moreover, in the stand-alone configuration, the patient interface 80 may be plugged into the ventilator ventilation gas output port 38 in the same way as shown in FIG. 15 and described with respect to extended range configuration.

With the ventilatory support apparatus 10 in the stand-alone configuration, the ventilator 12 may be wearable on a belt or waistband via the belt clip 92 in the same way as described above with respect to FIGS. 17A and 17B. Alternatively, in either the stand-alone configuration or the extended range configuration, the belt clip 92 may also be used to secure the ventilator 12 to a pole via a pole mount 94 as described below. However, it may also be seen that other methods of securing the ventilator 12 and locations where it may be secured may be achieved, without departing from the scope of the disclosure.

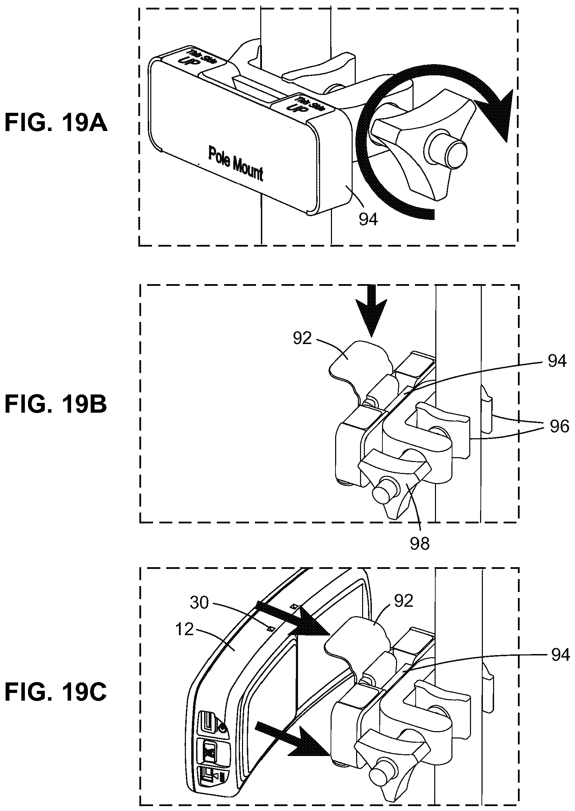

Turning now to FIGS. 19A-19C, perspective views showing an exemplary embodiment of the ventilator 12 being secured to a pole via the belt clip 92 and the pole mount 94 in an process having three steps are shown. First, as shown in FIG. 19A, the pole mount 94 is positioned around the pole in the desired orientation and secured to the pole. In the example shown in FIGS. 19A-19C, the pole mount 94 includes a vice clamp 96 that can be tightened around a pole by turning a knob 98, thereby securing the pole mount 94 to the pole. Second, as shown in FIG. 19B, the belt clip 92 is slid into a hole on the top of the pole mount 94 and pushed down (e.g., in the direction of the arrow shown in FIG. 19B) until secure. Third, and finally, the belt clip 92 is lined up with the belt clip sockets 30 on the ventilator 12, and the ventilator 12 is pushed toward the belt clip 92 until a connection is made, e.g., until the protrusions on the belt clip 92 enter the belt clip sockets 30 on the ventilator 12, causing an audible click.

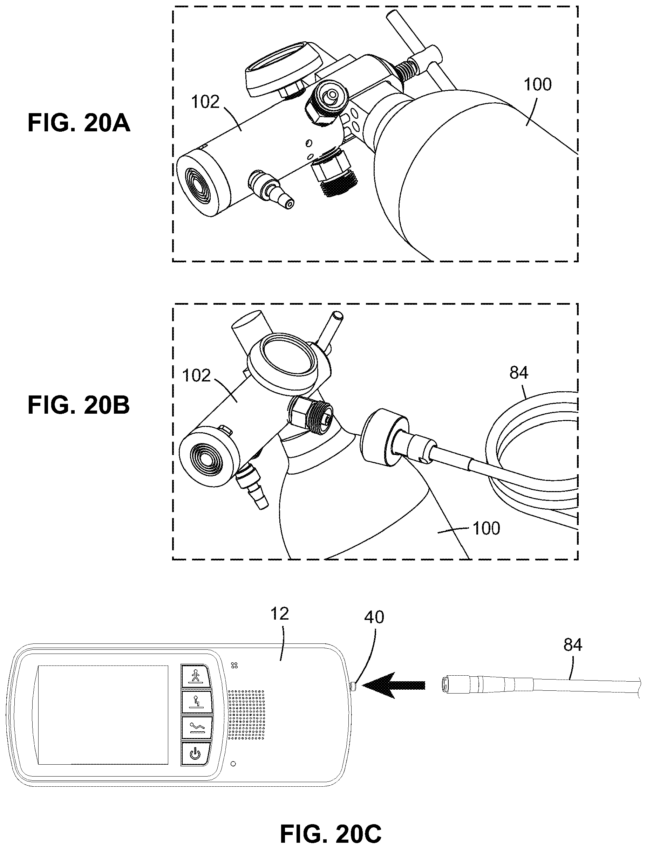

Turning now to FIGS. 20A-20C, perspective views (FIGS. 20A and 20B) and a top plan view (FIG. 20C) of the exemplary embodiment the ventilatory support apparatus 10 transitioning to the stand-alone configuration by connection of the ventilator 12 to an external compressed gas source 100 in an example process having three steps are shown. First, with the ventilator 12 powered off (the ventilator 12 may be powered off using the ventilator power button 20), an oxygen regulator 102 is connected to the external compressed gas source 100 as shown in FIG. 20A, e.g., by sliding the oxygen regulator 102 over the neck of an oxygen cylinder (external compressed gas source 100), lining up pins on the oxygen regulator 102 with holes on the neck, and tightening a tee screw on the oxygen regulator 102 by turning a handle. Second, as shown in FIG. 20B, the compressed gas hose 84 is connected to the oxygen regulator 102, e.g. to a DISS connector end of the oxygen regulator 102. Third, and finally, as shown in FIG. 20C, after turning on the gas supply according to the preferred usage method of the external compressed gas source 100 and oxygen regulator 102, the other end of the compressed gas hose 84 is connected to the ventilator 12, e.g., by pushing the small quick connect end onto the ventilator compressed gas inlet port 40 until it clicks into place. The external compressed gas source 100 may thereafter be replaced, e.g., as described in the Example.

In the exemplary embodiment illustrated in FIGS. 20A-20C, the external compressed gas source 100 is an oxygen cylinder and the compressed gas hose 84 and oxygen regulator 102 are used. However, it may be seen that in other embodiments, the external compressed gas source 100 may be different, e.g., another gas or gas blend other than oxygen, a portable gas compressor, or another oxygen source such as an oxygen concentrator. If the external compressed gas source 100 is an air cylinder, another hose such as an air hose may be used in place of the compressed gas hose 84 and another appropriate regulator may be used in place of the oxygen regulator 102.

When the ventilatory support apparatus 10 is transitioned to the stand-alone configuration, the ventilator 12 is not docked at the ventilator dock 42, the ventilator compressed gas inlet port 40 is in fluid communication with an external compressed gas source 100, and the patient interface gas inlet port 81 is in fluid communication with the ventilator ventilation gas output port 38, such that compressed gas is provided by the external compressed gas source 100 to the ventilator 12 and ventilation gas is provided by the ventilator 12 to the patient interface 80 without passing through the compressor unit 14.

With the ventilatory support apparatus 10 of the exemplary embodiment in the stand-alone configuration, the ventilator 12 may be powered off by pressing the ventilator power button 20 in the same way as in the extended range configuration, e.g., by pressing the ventilator power button 20 for three seconds and confirming power off using the display 16 (e.g., as described on in the Example). With the ventilator 12 powered off, the stand-alone range configuration can be terminated by disconnecting the compressed gas hose 84 from the ventilator 12 and the external compressed gas source 100.

As noted above, the ventilator 12 may include a wireless transmitter 31 and the compressor unit 14 may include a wireless receiver 79. In any of the above-described configurations (e.g. the stationary configuration, the extended range configuration, or the stand-alone configuration), the compressor 83 of the compressor unit 14 may be controllable by signal transmission from the wireless transmitter 31 to the wireless receiver 83 initiated by user input at the user interface 18. In this way, whether or not the ventilator 12 is docked with the compressor unit 14, the patient or another user of the ventilator 12 can wireless control the ventilatory support apparatus 10. Signal transmission between the wireless transmitter 31 and wireless receiver 83 may be according to any wireless communication standard known in the art. Alternatively, the ventilator 12 may communicate with the compressor unit 14 by a wired connection, in which case the wireless transmitter 31 and the wireless receiver 79 may be omitted. However, it may be seen that wireless communication may be advantageous, in that the ventilator 12 may be configured to control all electronically controllable aspects of the ventilatory support apparatus 10 in all configurations, without necessitating a separate set of controls on the compressor unit, and without requiring the presence of a wired signal link.

The ability to use the ventilatory support apparatus 10 in any one of the aforementioned configurations is attributable, at least in part, to the structural and functional features of its electromechanical pneumatic system which is under the control of a microprocessor. A pneumatic diagram of this system is provided in the Example. Along these lines, the Example sets forth the overall performance specifications of the ventilatory support apparatus 10 corresponding to its use in any of the aforementioned configurations.

By way of example, it is contemplated that an alternative version of the ventilatory support apparatus 10 may be provided which is adapted to be used in only the stationary and extended range configurations, and is not necessarily adapted for use in the stand-alone configuration. In such a variation, structures and on-board control algorithms/software corresponding to functionality in such stand-alone configuration could be eliminated in the ventilatory support apparatus 10. Along these lines, it is also contemplated that an alternative version of the ventilatory support apparatus 10 may be provided which eliminates features such as the low flow gas input port 64 in the compressor unit 14, along with its ancillary structural and functional/control features. In this instance, with the elimination of the low flow gas input port 64 and the resultant inability to introduce, for example, oxygen directly into the compressor unit 14 via that low flow gas input port 64, it is further contemplated that such variant of the ventilatory support apparatus 10 may be used in conjunction with a patient interface which is uniquely configured to allow, for example, oxygen to be introduced directly into such patient interface from a suitable source.

Example

1

Indications for Use

Symbols and Conventions

CHAPTER 1: LIFE2000™ VENTILATION SYSTEM INTRODUCTION _____

Safety Information

Features

Packaging Contents

System Components

Ventilator

Compressor

Configurations

Introduction to Stationary Configuration

Testing the Ventilation System

CHAPTER 2: STATIONARY CONFIGURATION _____

Positioning and Carrying the Compressor

Supplying Power to the Compressor

Docking the Ventilator into the
Compressor_____

Connecting an Interface to the Compressor

Connecting to a Low Flow Oxygen Source

Powering On Sequence for Stationary Configuration
Checking the Compressor's Internal Battery Status

Compressor Battery Charge Status (When Powered by Internal Battery)

Compressor Battery Charge Status (When Connected to AC Power)

Silence Alarm Button on the Compressor

Powering Off Sequence

Introduction to Extended Range Configuration
Testing the Ventilation System

CHAPTER 3: EXTENDED RANGE CONFIGURATION
Positioning and Carrying the Compressor

Supplying Power to the Compressor

Connecting to a Low Flow Oxygen Source

Undocking the Ventilator from the Compressor

Connecting the Ventilator and Compressor in Extended Range Configuration

Connecting an Interface to the Ventilator in Extended Range Configuration

2 3

Wearing the Pillows Interface

Checking the Pillows Interface Positioning

Introduction to Ventilation Settings

_____

Home Screen

_____

CHAPTER 6: VENTILATION SETTINGS _____

Moving Between the Home Screen and Menu Screen

_____

Menu Screen

_____

Touch Screen Energy-Save Mode

_____

Defining Clinical Settings

_____

Accessing the Settings Menu

_____

Accessing the Clinician's Settings Menu

_____

Disabling Access to the Clinician's Settings Menu

_____

Viewing and Editing Prescription Settings

_____

Factory Default Prescription Settings

_____

Breath Types

_____

Therapy
Modes_____

Setting Ventilation Parameters in Control Ventilation Mode

_____

Setting Ventilation Parameters in Assist/Control Ventilation Mode

_____

Setting Ventilation Parameters in Assist Ventilation Mode

_____

Setting Alarm Limits for Breath Rate and PIP

_____

Setting Breath Timeout (Apnea Backup Ventilation Mode)

Selecting the Source Gas

Choosing an Activity Button (Patient-Selectable)

"This Prescription Setting is Not Active" Message

"Connect Oxygen Source" or "Disconnect Oxygen Source" Message

Adjusting the Trigger Sensitivity (Patient-Adjustable)

Accessing the Trigger Sensitivity Screen

Changing Trigger Sensitivity

Accessing the Utilities Menu

Setting Time and Date

Setting Vibration Mode

Setting Audio Loudness

Adjusting Screen Brightness

Viewing Software Version Information

Powering On Sequence in Extended Range Configuration

Checking the Compressor's Internal Battery Status

Compressor Battery Charge Status (When Powered by Internal Battery)

Compressor Battery Charge Status (When Connected to AC Power)

Checking the Ventilator Battery Charge

Ventilator Battery Charge Icons, Meaning, and Approximate Time Remaining

Assembling the Ventilator Battery Charger and Charging the Ventilator

Securing the Ventilator

Belt Clip

Ventilator Silence Alarm Button

Powering OW Sequence In Extended Range Mode

Disconnecting Extended-Range Configuration

Introduction to Stand-alone Configuration

Testing the Ventilator

CHAPTER 4: STAND@ALONE CONFIGURATION
Undocking the Ventilator from the Compressor

Powering On Sequence For the Ventilator

Checking the Ventilator Battery Charge

Ventilator Battery Charge Icons, Meaning, and Approximate Time Remaining

Assembling the Ventilator Battery Charger and Charging the Ventilator

Securing the Ventilator

Belt Clip

Pole Mount

Ventilator Silence Alarm Button

Connecting an Interface to the Ventilator in Stand-alone configuration

Connecting to a Cylinder

Replacing the Source Gas Cylinder

Cylinder Duration Information

The Universal CircuitTM Interface and the Pillows Interface

Connecting an Interface to the Compressor in Stationary Configuration

CHAPTER 5: CONNECTING AN INTERFACE _____

The Universal CircuitTM Interface

Connecting the Universal CircuitTM Interface to ET Tubes or Masks

The Breathe Pillows Interface

4 5

Summary of Factory Default Settings

Introduction to Alarms and Alerts

CHAPTER 7: ALARMS, ALERTS, AND TROUBLESHOOTING _____

Ventilator On-Screen Alarm Sounds and Message Display

Active Alarms Window

Silencing and Clearing On-Screen Alarms

Ventilator Alarms

Medium-Priority Alarms

Low-Priority Alarms

Compressor Alerts

Troubleshooting

Cleaning Before First Use

Daily Checks

Environmental Specifications

CHAPTER 8: MAINTENANCE
Alarm Checks

Cleaning and Disinfecting the Ventilation System

Cleaning for Single-Patient Use

Cleaning for Multi-Patient Use

Cleaning the Breathe Interfaces

Purging the Pillows Interface and Universal CircuitTM Interface

Preventive Maintenance

Battery Replacement

Checking and Replacing the Water Tray

_____

Checking and Replacing the Air Inlet Filter

_____

Checking and Replacing the Cooling Air Filter

_____

Testing Ventilator Alarms

_____

Recommendations for Frequency of Testing

_____

Verifying Power-On Self-Test Alarms

_____

Verifying Backup Alarm Buzzer

_____

Checking Alarm Conditions

_____

CHAPTER 9: ACCESSORIES _____
Battery Information

_____

Ventilator Battery Specifications

_____

Compressor Battery Specifications

_____

Ventilator Battery Charger and Power Cord Specifications

_____

Compressor Power Supply and Power Cord Specifications

_____

Oxygen Monitor

_____

Exhalation Volume Monitor

_____

Ventilator Altitude Volume Adjustment Table

_____

Accessories and Replacement Parts

_____

General Overview

_____

Operation Summary

_____

Pneumatic Diagram of the Life2000TM Ventilation System

_____

CHAPTER 10: PRINCIPLES OF OPERATION _____
CHAPTER 11: PERFORMANCE SPECIFICATIONS _____
CHAPTER 12: COMPLIANCE AND IEC CLASSIFICATION _____
CHAPTER 13: ICONS _____
PRODUCT WARRANTY _____

6 7

67

INDICATIONS FOR USE

The Breathe Technologies Life2000TM Ventilation System is intended to provide continuous or intermittent ventilatory support for the care of individuals who require mechanical ventilation.

The ventilator is intended for use by qualified, trained personnel under the direction of a physician. Specifically, the ventilator is applicable for adult patients who require the following types of ventilatory support:

- Positive Pressure Ventilation, delivered invasively (via ET tube) or non-invasively (via mask).
- Assist/Control mode of ventilation.

The ventilator is suitable for use in home and institutional settings.

WARNING:

Use the Breathe Technologies Life2000TM Ventilation System only for patients who meet the Indications for Use.

If the ventilation system is used for patients that do not meet the Indications for Use, patients may not receive appropriate respiratory therapy.

**SYMBOLS AND CONVENTIONS**

The following symbols and conventions are used throughout this manual:

Table 1

| THIS | MEANS THIS |
|---|---|
| ⚠ WARNING: | Indicates hazards that, If not avoided, may cause severe injury or death. |
| ⚠ CAUTION: | Indicates hazards that, If not avoided, may resutl In minor or moderate injury, or damage to or impaired performance of equipment. |
| ⓘ TIP: | Indicates tips that may be helpful when using the ventilation system. |
| NOTE: and NOTES: | Indicates additional information about a behavior or feature. |

(continued)

| THIS | MEANS THIS |
|---|---|
| **BOLD TEXT** | The names of menu Items and buttons displayed on the touch screen are indicated with bold text.<br>For example, the Menu screen has several buttons, including Home Screen, Settings, and Information. |

SAFETY INFORMATION

Please read the following safety warnings and cautions in their entirety before using the Breathe Technologies Life2000TM Ventilation System. Warnings and cautions can also be found throughout this Instructions for Use.

WARNING:

- The Breathe Technologies Life2000TM Ventilation System is a restricted medical device intended for use by qualified, trained personnel under the direction of a physician.
- Use the Breathe Technologies Life2000TM Ventilation System only for patients who meet the Indications for Use. If the ventilation system is used for patients that do not meet the Indications for Use, patients may not receive appropriate respiratory therapy.
- If the Breathe Technologies Life2000TM Ventilation System is not functioning properly, respiratory therapy may be compromised and may result in patient harm or death. Always have an alternate means of ventilation or oxygen therapy available.
- The operator of the ventilation system is responsible for reading and understanding this manual before use.
- Failure to read this Instructions for Use may result in product misuse, which may cause equipment damage or patient mistreatment.
- The prescription and other device settings should only be changed on the order of the supervising physician.
- When the ventilation system is in use, keep it in a well-ventilated area to prevent it from overheating. The ventilation system may overheat and be permanently damaged if it is used in an area that is not well ventilated.
- Do not allow smoking near oxygen sources or near the ventilation system and do not place oxygen sources or the ventilation system near any source of direct heat or open flame because flammable materials burn more readily in the presence of oxygen.
- Do not submerge the ventilation system in liquids or pour liquids on it. Liquids may cause components in the system to malfunction.
- Do not use the Breathe Technologies Life2000TM Ventilation System in magnetic resonance imaging (MRI) environments. MRI equipment may cause electronic components in the system to malfunction.
- Do not use the ventilator or compressor in the presence of flammable anesthetics.
- Do not use the ventilation system with oxygen in the presence of flammable anesthetics such as fluroxene, cyclopropane, divinyl ether, ethyl chloride, ethyl ether, and ethylene, as they may form flammable or explosive mixtures with oxygen.
- Do not use the ventilator or compressor with helium or helium mixtures.
- Do not use the ventilator or compressor with nitric oxide.
- Do not use the ventilator or compressor in a hyperbaric chamber.
- Do not eat, drink, or chew gum while using the ventilation system. Food or liquids that make contact with the ventilation system may cause components in the system to malfunction. Eating, drinking, or chewing gum while using the system may also increase the risk of choking.
- Do not power on or use the compressor without the filters and water tray properly installed.
- Do not insert foreign objects into any part of the ventilation system.
- For any accessories, read the label and accompanying document(s) before use.

CAUTION:

- No user serviceable components are inside the device.
- Do not place the battery charger on wet surfaces or use in wet environments. Wet environments may damage the battery charger and may cause electric shock.
- Use only the Breathe Technologies approved battery charger and cord set with the ventilation system. If an unauthorized battery charger or cord set is used with the ventilation system the system may be damaged.
- If using in Extended Range or Stand-alone Configuration (wearable configurations), make sure the clip is securely fastened to the belt and the ventilator. If the clip is not securely fastened to the belt or the ventilator, the ventilator may fall and be damaged.
- If using in Extended Range or Stand-alone Configuration (wearable configurations), secure the ventilator to prevent it from falling or becoming damaged.
- Use the Pillows Interface or Universal CircuitTM interface for a maximum of 30 days. If an interface is used for more than 30 days, its performance may degrade and the patient may not receive adequate respiratory therapy.
- Do not use a Pillows Interface or Universal CircuitTM interface that is cracked, odorous, broken, or kinked. If a damaged interface is used, the patient may not receive adequate respiratory therapy.
- 70% isopropyl alcohol may damage the touch screen. When cleaning external surfaces of the ventilation system with 70% isopropyl alcohol, avoid contact with the touch screen.
- Keep in a clean environment to protect the equipment from ingress of dust, lint, and pests.
- Do not leave exposed to the sun or other sources of radiant heat, it may overheat.
- Do not allow children or pets to access the equipment, it may become damaged.
- The performance of the Life2000TM Compressor has only been validated with the Life2000TM Ventilator.
- Two such devices-nebulizers, ventilators, or purge connectors-cannot be connected to the compressor simultaneously.

WARNING:

- Use only Breathe Technologies approved accessories and replacement parts with the ventilation system. If unauthorized accessories or replacement parts are used with the system, the ventilation system may be damaged and performance may be degraded.
- Do not connect the ventilation system components or accessories to any other equipment that is not described in this Instructions for Use.
- Adding attachments or other components and/or sub-assemblies to the ventilator breathing system can cause an increase in expiratory resistance at the patient connection
- Adding humidification or nebulization can increase the resistance of the breathing circuit. The operator of the ventilation system needs to monitor the breathing system for increased resistance and blockage.
- Ventilator accuracy can be affected by the gas added by use of a nebulizer.
- To ensure accuracy of oxygen administration and to monitor for the presence of contamination (incorrect gas connected), use an external oxygen monitor to verify the oxygen concentration in the delivered gas.
- To monitor minute volume, use an external exhaled volume monitor.
- Before beginning ventilation therapy in Stand-Alone Configuration, verify that there is an adequate supply of source gas for the intended duration of the therapy. Otherwise, the patient may not receive appropriate therapy.
- Use only a Breathe Technologies approved oxygen or air hose with the ventilation system. If an unauthorized oxygen or air hose is used with the ventilation system, the system may be damaged.
- Only use the ventilator with the compressor or approved medical grade compressed gas (oxygen or dry air). Use with non-approved sources of gas may cause the ventilator to malfunction and the patient may not receive appropriate respiratory therapy.
- If using the ventilator with an alternate gas source in Stand-Alone Configuration, and the ventilator is not used with a regulator capable of 41 PSI to 87 PSI (nominal 50 PSI) with greater than 40 LPM capability, patients may not receive appropriate respiratory therapy.
- The provided oxygen regulator should not be used with a cylinder rated at 3000 PSI or higher.
- Breathe interfaces are designed for single-patient use. To prevent risk of cross-contamination use a new Pillows Interface or Universal CircuitTM interface for each new patient. For third-party masks, refer to the user guide provided by the manufacturer for replacement and/or cleaning and disinfection instructions.
- Do not subject Breathe interfaces to heat sterilization, hot water pasteurization, autoclaving, radiation sterilization, ethylene oxide gas sterilization, or attempt to clean them in a dishwasher or microwave oven.

Doing any of these may damage the interfaces and impair gas delivery.

- If using the Breathe Pillows Interface, properly secure the patient interface to the face and route tubing around the ears to avoid strangulation.
- Do not cover or block the compressor's speakers with tape or any other object. Covering the speakers may make it difficult for a patient or caregiver to hear alarms, which may result in inadequate respiratory therapy.
- Do not cover the ventilator, touch screen, speaker, or backup alarm buzzer with tape or any other object. Covering the ventilator or any of its parts might cause difficulty in hearing alarms and might affect ventilator performance.
- Reducing the alarm loudness level to lower than the ambient sound level will impede alarm condition recognition.
- The backside of the ventilator enclosure may reach 49°C in a 40°C environment.

FEATURES

The Breathe Technologies Life2000TM Ventilation System is a critical care, volume control mechanical ventilation

system designed for a broad range of applications in critical care and home settings. The modular Life2000TM Ventilation System (system) is composed of the Life2000TM Ventilator (ventilator) and the

Life2000TM Compressor (compressor).

The ventilator:

- Offers three different volume control modes of operation:
- Control Ventilation
- Assist/Control Ventilation
- Assist Ventilation.
- Can be used with a variety of commercially available invasive interfaces (such as ET tubes) or non-invasive masks such as full face, nasal, and pillows masks.
- Enables clinicians to define three Prescription Settings based on patient needs.
- Allows for an adjustable PEEP setting for each Prescription Setting.
- Allows for an adjustable trigger sensitivity for each Prescription Setting.
- Includes the ability to set various critical alarms for each Prescription Setting.
- Has up to four hours of battery-powered operation.
- Displays patient breath rate, Peak Inspiratory Pressure (PIP), average flow, and current volume level.

The compressor:

- Provides a continuous 50-PSI pressure source.
- Is a charging station for the ventilator.
- Has an internal battery with up to two hours of operation.
- Offers a connection point for an optional low flow oxygen source

PACKAGING CONTENTS

WARNING: For any accessories, read the label and accompanying document(s) before use.

TIP:

The ventilation system is shipped in specially designed, protective boxes. Do not throw away the boxes; keep them for future transportation needs.

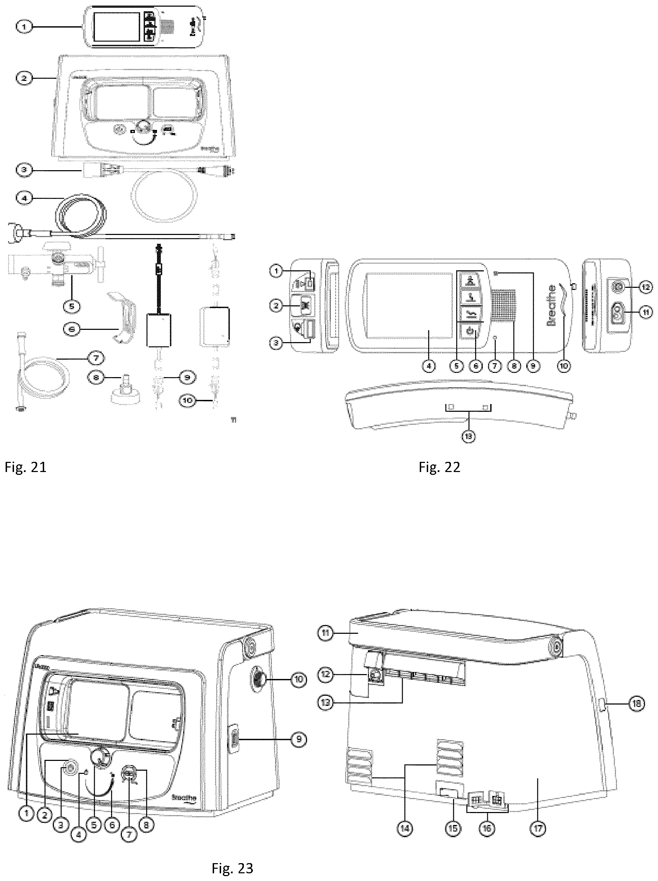

As an example, Fig 21 shows

1 Life2000TM Ventilator (ventilator)
The ventilator can be used with the Life2000TM
Compressor or an alternate 50-PSI pressure source.
2 Life2000TM Compressor (compressor)
The compressor is an electropneumatic power unit
that provides the ventilator with a continuous pressure
source and is a charging station for the ventilator.

3 Universal CircuitTM interface
The Universal CircuitTM is used to connect any commercially-available non-invasive mask (full face, nasal, pillows) or tracheostomy tube to the ventilation system or ventilator.
4 Oxygen hoses (6 ft. and 50 ft.)
These high-pressure hoses can be used to connect the ventilator to the compressor when in Extended Range Configuration or can connect the ventilator to an oxygen cylinder when in Stand-alone Configuration.
5 Oxygen regulator
The regulator can be used to connect the ventilator to a cylinder in Stand-alone Configuration.
6 Belt clip for ventilator
The belt clip is used to secure the ventilator when it is used in wearable configurations.
7 Purge tube
The purge tube is used during the interface cleaning process.
8 Purge tube connector
The purge tube connector connects the purge tube to the compressor during the interface cleaning process.
9 Battery charger and AC power cord for the ventilator
The battery charger and AC power cord connect the ventilator to an AC power source.
10 External power supply and AC power cord for the compressor
The external power supply and AC power cord connect the compressor to an AC power source.

As an example, Fig 23 shows
COMPRESSOR

FRONT

1 Ventilator docking cradle
2 Power source indicator light
3 Power button for compressor
4 Locked icon
(ventilator charging indicator light)
5 Locking knob
6 Unlocked icon
7 Battery charge status button
8 Battery charge indicator lights
and battery charge scale

SIDE

9 Interface connection
10 DISS 1240 output connection

BACK

11 Handle
12 Low flow oxygen input
connection
and filter cover
13 Cooling air filter cover
14 Cooling vents

15 Water tray
16 Power supply connection
and cover

SIDE

17 Alarm speaker (internal)
18 Silence Alarm button

As an example, Fig 22 shows
SYSTEM COMPONENTS
VENTILATOR

TOP

1 Battery charger connection
2 Silence Alarm button
3 For manufacturer's use only

FRONT

4 Touch screen
5 Prescription Settings buttons
High Activity button
Medium Activity button
Low Activity button
6 Ventilator Power button
7 Power indicator light
8 Alarm speaker
9 Backup alarm speaker
10 Breath indicator light

SIDE
13 Belt clip sockets
BOTTOM

11 Interface connection
12 Gas inlet connection

CONFIGURATIONS

The modular Life2000TM Ventilation System (system) is composed of the Life2000TM Ventilator (ventilator) and the
Life2000TM Compressor (compressor). The system can be used in three different configurations.

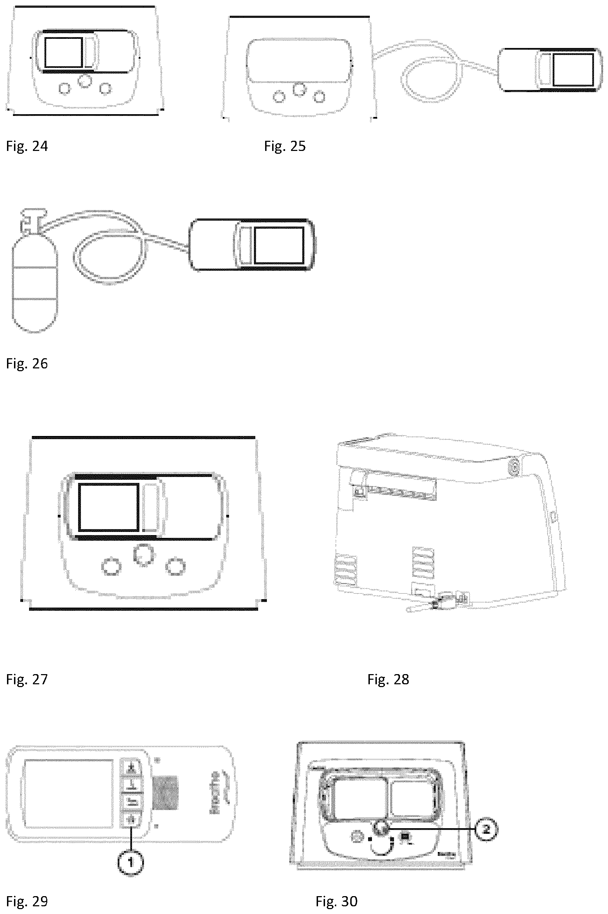

STATIONARY CONFIGURATION (Fig. 24)

The ventilator is docked into the compressor for ventilation while resting. For information about how to setup the ventilation system in this configuration, see the following chapter "Chapter 2: Stationary Configuration".

EXTENDED RANGE (WEARABLE) CONFIGURATION (Fig. 25)

The ventilator is connected to the compressor with a gas supply hose to enable the activities of daily living. For information about how to setup the ventilation system in this configuration, see "Chapter 3: Extended Range Configuration" on page 26.

STAND-ALONE (WEARABLE) CONFIGURATION (Fig. 26)

The ventilator is connected to an alternate pressure source such as an oxygen or air cylinder, or hospital wall source. For more information about how to setup the ventilator in this configuration, see "Chapter 4: Stand-alone Configuration".

INTRODUCTION TO STATIONARY CONFIGURATION

In Stationary Configuration, the ventilator is docked into the compressor (Fig. 27). The compressor provides the ventilator

with a continuous pressure source and functions as a charging station.

TESTING THE VENTILATION SYSTEM

In a multi-patient setting, the ventilation system must be tested before it is assigned to a new patient. For instructions on testing the ventilation system, see "Testing Ventilator Alarms".

POSITIONING AND CARRYING THE COMPRESSOR

Position the compressor upright on a flat, level surface. Make sure that the cooling vents and air inlet on the back of the compressor are not blocked, and there is sufficient clearance from surrounding objects.
The compressor should not be used adjacent to or stacked with other equipment and that if adjacent or stacked use is necessary, the compressor should be observed to verify normal operation in the configuration in which it will be used.
When carrying the compressor, make sure to use the handle and keep the compressor in an upright position.
NOTE: The performance of the compressor may degrade in high temperature, high humidity, or high altitude environments. If degradation is seen, switch to an alternate means of ventilation. Verify the performance of the compressor for adequate therapy delivery in the environment(s) in which it will be used and adjust the volume to compensate for altitude when necessary.

WARNING:

- Do not use the ventilation system in the presence of flammable anesthetics.
- Do not cover or block the ventilation system's speakers with tape or any other object. Covering the speakers may make it difficult for a patient or caregiver to hear alarms, which may result in inadequate respiratory therapy.
- Do not cover the ventilator, touch screen, speaker, or backup alarm buzzer with tape or any other object. Covering the ventilator or any of its parts might cause difficulty in hearing alarms and might affect ventilator performance.
- When the ventilator is in use, keep it in a well-ventilated area to prevent it from overheating. The ventilator may overheat and be permanently damaged if it is used in an area that is not well ventilated.
- Do not connect the ventilation system components or accessories to any other equipment that is not described in this Instructions for Use.

CAUTION:

- Keep in a clean environment to protect the ventilation system from ingress of dust, lint, and pests.
- Do not leave the ventilation system exposed to the sun or other sources of radiant heat, it may overheat.
- Do not allow children or pets to access the ventilation system, it may become damaged.

SUPPLYING POWER TO THE COMPRESSOR

An AC power cord and external power supply are included with the compressor (Fig. 28).

1 Assemble the compressor's external power supply and cordset by attaching the AC power cord to the compressor's external power supply and cord.

2 Connect the pronged end of the compressor's AC power cord to an AC power source and verify that the green LED indicator on the external power supply lights up to indicate the connection.

3 Open the power supply connection cover.

4 Insert the compressor's power connector into the power supply connection on the back of the compressor until it clicks into place; the lock tab on the cord will be on the top.

NOTE: To remove the power cord from the compressor, press in the lock tab on the power cord to release the cord from the compressor.

TIP:

The compressor's power supply connection is equipped with a protective cover to prevent ingress of water and debris. Use the cover to keep the power supply connection covered when not in use.

CAUTION:

- Do not place the compressor cordset or external power supply on wet surfaces or use in wet environments. Wet environments may damage the power cordset or external power supply and may cause electric shock.
- Use only the Breathe Technologies approved cordset and external power supply with the compressor. Using an unauthorized cordset or external power supply may damage the compressor.

DOCKING THE VENTILATOR INTO THE COMPRESSOR

1 Ensure that the ventilator is powered off (Fig. 29).

NOTE: Alarms may be encountered and/or the Prescription Setting might be inadvertently changed if the ventilator is not powered off before docking.

2 To dock the ventilator into the compressor, make sure the locking knob is in the unlock position as shown in Fig. 30

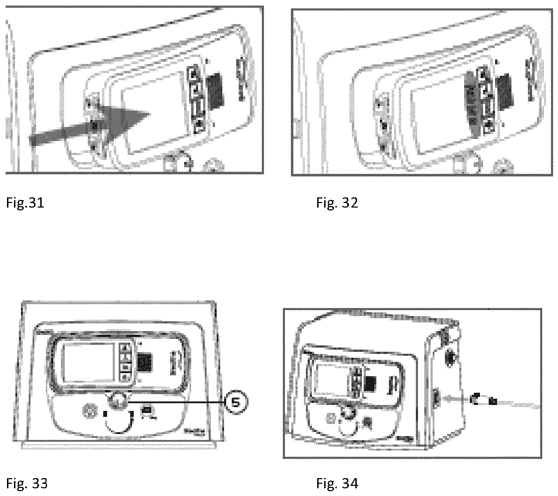

3 Position the ventilator into the compressor cradle as shown in Fig 31.

Push the ventilator into the compressor in the direction indicated by the arrow until it clicks into place.

4 Gently push the center of the ventilator (in the area indicated in Fig 32) until the front of the ventilator is flush with the front of the compressor and clicks into place.

5 Turn the locking knob clockwise to the locked position to lock the ventilator in the compressor (Fig. 33).

The locked icon also functions as a charging indicator for the ventilator; it will light when the compressor is on and the ventilator is properly docked to indicate that the ventilator is being charged by the compressor.

NOTE: For undocking instructions, see "Undocking the Ventilator from the Compressor".

CONNECTING AN INTERFACE TO THE COMPRESSOR (Fig. 34)

Plug the Universal CircuitTM interface or Pillows Interface into the interface port on the side of the compressor until it clicks. For more information about wearing interfaces, see "Chapter 5: Connecting an Interface".

CONNECTING TO A LOW FLOW OXYGEN SOURCE

Connect an oxygen source to the compressor when using a Prescription Setting that uses oxygen as the source gas. For more information, see "Selecting the Source Gas" on page 77.

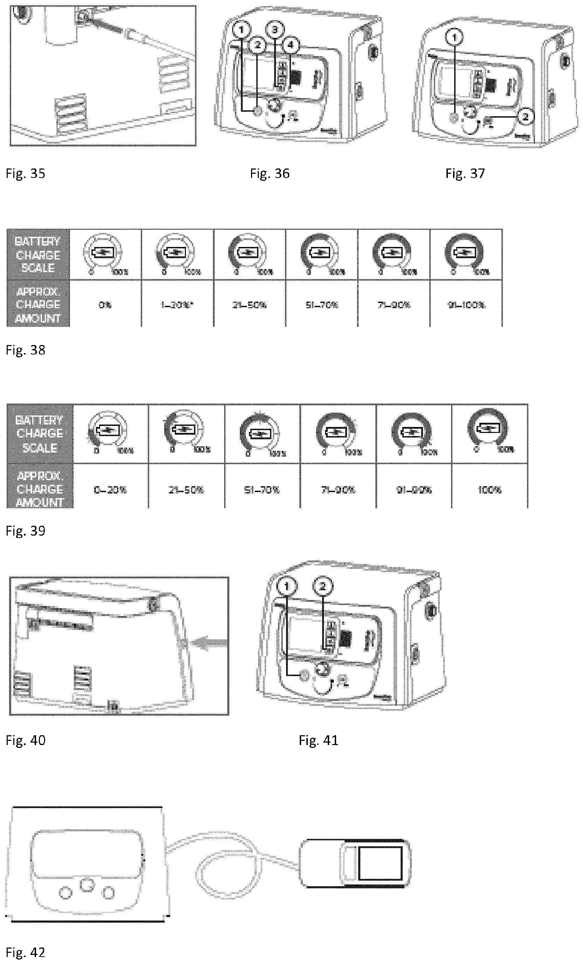

1 To connect a low flow supplemental oxygen source, such as a stationary oxygen concentrator, to the compressor, first obtain oxygen connecting tubing (Fig. 35).

2 Attach one end of the tubing to the low flow oxygen input on the back of the compressor by pushing and turning the oxygen connecting tubing until it is completely and securely attached.

3 Connect the other end of the oxygen connecting tubing to the supplemental low flow oxygen source.

4 Turn on the oxygen source.

NOTE: Make sure to connect and turn on the oxygen source, when used.

WARNING:

- Do not use the Breathe Technologies Life2000TM Ventilation System with oxygen in the presence of flammable anesthetics such as fluroxene, cyclopropane, divinyl ether, ethyl chloride, ethyl ether, and ethylene, as they may form flammable or explosive mixtures with oxygen.
- Do not allow smoking near oxygen sources or near the ventilation system and do not place oxygen sources or the ventilation system near any source of direct heat or open flame because flammable materials burn more readily in the presence of oxygen.

CAUTION:

Do not use the humidifier bottle attached to the oxygen concentrator when connected to the Life2000TM Compressor.

POWERING ON SEQUENCE FOR STATIONARY CONFIGURATION (Fig. 36)

1 Power on the compressor by pressing the power button for the compressor.
2 When powered on, lights surrounding the compressor power button will illuminate: Green indicates the compressor is connected to AC power.
Orange indicates the internal battery is being used.
3 Power on the ventilator by pressing the power button for the ventilator.
4 The green power indicator light on the ventilator shows that the ventilator is on.
5 When the startup screen is displayed, listen for audible tones to test the alarm speaker.
During the ventilator's start up sequence, the ventilator will perform a self test. During the test, all ventilator indicator lights should briefly flash and an audible alarm should briefly sound. This self test can take up to 15 seconds to complete. If you do not hear tones when you turn on the ventilator, contact your Breathe Technologies service representative.
6 When the Home screen is displayed, the touch screen is ready to use. The Home screen will display Air or O2 based on the option prescribed and selected.

NOTE: Ventilation will not begin until a Prescription Setting button is selected. For more information see "Choosing an Activity Button (Patient-Selectable)".

CHECKING THE COMPRESSOR'S INTERNAL BATTERY STATUS

The compressor is equipped with an internal battery for temporary power disruptions. This internal battery:

- charges when the compressor is attached to AC power.
- has a maximum charge of two hours.

0100%

1 When the compressor is powered on and the power source indicator lights surrounding the power button are illuminated in orange the compressor is running on its internal battery (Fig 37).
2 The battery charge indicator lights and battery charge scale surround the battery charge status button and indicate the compressor's current battery charge level. Consult the charts on the following page to determine the approximate amount of charge on the compressor's internal battery (Fig. 37).

TIP:

When the compressor is off, press and hold the Battery Charge Status button to display the indicator lights in the battery charge scale.

When the compressor is powered on, the indicator lights in the battery charge scale are visible. When the compressor is powered off, press and hold the battery charge status button to display the battery charge scale indicator lights.

The behavior of the indicator lights in the battery charge scale display is different depending on if the compressor is connected to an AC power source or running on its internal battery.

COMPRESSOR BATTERY CHARGE STATUS (WHEN POWERED BY INTERNAL BATTERY)

When the compressor is powered on and running on its internal battery, the indicator lights surrounding the compressor's power button are illuminated in orange (Fig. 38).

**[0097]** * The compressor's low battery alarm will sound when its internal battery charge drops below 20%.

COMPRESSOR BATTERY CHARGE STATUS (WHEN CONNECTED TO AC POWER)

When the compressor is powered on and connected to AC power, the indicator lights surrounding the compressor's power button are illuminated in green (Fig. 39).

TIP:

When the compressor's internal battery is charging, the blinking indicator light shows the current level of charge.

SILENCE ALARM BUTTON ON THE COMPRESSOR

Silencing and clearing alarms is a multi-step process that depends on alarm priority and how many alarms are active. For more information see "Chapter 7: Alarms, Alerts, and Troubleshooting".

1 Silence Alarm button on the compressor (Fig 40).
Press the Silence Alarm button to temporarily silence the alarm for 60 seconds. Pressing the Silence Alarm button silences only one alarm at a time-in audible or vibrating alarm mode. If more than one alarm occurs, press the Silence Alarm button once for each alarm. If the alarm is a medium- or high-priority alarm and is not silenced after 60 seconds, the alarm will continue with an additional buzzer.
2 Resolve the condition that triggered the alarm. For help resolving alarms, see the alarm and troubleshooting tables in "Chapter 7: Alarms, Alerts, and Troubleshooting" for possible causes of an alarm and options to resolve it. If an alarm silence button is pressed but the condition that triggered the alarm is not resolved, the alarm will sound again after 60 seconds.
3 After resolving a High Temperature, High Circuit Pressure, or High PEEP Pressure high-priority alarm, touch OK in the message that indicates the alarm has been resolved.

POWERING OFF SEQUENCE

(Fig. 41)

1 To power off the compressor, press the Compressor Power button.
2 To power off the ventilator, press the Ventilator Power button for three seconds until a confirmation screen appears.
3 To continue to power off the ventilator, choose CONFIRM.

NOTE: If a selection is not made within 20 seconds or if the BACK button is selected, the previous screen will be displayed and the ventilation status will not be affected.

INTRODUCTION TO EXTENDED RANGE CONFIGURATION (Fig 42)

The Life2000TM Ventilation System can be used in different configurations of operation as the patient's needs change. In the Extended Range Configuration, the ventilator is connected to the compressor with an oxygen hose to enable the activities of daily living.
NOTE: If not directly connected to AC power, make sure the ventilator battery has sufficient charge for your length of use.

TESTING THE VENTILATION SYSTEM

In a multi-patient setting, the ventilation system must be tested before it is assigned to a new patient. For instructions on testing the ventilation system, see "Testing Ventilator Alarms".

POSITIONING AND CARRYING THE COMPRESSOR

Position the compressor upright on a flat, level surface. Make sure that the cooling vents and air inlet on the back of the compressor are not blocked, and there is sufficient clearance from surrounding objects.
The compressor should not be used adjacent to or stacked with other equipment and that if adjacent or stacked use is necessary, the compressor should be observed to verify normal operation in the configuration in which it will be used.
When carrying the compressor, make sure to use the handle and keep the compressor in an upright position. NOTE: The performance of the compressor may degrade in high temperature, high humidity, or high altitude environments. If degradation is seen, switch to an alternate means of ventilation. Verify the performance of the compressor for adequate therapy delivery in the environment(s) in which it will be used and adjust the volume to compensate for altitude when necessary.

WARNING:

•    Do not use the ventilation system in the presence of flammable anesthetics.
•    Do not cover or block the ventilation system's speakers with tape or any other object. Covering the speakers may make it difficult for a patient or caregiver to hear alarms, which may result in inadequate respiratory therapy.
•    Do not cover the ventilator, touch screen, speaker, or backup alarm buzzer with tape or any other object. Covering the ventilator or any of its parts might cause difficulty in hearing alarms and might a]ect ventilator performance.
•    When the ventilator is in use, keep it in a well-ventilated area to prevent it from overheating. The ventilator may overheat and be permanently damaged if it is used in an area that is not well ventilated.
•    Do not connect the ventilation system components or accessories to any other equipment that is not described in this Instructions for Use.

CAUTION:

•    Keep in a clean environment to protect the ventilation system from ingress of dust, lint, and pests.
•    Do not leave the ventilation system exposed to the sun or other sources of radiant heat, it may overheat.
•    Do not allow children or pets to access the ventilation system, it may become damaged.

SUPPLYING POWER TO THE COMPRESSOR

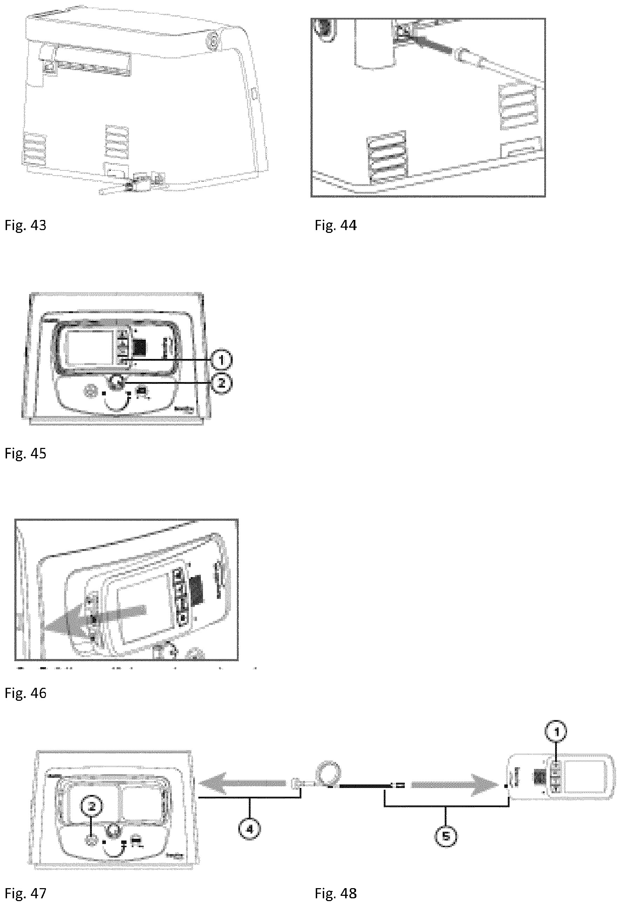

Follow the steps below to connect the compressor to an AC power source (Fig 43).

1 Assemble the compressor's external power supply and cordset by attaching the AC power cord to the compressor's external power supply and cord.
2 Connect the pronged end of the compressor's AC power cord to an AC power source and verify that the green LED indicator on the external power supply lights up to indicate the connection.

3 Open the power supply connection cover.
4 Insert the compressor's power connector into the power supply connection on the back of the compressor until it clicks into place; the lock tab on the cord will be on the top.

NOTE: To remove the power cord from the compressor, press in the lock tab on the power cord to release the cord from the compressor.

TIP:

The compressor's power supply connection is equipped with a protective cover to prevent ingress of water and debris. Use the cover to keep the power supply connection covered when not in use.

CAUTION:

• Do not place the compressor cordset or external power supply on wet surfaces or use in wet environments. Wet environments may damage the power cordset or external power supply and may cause electric shock.
• Use only the Breathe Technologies approved cordset and external power supply with the compressor. Using an unauthorized cordset or external power supply may damage the compressor.

CONNECTING TO A LOW FLOW OXYGEN SOURCE

Connect an oxygen source to the ventilation system when using a Prescription Setting that uses oxygen as the source gas. For more information, see "Selecting the Source Gas" on page 77.

1 To connect a low flow supplemental oxygen source, such as a stationary oxygen concentrator, to the ventilation system, first obtain oxygen connecting tubing (Fig. 44).
2 Attach one end of the tubing to the low flow oxygen input on the back of the compressor by pushing and turning the oxygen connecting tubing until it is completely and securely attached.
3 Connect the other end of the oxygen connecting tubing to the supplemental low flow oxygen source.
4 Turn on the oxygen source.

NOTE: Make sure to connect and turn on the oxygen source, when used. Don't use the bubbler on the oxygen concentrator when connected to the compressor.

WARNING:

• Do not use the Breathe Technologies Life2000TM Ventilation System with oxygen in the presence of flammable anesthetics such as fluroxene, cyclopropane, divinyl ether, ethyl chloride, ethyl ether, and ethylene, as they may form flammable or explosive mixtures with oxygen.
• Do not allow smoking near oxygen sources or near the ventilation system and do not place oxygen sources or the ventilation system near any source of direct heat or open flame because flammable materials burn more readily in the presence of oxygen.

CAUTION:

Do not use the humidifier bottle attached to the oxygen concentrator when connected to the Life2000TM compressor.

UNDOCKING THE VENTILATOR FROM THE COMPRESSOR

(Fig. 45)

1 Power off the ventilator.

NOTE: Alarms may be encountered and/or the Prescription Setting might be inadvertently changed if the ventilator is not powered off before undocking.

2 Turn the locking knob counterclockwise to the unlocked position until the compressor ejects the ventilator.

NOTE: Some tension will be encountered while turning during ejection.

3 Pull the ventilator as shown in Fig 46 to separate it from the compressor cradle. NOTE: For docking instructions, see "Docking the Ventilator into the Compressor".

CONNECTING THE VENTILATOR AND COMPRESSOR IN EXTENDED RANGE CONFIGURATION

In this configuration, the ventilator and compressor are connected by an oxygen hose. This allows use of the ventilator without having to be docked into the compressor (Fig.47 and Fig. 48)

1 Ensure the ventilator is powered off.

NOTE: Alarms may be encountered and/or the Prescription Setting might be inadvertently changed if the ventilator is not powered off before undocking.

2 The compressor may be powered off or powered on.

3 Undock the ventilator from the compressor; for more information, see "Undocking the Ventilator from the Compressor" on page 30.

4 Attach the DISS connection on the oxygen hose to the DISS 1240 output on the compressor.

NOTE: A 6 ft. oxygen hose and a 50 ft. oxygen hose are included with the ventilation system. To purchase other hose lengths, see "Accessories and Replacement Parts" on page 141.

5 Connect the other end of the oxygen hose to the ventilator by pushing the small quick connect end onto the gas inlet connection on the ventilator; when connected, the quick connect end will click into place.

6 For more information about powering on, see "Powering On Sequence in Extended Range Configuration" on page 33.

NOTES:

Ventilation will not begin until a Prescription Setting button is selected. For more information see "Choosing an Activity Button (Patient-Selectable)" on page 78.

- During use in Extended Range Configuration, the oxygen hose should remain connected to the ventilator at all times, except when required to be disconnected for maintenance, testing, or replacement. If it is disconnected while the ventilator is on and delivering therapy, a Low Gas Pressure alarm will occur. For more information see "Low Gas Pressure" on page 103.

CAUTION: Use only a Breathe Technologies approved oxygen hose with the ventilation system.

TIP:

A low flow oxygen source can also be connected to the compressor, based on prescription. For more information see "Connecting to a Low Flow Oxygen Source" on page 20.

32 33

CONNECTING AN INTERFACE TO THE VENTILATOR IN EXTENDED RANGE CONFIGURATION

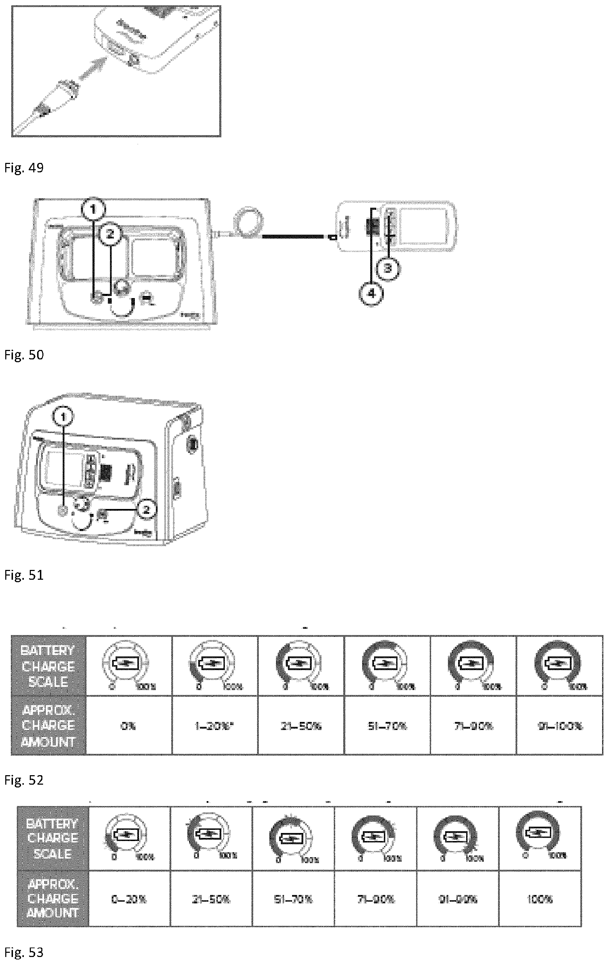

Plug the Pillows Interface or Universal CircuitTM interface into the interface port on the bottom of the ventilator until it clicks (Fig. 49). For more information about wearing interfaces, see "Chapter 5: Connecting an Interface".

NOTE: Ensure that the Universal CircuitTM interface or Pillows Interface is connected to the ventilator in Extended Range Configuration and Stand-alone Configuration.

Powering on Sequence in extended range configuration (Fig. 50)

1 Power on the compressor by pressing the power button for the compressor.

2 When powered on, lights surrounding the compressor power button will illuminate: Green indicates the compressor is connected to AC power.

Orange indicates the internal battery is being used.

3 Power on the ventilator by pressing the power button for the ventilator.

4 The green power indicator light on the ventilator shows that the ventilator is on.

5 When the startup screen is displayed, listen for audible tones to test the alarm speaker.

During the ventilator's start up sequence, the ventilator will perform a self test. During the test, all ventilator indicator lights should briefly flash and an audible alarm should briefly sound. This self test can take up to 15 seconds to complete. If you do not hear tones when you turn on the ventilator, contact your Breathe Technologies service representative.

6 When the Home screen is displayed, the touch screen is ready to use. The Home screen will display Air or O2 based on the option prescribed and selected.

NOTE: Ventilation will not begin until a Prescription Setting button is selected. For more information see "Choosing an Activity Button (Patient-Selectable)".

CHECKING THE COMPRESSOR'S INTERNAL BATTERY STATUS

The compressor is equipped with an internal battery for temporary power disruptions. This internal battery:

- charges when the compressor is attached to AC power.
- has a maximum charge of two hours.

Fig. 51

1 When the compressor is powered on and the power source indicator lights surrounding the power button are illuminated in orange the compressor is running on its internal battery.

2 The battery charge indicator lights and battery charge scale surround the battery charge status button and indicate the compressor's current battery charge level. Consult the charts on the following page to determine the approximate amount of charge on the compressor's internal battery.

TIP:

When the compressor is off, press and hold the Battery Charge Status button to display the indicator lights in the battery charge scale.

When the compressor is powered on, the indicator lights in the battery charge scale are visible. When the compressor is powered off, press and hold the battery charge status button to display the battery charge scale indicator lights.

The behavior of the indicator lights in the battery charge scale display is different depending on if the compressor is connected to an AC power source or running on its internal battery.

COMPRESSOR BATTERY CHARGE STATUS (WHEN POWERED BY INTERNAL BATTERY)

When the compressor is powered on and running on its internal battery, the indicator lights surrounding the compressor's power button are illuminated in orange (see Fig. 52)

* The compressor's low battery alarm will sound when its internal battery charge drops below 20%.

COMPRESSOR BATTERY CHARGE STATUS (WHEN CONNECTED TO AC POWER)

When the compressor is powered on and connected to AC power, the indicator lights surrounding the compressor's power button are illuminated in green.

TIP:

When the compressor's internal battery is charging, the blinking indicator light shows the current level of charge (Fig. 53)

CHECKING THE VENTILATOR BATTERY CHARGE

If you intend to use the ventilator on battery power, make sure that the battery is sufficiently charged for your duration of use.

It takes approximately three to four hours to fully recharge the battery whether the ventilator is off or on. A fully charged battery should last up to four hours.

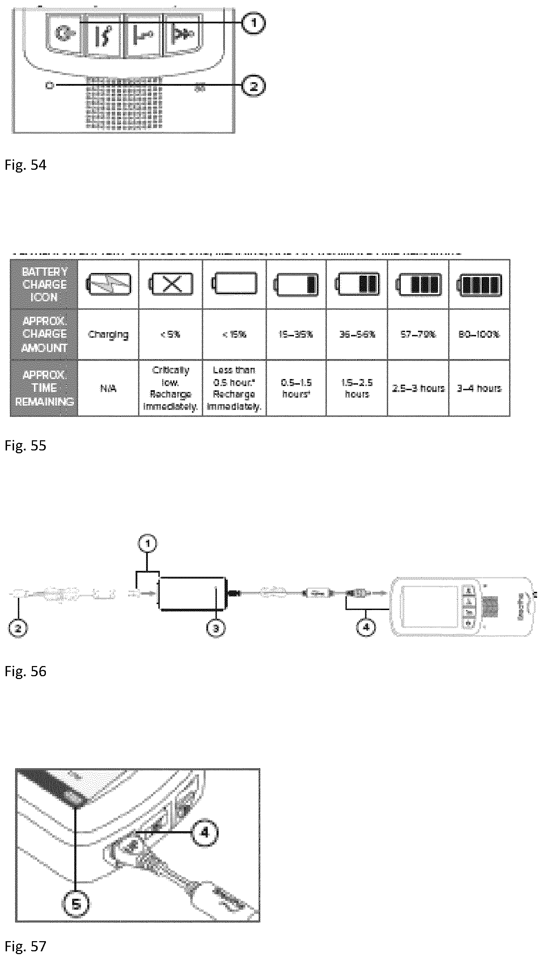

(see Fig. 54)

1 Press the Power button to turn on the ventilator.
2 The green power indicator light shows that the ventilator is on.
3 Check the Ventilator Battery Charge icon on the touch screen to see the current battery charge level for the ventilator. Refer to the chart on the following page to determine the approximate amount of ventilator battery charge and the approximate time remaining.

NOTE: The Ventilator Battery Charge icon is always displayed on the touch screen during normal operation.

VENTILATOR BATTERY CHARGE ICONS, MEANING, AND APPROXIMATE TIME REMAINING (see Fig. 55)

ASSEMBLING THE VENTILATOR BATTERY CHARGER AND CHARGING THE VENTILATOR

1 Plug the ventilator AC power cord into the ventilator battery charger (Fig. 56).
2 Connect the ventilator AC plug into an AC power source (Fig. 56).
3 The ventilator battery charger indicator light will turn green or red when connected to AC power (Fig. 56).
NOTE: The ventilator can be used while the battery is charging.
4 Connect the ventilator battery charger cord to the ventilator battery charger connection port. The word UP on the battery charger cord will be on top (see Fig. 57).
5 If powered on, check the Ventilator Battery Charge icon on the touch screen to see the ventilator's current battery charge status (Fig. 57).

NOTES:

- When charging the battery, there may be a 5-10 second delay before the Ventilator Battery Charge icon appears on the touch screen.
- The ventilator performance is the same regardless of power source (internal battery or AC).

CAUTION:

- Do not place the battery charger on wet surfaces or use in wet environments. Wet environments may damage the battery charger and may cause electric shock.
- Use only the Breathe Technologies approved battery charger and cord set with the ventilator. If an unauthorized battery charger or cord set is used with the ventilator, the ventilator may be damaged.

SECURING THE VENTILATOR

BELT CLIP

You can attach the ventilator to a belt or waistband using the included belt clip. The ventilator can be worn on either the right or left side.

CAUTION:

- Make sure the belt clip is securely fastened to the belt and the ventilator. If the belt clip is not securely fastened to the belt and the ventilator, the ventilator may fall and be damaged.

- Always secure the ventilator to prevent it from falling or becoming damaged.
- Use only the Breathe Technologies approved belt clip with the ventilator.

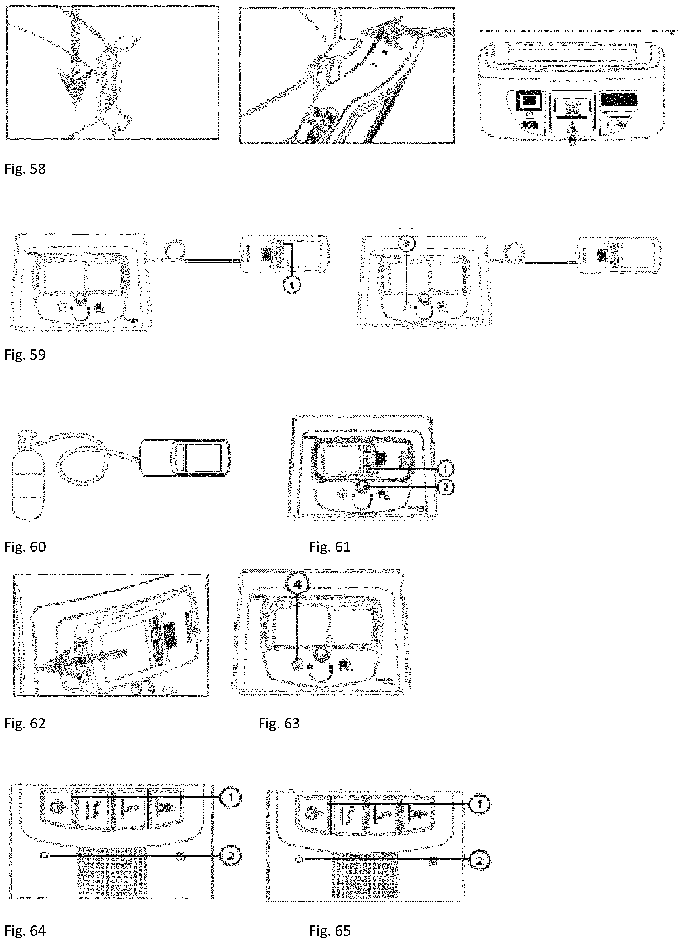

1 Position the clip over the belt, and push down until it is secure (see Fig. 58).
2 Line up the belt clip with the belt clip sockets on the ventilator. Push in the ventilator towards the belt clip until the ventilator audibly clicks into place (see Fig. 58).

POLE MOUNT

The ventilator may also be secured with the use of an optional pole mount. For more information, see "Pole Mount" on page 48.

VENTILATOR SILENCE ALARM BUTTON

Silencing and clearing alarms is a multi-step process that depends on alarm priority and how many alarms are active. For more information see "Chapter 7: Alarms, Alerts, and Troubleshooting".

1 Silence Alarm button on ventilator (see Fig. 58).
Press the Silence Alarm button to temporarily silence the alarm for 60 seconds. Pressing the Silence Alarm button silences only one alarm at a time-in audible or vibrating alarm mode. If more than one alarm occurs, press the Silence Alarm button once for each alarm. If the alarm is a medium- or high-priority alarm and is not silenced after 60 seconds, the alarm will continue with an additional buzzer.
2 Resolve the condition that triggered the alarm. For help resolving alarms, see the alarm and troubleshooting tables in "Chapter 7: Alarms, Alerts, and Troubleshooting" for possible causes of an alarm and options to resolve it. If a Silence Alarm button is pressed but the condition that triggered the alarm is not resolved, the alarm will sound again after 60 seconds.
3 After resolving a High Temperature, High Circuit Pressure, or High PEEP Pressure high-priority alarm, touch OK in the message that indicates the alarm has been resolved.

POWERING OFF SEQUENCE IN EXTENDED RANGE MODE

Fig. 59

1 To power off the ventilator, press the Ventilator Power button for three seconds until a confirmation screen appears.
2 To continue to power off the ventilator, choose CONFIRM.
NOTE: If a selection is not made within 20 seconds or if the BACK button is selected, the previous screen will be displayed and the ventilation status will not be affected.

(Fig. 59)

4 To power off the compressor, press the Compressor Power button.

DISCONNECTING EXTENDED@RANGE CONFIGURATION

1 Power off the ventilator.
2 Disconnect the oxygen hose from the ventilator by pulling back on the knurled ring on the quick connect end of the oxygen hose until the oxygen hose detaches from the ventilator.
3 Disconnect the oxygen hose from the compressor by unscrewing the DISS connection on the oxygen hose from the DISS 1240 output on the compressor.
4 Store the oxygen hose for future use.

INTRODUCTION TO STAND@ALONE CONFIGURATION (Fig. 60)

This section provides instructions on connecting the Life2000TM Ventilator to an alternate pressure source (50-PSI, t 40 LPM at 41 PSI), such as an air or oxygen gas cylinder by using an oxygen hose or optional air hose.

For connection to other sources, such as wall connections, follow your facility's procedures.

NOTES:

- The ventilator is compatible with medical grade compressed air or oxygen. Contact your local provider for more information.
- If not connected to AC power, make sure the ventilator battery has sufficient charge for your length of use.

TESTING THE VENTILATOR

In a multi-patient setting, the ventilator must be tested before it is assigned to a new patient. For instructions on testing the ventilator, see "Testing Ventilator Alarms".

UNDOCKING THE VENTILATOR FROM THE COMPRESSOR

Fig. 61

1 Power off the ventilator.
NOTE: Alarms may be encountered and/or the Prescription Setting might be inadvertently changed if the ventilator is not powered off before undocking.
2 Turn the locking knob counterclockwise to the unlocked position until the compressor ejects the ventilator.
NOTE: Some tension will be encountered while turning during ejection.
3 Pull the ventilator as shown in Fig 62 to separate it from the compressor cradle.
4 Power off the compressor (Fig. 63).

NOTE: For docking instructions, see "Docking the Ventilator into the Compressor" on page 18.

POWERING ON SEQUENCE FOR THE VENTILATOR

Fig. 64

1 Power on the ventilator by pressing the power button for the ventilator.
2 The green power indicator light on the ventilator shows that the ventilator is on.
3 When the startup screen is displayed, listen for audible tones to test the alarm speaker.
During the ventilator's start up sequence, the ventilator will perform a self test. During the test, all ventilator indicator lights should briefly flash and an audible alarm should briefly sound. This self test can take up to 15 seconds to complete. If you do not hear tones when you turn on the ventilator, contact your Breathe Technologies service representative.
4 When the Home screen is displayed, the touch screen is ready to use. The Home screen will display Air or O2 based on the option prescribed and selected.

NOTE: Ventilation will not begin until a Prescription Setting button is selected. For more information see "Choosing an Activity Button (Patient-Selectable)" on page 78.

CHECKING THE VENTILATOR BATTERY CHARGE

If you intend to use the ventilator on battery power, make sure that the battery is sufficiently charged for your duration of use.

It takes approximately three to four hours to fully recharge the battery whether the ventilator is off or on. A fully charged battery should last up to four hours.

Fig. 65

1 Press the Power button to turn on the ventilator.
2 The green power indicator light shows that the ventilator is on.
3 Check the Ventilator Battery Charge icon on the touch screen to see the current battery charge level for the ventilator. Refer to the chart on the following page to determine the approximate amount of ventilator battery charge and the approximate time remaining.

NOTE: The Ventilator Battery Charge icon is always displayed on the touch screen during normal operation.

VENTILATOR BATTERY CHARGE ICONS, MEANING, AND APPROXIMATE TIME REMAINING

Fig 66

[0152]   * Very low battery alarm will sound with less than 15% charge.
† Low battery alarm will sound with less than 25% charge.

ASSEMBLING THE VENTILATOR BATTERY CHARGER AND CHARGING THE VENTILATOR

Fig 67

1 Plug the ventilator AC power cord into the ventilator battery charger.
2 Connect the ventilator AC plug into an AC power source.
3 The ventilator battery charger indicator light will turn green or red when connected to AC power.

NOTE: The ventilator can be used while the battery is charging.

(Fig 68)

4 Connect the ventilator battery charger cord to the ventilator battery charger connection port. The word UP on the battery charger cord will be on top.
5 If powered on, check the Ventilator Battery Charge icon on the touch screen to see the ventilator's current battery charge status.

NOTES:

• When charging the battery, there may be a 5-10 second delay before the Ventilator Battery Charge icon appears on the touch screen.
• The ventilator performance is the same regardless of power source (internal battery or AC).

CAUTION:

• Do not place the battery charger on wet surfaces or use in wet environments. Wet environments may damage the battery charger and may cause electric shock.
• Use only the Breathe Technologies approved battery charger and cord set with the ventilator. If an

unauthorized battery charger or cord set is used with the ventilator, the ventilator may be damaged.

SECURING THE VENTILATOR

BELT CLIP

You can attach the ventilator to a belt or waistband using the included belt clip. The ventilator can be worn on either the right or left side.

CAUTION:

•   Make sure the clip is securely fastened to the belt and the ventilator. If the clip is not securely fastened to the belt and the ventilator, the ventilator may fall and be damaged.
•   Always secure the ventilator to prevent it from falling or becoming damaged.
•   Use only the Breathe Technologies approved belt clip with the ventilator.

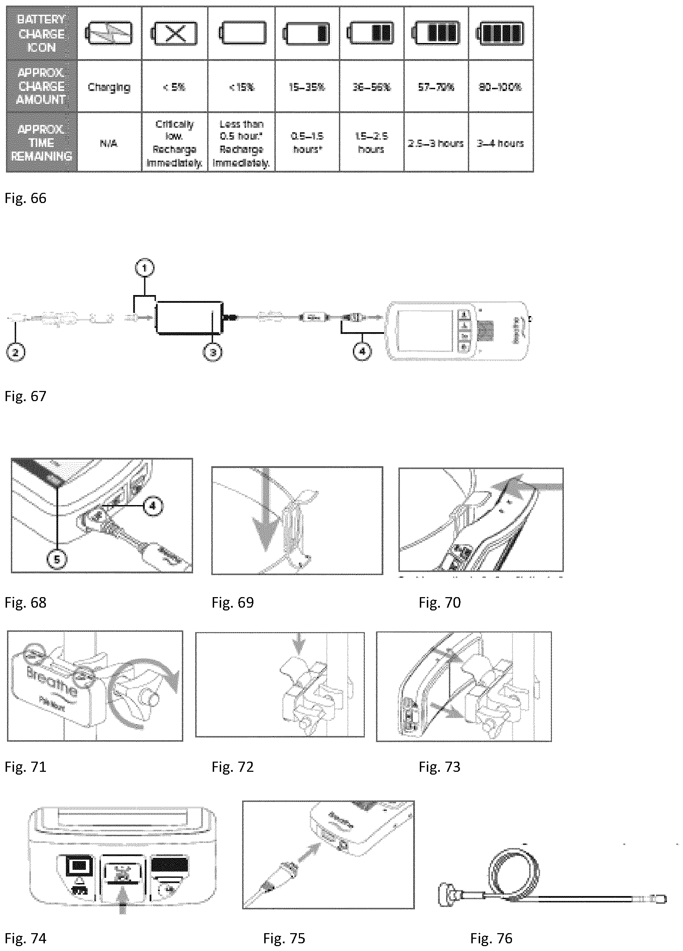

    1 Position the clip over the belt, and push down until it is secure (Fig 69).
    2 Line up the belt clip with the belt clip sockets on the ventilator. Push in the ventilator towards the belt clip until the ventilator audibly clicks into place (Fig 70).

POLE MOUNT

You can attach the ventilator to a pole using an optional pole mount. For information about ordering accessories and replacement parts, see "Accessories and Replacement Parts" on page 141.

CAUTION:

•   Make sure the pole mount is securely attached to the pole, and the ventilator and clip are securely fastened to the pole mount and the ventilator. If the clip is not securely attached to the pole mount and the ventilator, the ventilator may fall and be damaged.
•   Always secure the ventilator to prevent it from falling or becoming damaged.
•   Use only the Breathe Technologies approved belt clip and pole mount with the ventilator.

    1 Position the pole mount around the pole in the correct orientation (see Fig 71).
    2 Turn the knob on the pole mount until the pole mount is securely attached to the pole (see Fig. 71).
    3 Slide the belt clip for the ventilator into the hole on the top of the pole mount and push down until it is secure (Fig 72).
    4 Attach the ventilator to the belt clip on the pole mount by lining up the belt clip with the belt clip sockets on the ventilator. Push the ventilator in towards the vent clip and pole mount until the ventilator audibly clicks into place (Fig. 73).

VENTILATOR SILENCE ALARM BUTTON

Silencing and clearing alarms is a multi-step process that depends on alarm priority and how many alarms are active. For more information see "Chapter 7: Alarms, Alerts, and Troubleshooting".

    1 Silence Alarm button on ventilator (see Fig. 74).
    Press the Silence Alarm button to temporarily silence the alarm for 60 seconds. Pressing the Silence Alarm button silences only one alarm at a time-in audible or vibrating alarm mode. If more than one alarm occurs, press the Silence Alarm button once for each alarm. If the alarm is a medium- or high-priority alarm and is not silenced after 60 seconds, the alarm will continue with an additional buzzer.
    2 Resolve the condition that triggered the alarm. For help resolving alarms, see the alarm and troubleshooting tables in "Chapter 7: Alarms, Alerts, and Troubleshooting" for possible causes of an alarm and options to resolve it. If a Silence Alarm button is pressed but the condition that triggered the alarm is not resolved, the alarm will sound again after 60 seconds.

3 After resolving a High Temperature, High Circuit Pressure, or High PEEP Pressure high-priority alarm, touch OK in the message that indicates the alarm has been resolved.

## CONNECTING AN INTERFACE TO THE VENTILATOR IN STAND@ALONE CONFIGURATION

Plug the Pillows Interface or Universal CircuitTM interface into the interface port on the bottom of the ventilator until it clicks (Fig 75). For more information about wearing interfaces, see "Chapter 5: Connecting an Interface".

NOTE: Ensure that the Universal CircuitTM interface or Pillows Interface is connected to the ventilator in Extended Range Configuration and Stand-alone Configuration.

## CONNECTING TO A CYLINDER

If not using the regulator provided with the Life2000TM Ventilation System, ensure that the gas regulator meets the

requirements below and is appropriate for the cylinder being used.

Table 2

| REGULATOR REQUIREMENTS | |
|---|---|
| Pressure output | 41-87 PSI [50 PSI nominal] |
| Pressure flow | $\geq$ 40 LPM at 41 PSI |
| Pressure friting | DISS 1240 for use with an oxygen hose and cylinder |
| | DISS 1160-A for use with an air hose and cylinder |
| Flow friting | Barb |

WARNING:

- If the ventilator is not used with a regulator capable of 41 PSI to 87 PSI (nominal 50 PSI) with greater than 40 LPM capability, patients may not receive appropriate respiratory therapy.
- The provided oxygen regulator should not be used with a cylinder rated at 3000 PSI or higher.
- Use the ventilation system with only approved medical compressed gas (oxygen or dry air). Use with nonapproved sources of gas may cause the ventilation system to malfunction and the patient may not receive appropriate respiratory therapy.
- Use only a Breathe Technologies approved oxygen or air hose with the ventilation system. If an unauthorized oxygen or air hose is used with the ventilation system, the system may be damaged.
- Do not use the Breathe Technologies Life2000TM Ventilation System with oxygen in the presence of flammable anesthetics such as fluroxene, cyclopropane, divinyl ether, ethyl chloride, ethyl ether, and ethylene, as they may form flammable or explosive mixtures with oxygen.
- Before beginning ventilation therapy, verify that there is an adequate supply of source gas for the intended duration of the therapy. Otherwise, the patient may not receive appropriate therapy.
- Do not allow smoking near oxygen sources or near the ventilation system and do not place oxygen sources or the ventilation system near any source of direct heat or open flame because flammable materials burn more readily in the presence of oxygen.
- Do not use the ventilator in the presence of flammable anesthetics.

TIPS:

- Make sure your source gas supply is sufficient for your length of use.
- If not connected to AC power, make sure the ventilator battery has sufficient charge for your length of use.
- Refer to the regulator and source gas supply manufacturers' instructions for more information.

- During use in Stand-alone Configuration, the oxygen hose should remain connected to the ventilator at all times, except when required to be disconnected for maintenance, testing, or replacement. If it is disconnected while the ventilator is on and delivering therapy, a Low Gas Pressure alarm will occur. For more information see "Low Gas Pressure" on page 103.
- The Life2000TM Ventilation System can work using most common medical grade gas cylinders when using the appropriately-rated regulator. Ensure the appropriate regulator for the cylinder is being used before connecting to the ventilator. Information about the gas cylinder and its specific regulator requirements may be found by contacting the gas cylinder supplier.

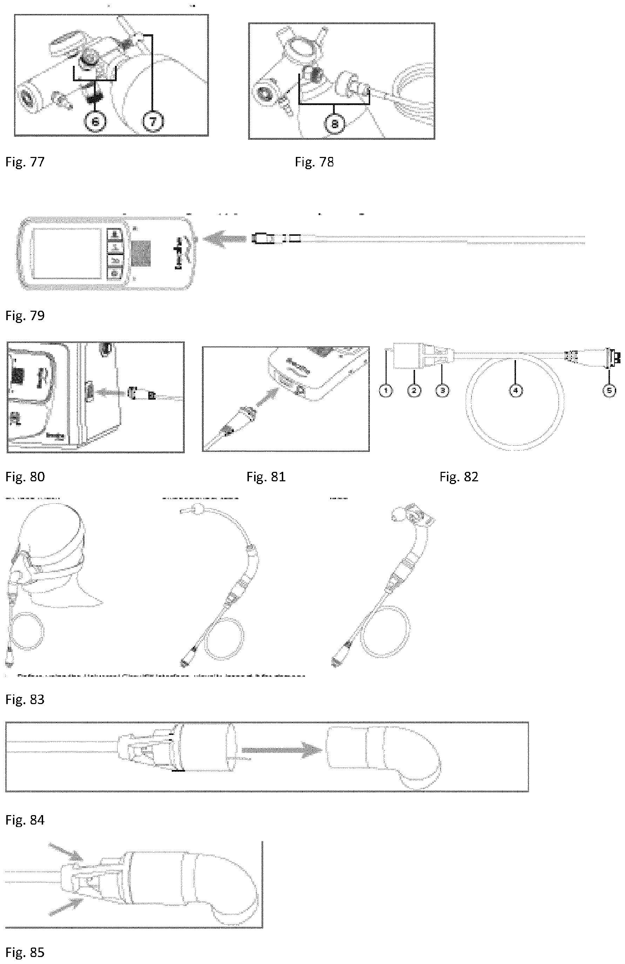

1 Choose the appropriate hose for the source gas cylinder you will use.
NOTE: A 6 ft. oxygen hose and a 50 ft. oxygen hose are included with the ventilation system and are compatible with oxygen cylinders. Connection to an air cylinder requires an optional air hose. For information about ordering accessories and replacement parts, see "Accessories and Replacement Parts" (Fig. 76).
2 Visually inspect the oxygen or air hose for damage before using it.
3 Ensure the ventilator is powered off.
4 Undock the ventilator from the compressor. For more information see "Undocking the Ventilator from the Compressor" on page 43.
5 Use the oxygen regulator provided with the ventilation system; or if using another regulator, ensure that the outlet flow is t40 LPM at 41 PSI and that the regulator is preset to 50 PSI [or adjust the regulator pressure to 41 PSI to 87 PSI (50 PSI nominal)].
6 Slide the regulator over the neck of the cylinder, and line up the pins on the regulator with the holes in the cylinder neck (Fig. 77).
7 Tighten the tee screw on the regulator by turning the handle clockwise (Fig. 77).
8 Connect the oxygen or air hose to the DISS connector end of the regulator by turning it clockwise (Fig. 78).
NOTE: To conserve your gas supply, the barbed outlet flow regulator should be set to 0 or OFF during use.
9 Turn on the gas supply according to the regulator and gas supply manufacturers' instructions (Fig. 79).
NOTE: Make sure your source gas supply is sufficient for your length of use.
10 Connect the other end of the oxygen hose to the ventilator by pushing the small quick connect end onto the gas inlet connection on the ventilator; when connected, the quick connect end will click into place.

REPLACING THE SOURCE GAS CYLINDER

When the source gas cylinder needs to be replaced:

1 Place the patient on an alternate means of ventilation, if necessary.
2 Power off the ventilator.
NOTE: Alarms may be encountered and/or the Prescription Setting might be inadvertently changed if the ventilator is not powered off before replacing the cylinder.
3 Turn off gas supply according to the regulator and gas supply manufacturers' instructions.
4 Remove the regulator and attached gas hose from the old cylinder by turning the handle counterclockwise.
5 Slide the regulator over the neck of the new cylinder, and line up the pins on the regulator with the holes in the cylinder neck. Tighten the tee screw on the regulator by turning the handle clockwise. (The gas supply hose should still be connected to the regulator.)
6 Turn on gas supply according to the regulator and gas supply manufacturers' instructions.
7 Power on the ventilator.

NOTE: Ventilation will not begin until a Prescription Setting button is selected. For more information see "Choosing an Activity Button (Patient-Selectable)" on page 78.

CYLINDER DURATION INFORMATION

The duration of compressed medical oxygen and air cylinders depends on the volume of the cylinder and the breathing pattern of each patient, which can change throughout the day. Observe your daily oxygen consumption a few times before estimating typical use. The following tables can be used to obtain approximate values only.
NOTE: These tables use a PEEP value of 0 cmH2O.

CYLINDER SIZE B: 164 LITERS (M6)

| Volume (ml) | BREATHS PER MINUTE (BPM) | | | | | | | | |
| --- | --- | --- | --- | --- | --- | --- | --- | --- | --- |
| | 12 | 14 | 16 | 18 | 20 | 22 | 24 | 26 | 28 |
| | Duration in hours | | | | | | | | |
| 50 | 4.6 | 3.9 | 3.4 | 3.0 | 2.7 | 2.5 | 2.3 | 2.1 | 2.0 |
| 100 | 2.3 | 2.0 | 1.7 | 1.5 | 1.4 | 1.2 | 1.1 | 1.1 | 1.0 |
| 150 | 1.5 | 1.3 | 1.1 | 1.0 | 0.9 | 0.8 | 0.8 | 0.7 | 0.7 |
| 200 | 1.1 | 1.0 | 0.9 | 0.8 | 0.7 | 0.6 | 0.6 | 0.5 | 0.5 |
| 250 | 0.9 | 0.8 | 0.7 | 0.6 | 0.5 | 0.5 | 0.5 | 0.4 | 0.4 |
| 500 | 0.5 | 0.4 | 0.3 | 0.3 | 0.3 | 0.2 | 0.2 | 0.2 | 0.2 |
| 750 | 0.3 | 0.3 | 0.2 | 0.2 | 0.2 | 0.2 | 0.2 | 0.1 | 0.1 |

Table 3

CYLINDER SIZE D: 425 LITERS (M15)

| Volume (ml) | BREATHS PER MINUTE (BPM) | | | | | | | | |
| --- | --- | --- | --- | --- | --- | --- | --- | --- | --- |
| | 12 | 14 | 16 | 18 | 20 | 22 | 24 | 26 | 28 |
| | Duration in hours | | | | | | | | |
| 50 | 11.8 | 10.1 | 8.9 | 7.9 | 7.1 | 6.4 | 5.9 | 5.4 | 5.1 |
| 100 | 5.9 | 5.1 | 4.4 | 3.9 | 3.5 | 3.2 | 3.0 | 2.7 | 2.5 |
| 150 | 3.9 | 3.4 | 3.0 | 2.6 | 2.4 | 2.1 | 2.0 | 1.8 | 1.7 |
| 200 | 3.0 | 2.5 | 2.2 | 2.0 | 1.8 | 1.6 | 1.5 | 1.4 | 1.3 |
| 250 | 2.4 | 2.0 | 1.8 | 1.6 | 1.4 | 1.3 | 1.2 | 1.1 | 1.0 |
| 500 | 1.2 | 1.0 | 0.9 | 0.8 | 0.7 | 0.6 | 0.6 | 0.5 | 0.5 |
| 750 | 0.8 | 0.7 | 0.6 | 0.5 | 0.5 | 0.4 | 0.4 | 0.4 | 0.3 |

Table 4

CYLINDER SIZE E: 660 LITERS (M24)

| Volume (ml) | BREATHS PER MINUTE (BPM) | | | | | | | | |
| --- | --- | --- | --- | --- | --- | --- | --- | --- | --- |
| | 12 | 14 | 16 | 18 | 20 | 22 | 24 | 26 | 28 |
| | Duration in hours | | | | | | | | |
| 50 | 18.3 | 15.7 | 13.8 | 12.2 | 11.0 | 10.0 | 9.2 | 8.5 | 7.9 |
| 100 | 9.2 | 7.9 | 6.9 | 6.1 | 5.5 | 5.0 | 4.6 | 4.2 | 3.9 |
| 150 | 6.1 | 5.2 | 4.6 | 4.1 | 3.7 | 3.3 | 3.1 | 2.8 | 2.6 |
| 200 | 4.6 | 3.9 | 3.4 | 3.1 | 2.8 | 2.5 | 2.3 | 2.1 | 2.0 |
| 250 | 3.7 | 3.1 | 2.8 | 2.4 | 2.2 | 2.0 | 1.8 | 1.7 | 1.6 |
| 500 | 1.8 | 1.6 | 1.4 | 1.2 | 1.1 | 1.0 | 0.9 | 0.8 | 0.8 |
| 750 | 1.2 | 1.0 | 0.9 | 0.8 | 0.7 | 0.7 | 0.6 | 0.6 | 0.5 |

Table 5

For other cylinder sizes, use the following gas usage chart to estimate your cylinder duration.

GAS USAGE (LPM) CHART

| | BREATHS PER MINUTE (BPM) | | | | | | | | |
|---|---|---|---|---|---|---|---|---|---|
| | 12 | 14 | 16 | 18 | 20 | 22 | 24 | 26 | 28 |
| Volume (ml) | Gas Usage (LPM) | | | | | | | | |
| 50 | 0.6 | 0.7 | 0.8 | 0.9 | 1.0 | 1.1 | 1.2 | 1.3 | 1.4 |
| 100 | 1.2 | 1.4 | 1.6 | 1.8 | 2.0 | 2.2 | 2.4 | 2.6 | 2.8 |
| 150 | 1.8 | 2.1 | 2.4 | 2.7 | 3.0 | 3.3 | 3.6 | 3.9 | 4.2 |
| 200 | 2.4 | 2.8 | 3.2 | 3.6 | 4.0 | 4.4 | 4.8 | 5.2 | 5.6 |
| 250 | 3.0 | 3.5 | 4.0 | 4.5 | 5.0 | 5.5 | 6.0 | 6.5 | 7.0 |
| 500 | 6.0 | 7.0 | 8.0 | 9.0 | 10.0 | 11.0 | 12.0 | 13.0 | 14.0 |
| 750 | 9.0 | 10.5 | 12.0 | 13.5 | 15.0 | 16.5 | 18.0 | 19.5 | 21.0 |

Table 6

NOTE: This table uses a PEEP value of 0 cmH2O.

For other cylinder sizes or partially-filled cylinders, the following equation can be used in conjunction with the above gas usage table to estimate cylinder duration.

$$\text{cylinder duration} = \frac{\left(\frac{P_T V_T}{14.7}\right)}{\text{gas usage}}$$

Where:

$P_T$ = cylinder pressure (typically 2200 PSI for full cylinder)
$V_T$ = empty cylinder volume (4.5L for an E cylinder).

THE UNIVERSAL CIRCUITTM INTERFACE AND THE PILLOWS INTERFACE

The Breathe Technologies Life2000TM Ventilation System requires the use of either Breathe's Universal CircuitTM interface or Breathe's Pillows Interface. Breathe Technologies interfaces are only compatible with Breathe Technologies ventilators and compressors.
The Universal CircuitTM interface is used to connect any commercially available non-invasive mask (full face, nasal, or pillows) or tracheostomy tube to the ventilation system.
The Pillows Interface is a non-invasive patient mask that connects directly to the ventilation system.

NOTES:

• For information about ordering accessories and replacement parts, see "Accessories and Replacement Parts".
• Before using an interface, visually inspect it for damage.

CONNECTING AN INTERFACE TO THE COMPRESSOR IN STATIONARY CONFIGURATION

Fig. 80

Plug the Universal CircuitTM interface or Pillows Interface into the interface connection on the side of the compressor until it clicks.

CONNECTING AN INTERFACE TO THE VENTILATOR IN EXTENDED RANGE OR STAND@ALONE CONFIGURATION (Fig. 81)

Plug the Pillows Interface or Universal CircuitTM interface into the interface connection on the bottom of the ventilator until it clicks.

THE UNIVERSAL CIRCUITTM INTERFACE

The Breathe Technologies Life2000TM Ventilation System can be used with all commercially available non-invasive masks (full face, nasal, and pillows) or tracheostomy tubes for invasive ventilation using the Universal CircuitTM interface.
NOTE: The interface assembly is packaged clean but not sterile. The Universal CircuitTM interface does not need to be cleaned or sterilized prior to first use.

Fig 76

1 Sense tube
2 Universal CircuitTM interface connector (patient side)
3 Entrainment ports
4 Interface tubing
5 Compressor or ventilator connector

WARNING:

Breathe interfaces are designed for single-patient use. To prevent risk of cross-contamination use a new Universal CircuitTM interface for each new patient. For the third-party mask, refer to the user guide provided by the manufacturer.

CAUTION:

Use the Universal CircuitTM interface for a maximum of 30 days. If an interface is used for more than 30 days, its performance may degrade and the patient may not receive adequate respiratory therapy.

1 Before using the Universal CircuitTM interface, visually inspect it for damage.

CAUTION:

Do not use a Universal CircuitTM interface that is cracked, odorous, broken, or kinked. If a damaged interface is used, the patient may not receive adequate respiratory therapy.
CONNECTING THE UNIVERSAL CIRCUITTM INTERFACE TO ET TUBES OR MASKS THE BREATHE PILLOWS INTERFACE
The Universal CircuitTM
Interface with a
full face mask (See Fig. 83, left)
The Universal CircuitTM
interface with a tracheal
tube (see Fig. 83, right)
The Universal CircuitTM
interface with an
endotracheal tube (see Fig. 83, middle)

1 Before using the Pillows Interface, visually inspect it for damage.
CAUTION:
Do not use a Pillows Interface that is cracked, odorous, broken, or kinked. If a damaged interface is used,the

patient may not receive adequate respiratory therapy.

2 Connect the Universal Circuit™ interface to the mask or tracheostomy tube, ensuring that the sense tube does not become kinked or pinched (Fig 84).

3 Ensure that the entrainment ports are clear of any obstruction and are not covered by any clothing or bedding (Fig. 85).

The interface comes in four sizes: extra small, small, medium, and large.

NOTE: The interface assembly is packaged clean but not sterile. The Pillows Interface does not need to be cleaned or sterilized prior to first use.

(Fig. 86)

1 Nasal pillows (patient side)
2 Entrainment ports
3 Tube fit adjuster (cinch)
4 Interface tubing
5 Compressor or ventilator connector

WARNING:

- Properly secure the patient interface to the face and route tubing around the ears to avoid strangulation.
- Breathe interfaces are designed for single-patient use. To prevent risk of cross-contamination use a new Pillows Interface for each new patient.

CAUTION:

Use the Pillows Interface for a maximum of 30 days. If an interface is used for more than 30 days, its performance may degrade and the patient may not receive adequate respiratory therapy.

TIP:

When the interface is in use, periodically check that it is positioned correctly and make adjustments as required. If a patient's skin becomes irritated, replace or discontinue using the interface.

WEARING THE PILLOWS INTERFACE

Fig. (87)

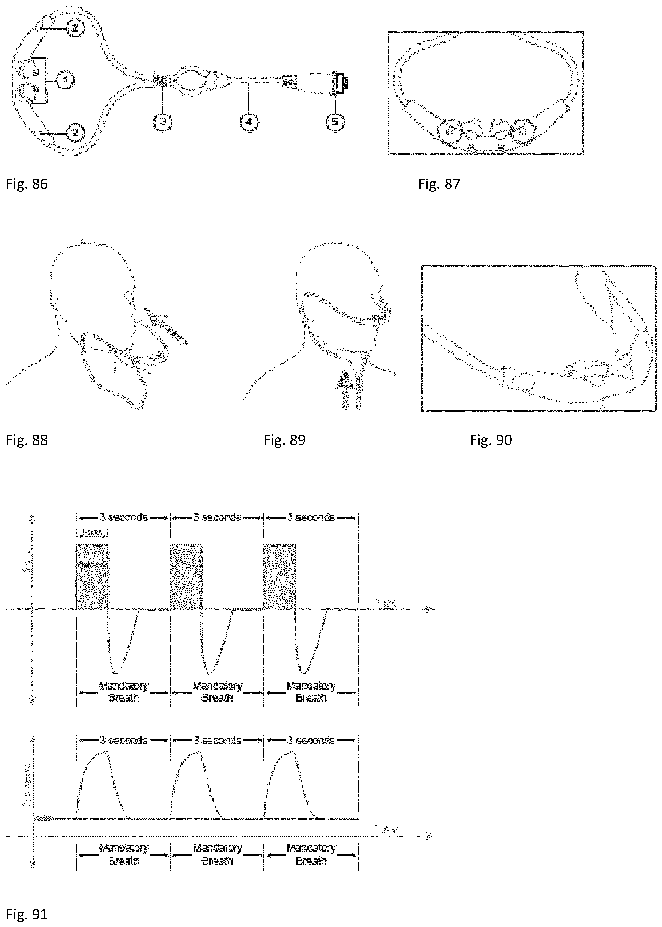

1 Place the interface in front of the patient with the arrows underneath pointing up and the curve of the interface towards the patient's face (Fig. 88).

2 Loop the interface tubing over the ears so the pillows of the interface are positioned snugly inside the nostrils (Fig. 89).

3 Using the tube fit adjuster (cinch), adjust the tubing length under the chin so that the interface is secured snugly and comfortably.

CHECKING THE PILLOWS INTERFACE POSITIONING

The interface is placed correctly when:

- The interface pillows rest snugly inside the nostrils, as shown in Fig. 90.
- The fit is comfortable.
- The interface does not make breathing difficult.

- Air does not flow to the eyes, cheeks, or lips.
- Entrainment ports are not obstructed.

If any one of these conditions is not met, reposition the interface. If problems persist, try a different interface size.

INTRODUCTION TO VENTILATION SETTINGS

This chapter explains the touch screen display and user interface used to view, edit, and input therapy-related parameters. To use the touch screen, simply touch a button or an area of the screen you want to make active. An audible "click" indicates the feature you touch is activated.

HOME SCREEN

When the ventilator is powered on, it completes a self test and then displays the Home screen. This screen indicates that the ventilator is ready for use.
When a Prescription Setting button is selected, the Home screen will display the breath rate (BPM), Peak Inspiratory Pressure (PIP), and gas flow rate (LPM).

1 The Wrench button is used to go to the Menu screen.
2 The current Prescription Setting icon and output volume (displayed on the Home screen during ventilation).
3 The Flip button flips the screen 180°.
4 Peak Inspiratory Pressure (PIP) indicator (displayed on the Home screen during ventilation).
5 Current breaths per minute (BPM) (displayed on the Home screen during ventilation).
6 Average gas flow (Air or O2) in liters per minute (LPM) based on Prescription Setting and patient's current breath rate (displayed on the Home screen during ventilation).
NOTE: Your Home screen will display Air or O2 based on the option prescribed.
7 Ventilator Battery Charge icon.
8 Current Prescription Setting icon (displayed during ventilation).
9 The Vibration icon indicates that the ventilator is in vibration mode.
10 Time and date.

62 63

MOVING BETWEEN THE HOME SCREEN AND MENU SCREEN

1 Touch the Wrench button to go to the Menu screen.

2 Touch the Home Screen button to go to the Home screen.

MENU SCREEN

Use the Menu screen to go to the Settings menu, get information about the ventilator's software version and total operating time, or go back to the Home screen.
To get to the Menu screen, touch the Wrench button from any screen.

1 Screen title.
2 Touch to go to the Home screen.
3 Touch to go to the Settings screen for Trigger Sensitivity, Clinician's Settings, and Utilities.
4 Touch to go to the Information screen.

TOUCH SCREEN ENERGY@SAVE MODE

After two minutes with no user interaction, the touch screen automatically enters energy-save mode and dims the screen. Touching the screen again will reactivate it and display the Home screen.

DEFINING CLINICAL SETTINGS

ACCESSING THE SETTINGS MENU

1 On any screen, touch the Wrench button.

2 On the Menu screen, touch Settings.

ACCESSING THE CLINICIAN'S SETTINGS MENU

1 On the Settings Menu screen, touch Clinician's Settings.
NOTE: Access to the Clinician's Settings menu is restricted to trained clinical personnel.
2 On the Clinician Password screen, enter the password.
Password: 4930
3 Touch OK to access the Clinician's Settings.

DISABLING ACCESS TO THE CLINICIAN'S SETTINGS MENU

There are two ways to disable access to the Clinician's Settings menu to prevent unintended changes to the programmed clinical settings.

AUTOMATIC TIMEOUT

An Automatic Timeout disables access to the Clinician's Settings menu after five minutes of inactivity in the Clinician's Settings menu.
The password must be re-entered to regain access to the Clinician's Settings menu. NOTE: Automatic Timeout begins after the Touch Screen Energy-Save Mode. For more information, see "Touch Screen Energy-Save Mode".

POWERING OFF

Powering off the ventilator will also disable access to the Clinician's Settings menu; when the ventilator is powered on again, the password must be re-entered to access the Clinician's Settings menu.

VIEWING AND EDITING PRESCRIPTION SETTINGS

The buttons on the Prescription Settings screen correspond to the Activity buttons on the ventilator.

Prescription Settings

1 Press the prescription setting button representing the Activity button you want to view or edit.
2 Check the box below a Prescription Setting button to make the button active (checked) or inactive (unchecked). Only active Prescription Setting buttons are available to the patient as therapy options.

NOTES:

• The BACK button on the Prescriptions Setting screen is used to return to the Settings Menu screen.
• At least one Prescription Setting must always be active.

TIP:

The ventilator is shipped with factory-set default values. Be sure to adjust the Prescription Settings based on a physician's order.

> 3 The icon at the top of the screen indicates the Prescription Setting that is being viewed or edited.
> 4 For each of the three Prescription Settings that may be made available to the patient, set up Ventilation Settings, Alarm Limits, Breath Timeout, and Source Gas parameters according to a physician's order.
> 5 If setting up more than one Prescription Setting, after setting up all the parameters for one Prescription Setting, use the BACK button to return to the Prescription Settings screen to set up additional Prescription Settings.

NOTE: Viewing or editing a Prescription Setting or making a Prescription Setting button active does not begin ventilation therapy. For more information, see "Choosing an Activity Button (Patient-Selectable)" on page 78.

66 67

FACTORY DEFAULT PRESCRIPTION SETTINGS

The following table lists the Life2000TM factory default Prescription Settings that may be edited. For a full list of factory defaults, see the "Summary of Factory Default Settings".

| DESCRIPTION | DEFAULT VALUE | | |
| --- | --- | --- | --- |
| | LOW ACTIVITY | MEDIUM ACTIVITY | HIGH ACTIVITY |
| Ventilation Settings | | | |
| Volume | 100 ml | 150 ml | 190 ml |
| I-Time (Inspiratory Time) | 750 ms | | |
| PEEP (Positive End Expiratory Pressure) | 0 cmH$_2$O | | |
| Sensitivity (Trigger Sensitivity) | 4* | | |
| BR (Breath Rate) | 12 BPM* | | |
| * Therapy mode: Assist/Control ventilation mode | | | |
| Alarm Limits | | | |
| High BR (Breath Rate) alarm limit | 50 BPM | | |
| Low BR (Breath Rate) alarm limit | 5 BPM | | |
| High PIP (Peak Inspiratory Pressure) alarm limit | 20 cmH$_2$O | | |
| Low PIP (Peak Inspiratory Pressure) alarm limit | 1 cmH$_2$O | | |
| Breath Timeout | | | |
| Breath Timeout Action | 12 BPM | | |
| Breath Timeout Period | 60 seconds | | |
| Source Gas | | | |
| Source Gas | Air | | |

Table 7

NOTE: Each Prescription Setting is independent of other Prescription Settings.

The following factory default cannot be edited:

| DESCRIPTION | DEFAULT VALUE |
|---|---|
| High PEEP (Positive End Expiratory Pressure) Pressure alarm limit | +7 cmH₂O (above PEEP setting) |

Table 8

BREATH TYPES

There are two breath types that apply to the Volume Control ventilation provided by the ventilation system:

- Mandatory
- Assisted

MANDATORY BREATH

A mandatory breath (or machine breath) is completely controlled by the ventilation system. The system controls both the beginning (triggering) and end (cycling) of the inspiratory phase.

ASSISTED BREATH

An assisted breath is controlled by both the patient and the ventilation system. Breaths are initiated by the patient's effort and volume delivery is controlled by the prescribed volume setting and inspiratory time.

THERAPY MODES

The ventilation system delivers an inspired tidal volume to the patient according to the clinical settings: volume, breath rate, trigger sensitivity, PEEP, and inspiratory time. Three different volume control modes are available:

- Control
- Assist/Control
- Assist

VENTILATION SETTINGS AND THERAPY MODES

The Ventilation Settings screen provides options to set different therapy modes. Consult the following table to adjust parameters accordingly:

| THERAPY MODE SETTINGS* | SET TRIGGER SENSITIVITY TO: | SET BREATH RATE TO: |
|---|---|---|
| Control ventilation mode | OFF | ≥1 |
| Assist/Control ventilation mode | 0 to 9 | ≥1 |
| Assist ventilation mode | 0 to 9 | 0 |
| | | * SETTINGS BASED ON PRESCRIPTION |

Table 9

SETTING VENTILATION PARAMETERS IN CONTROL VENTILATION MODE

In this mode, the ventilation system delivers volume control therapy only for mandatory breaths. A mandatory breath is delivered according to the breath setting (BPM). This also means that a breath will not be triggered based on patient's inspiratory effort.

(see Fig. 91)

To set a Prescription Setting for Control Ventilation Mode, the following parameters need to be set according to the table below:

| THERAPY MODE SETTINGS* | SET TRIGGER SENSITIVITY TO: | SET BREATH RATE TO: |
|---|---|---|
| Control Ventilation Mode | OFF | ≥ 1 |
| | | * SETTINGS BASED ON PRESCRIPTION |

Table 10

To set the Prescription Setting:

1 On the Clinician's Setting screen, touch Ventilation Settings.
2 On the Ventilation Settings screen, touch the box beside the setting you want to change.
3 Touch the Up arrow to increase the value in the box or the Down arrow to decrease it. If you press and hold an arrow, the value automatically increases or decreases.
4 Repeat steps 2 and 3 for each setting you want to change, and then press OK.
5 In the message asking if the settings are OK, touch CONFIRM.

NOTE: Changes to settings only take effect when you touch CONFIRM.

SETTING VENTILATION PARAMETERS IN ASSIST/CONTROL VENTILATION MODE

In this mode, the ventilation system provides Tidal Volume during inhalation for assisted and mandatory breaths.
An assisted breath is started when there is patient effort, but it is ended when the Inspiratory Time setting has been met. A mandatory breath is delivered if the patient does not breathe within the prescribed Breath Rate setting. This ensures that the patient receives a minimum number of breaths per minute.

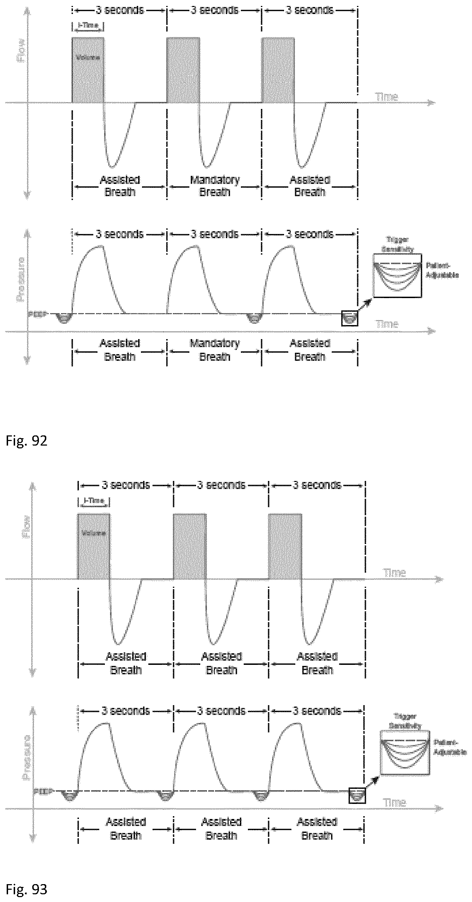

(see Fig. 92)

To set a Prescription Setting for Assist/Control ventilation mode, the following parameters need to be set according to the table below:

| THERAPY MODE SETTINGS* | SET TRIGGER SENSITIVITY TO: | SET BREATH RATE TO: |
|---|---|---|
| Assist/Control ventilation mode | 0 to 9 | ≥ 1 |
| | | * SETTINGS BASED ON PRESCRIPTION |

Table 11

To set the Prescription Setting:

1 On the Clinician's Setting screen, touch Ventilation Settings.
2 On the Ventilation Settings screen, touch the box beside the setting you want to change.
3 Touch the Up arrow to increase the value in the box or the Down arrow to decrease it. If you press and hold an arrow, the value automatically increases or decreases.
4 Repeat steps 2 and 3 for each setting you want to change, and then press OK.
5 In the message asking if the settings are OK, touch CONFIRM.

NOTE: Changes to settings only take effect when you touch CONFIRM.

SETTING VENTILATION PARAMETERS IN ASSIST VENTILATION MODE

In this mode, the ventilation system provides Tidal Volume during inhalation for Assist breaths. An assist breath is started when there is patient effort and is ended when the Inspiratory Time setting has been met. If the ventilation system does not detect breaths during the allotted time as defined by the "Breath Timeout parameter", the ventilation system will deliver breaths or a continuous flow of gas based on the " Timeout Action" parameter setting.

(see Fig. 93)

To set a Prescription Setting for Assist ventilation mode, the following parameters need to be set according to the table below:

| THERAPY MODE SETTINGS* | SET TRIGGER SENSITIVITY TO: | SET BREATH RATE TO: |
|---|---|---|
| Assist ventilation mode | 0 to 9 | 0 |
| | | * SETTINGS BASED ON PRESCRIPTION |

Table 12

To set the Prescription Setting:

1 On the Clinician's Setting screen, touch Ventilation Settings.
2 On the Ventilation Settings screen, touch the box beside the setting you want to change.
3 Touch the Up arrow to increase the value in the box or the Down arrow to decrease it. If you press and hold an arrow, the value automatically increases or decreases.
4 Repeat steps 2 and 3 for each setting you want to change, and then press OK.
5 In the message asking if the settings are OK, touch CONFIRM.

NOTE: Changes to settings only take eWect when you touch CONFIRM.

VENTILATION SETTINGS SUMMARY

| PARAMETER | MINIMUM | MAXIMUM | INCREMENT |
|---|---|---|---|
| Volume (ml) | 50 | 750 | 10 |
| I-Time (ms) | 150 | 3000 | 50 |
| PEEP (cmH$_2$O) | 0 | 10 | 1 |
| Sensitivity | OFF (Control ventilation mode) and 0 (Assist or Assist/Control ventilation mode) | 9 | 1 |
| BR (/min) | 0 | 40 | 1 |

Table 13

TIPS:

- Volume: You can set an output volume between 50 ml and 750 ml, in increments of 10 ml.

NOTE: Volume levels are not adjusted for altitude. For more information see the "Ventilator Altitude Volume
Adjustment Table" on page 140.

- I-Time (InspiratoryTime): The time over which the selected target volume is delivered. NOTE: The actual I-Time may be longer or shorter than set if additional time is required to deliver the set volume. The actual delivery time may also be longer or shorter than set to maintain a minimum delivered peak gas flow rate of 8-40 LPM range.
- PEEP (Positive End Expiratory Pressure): PEEP can be adjusted as per the prescription. PEEP values can be set from 0 - 10 cmH2O.
- Sensitivity (Trigger Sensitivity): Specifies the minimum negative pressure threshold necessary to trigger a delivery. The sensitivity setting, adjustable in increments of 1, ranges from 0-9 or OFF, with 0 being the most sensitive and 9 being the least sensitive.

NOTE: Trigger sensitivity can be adjusted by the patient unless it is set to OFF in the Ventilation Settings screen. For more information, see "Adjusting the Trigger Sensitivity (Patient-Adjustable)".

Trigger sensitivity settings vary in a non-linear fashion, with relatively finer resolution at lower settings and relatively coarser resolution at higher settings.

Trigger sensitivity can be adjusted by both the clinician and the patient. Breaths can be triggered by the patient or by the ventilator based on the Ventilation Settings.

- BR (Breath Rate): The breath rate per minute determines the minimum quantity of machine breaths delivered.

SETTING ALARM LIMITS FOR BREATH RATE AND PIP

To view or edit critical alarms, access the Alarm Limits screen from the Clinician's Settings screen.

1 On the Clinician's Setting screen, touch Alarm Limits.
2 On the Set Alarm Limits screen, touch the box corresponding to the alarm limit you want to change.
3 Touch the Up arrow or the Down arrow to change the value in the box. If you press and hold an arrow, the value automatically increases or decreases.
4 Repeat steps 2 and 3 for each setting you want to change, and then press OK.
5 In the message asking if the settings are OK, touch CONFIRM.

NOTE: Changes to settings only take effect when you touch CONFIRM.

ALARM LIMITS SETTINGS SUMMARY

| ALARM | DEFAULT | MINIMUM | MAXIMUM | INCREMENT |
|---|---|---|---|---|
| High Breath Rate Alarm Limit (BPM) | 50 | 5 | 120 | 1 |
| Low Breath Rate Alarm Limit (BPM) | 5 | 0 | 119 | 1 |
| High PIP Alarm Limit (cmH$_2$O) | 20 | 5 | 40 | 1 |
| Low PIP Alarm Limit (cmH$_2$O) | 1 | 1 | 15 | 1 |

Table 14

TIP: The breath rate monitor value is based on a four-breath average.

SETTING BREATH TIMEOUT 'APNEA BACKUP VENTILATION MODEa

1 On the Clinician's Setting screen, touch Breath Timeout.
Breath Timeout
2 On the Breath Timeout screen, touch the Timeout Period or Timeout Action box you want to change.
3 Touch the Up arrow or the Down arrow to change the value in the box. If you press and hold an arrow, the value automatically sequences through the values.
4 Repeat steps 2 and 3 for each setting you want to change, and then press OK.
5 In the message asking if the settings are OK, touch CONFIRM.

NOTE: Changes to settings only take eWect when you touch CONFIRM.

TIP:

The Breath Timeout screen has two options for setting the Breath Timeout alarm. You can set the time to trigger the alarm at 20 or 60 seconds and the backup ventilation mode to 3 LPM continuous flow or 12 BPM at the current volume setting.

BREATH TIMEOUT SETTINGS SUMMARY

| PARAMETER | DEFAULT | ALTERNATE |
|---|---|---|
| Timeout Period | 60 seconds | 20 seconds |
| Timeout Action | 12 BPM | 3 LPM |

Table 15

Selecting the gas source

The Breathe Technologies Life2000TM Ventilation System uses Air as the factory set default source gas. If using supplemental oxygen or an oxygen cylinder, select O2 as the Source Gas.

1 On the Clinician's Setting screen, touch Source Gas.
2 On the Source Gas screen, touch the appropriate check box for the source gas prescribed by the physician.
3 Touch OK to confirm your selection.
4 In the message asking if the settings are OK, touch CONFIRM.

NOTE: Changes to settings only take effect when you touch CONFIRM.

SOURCE GAS SETTINGS SUMMARY

| DEFAULT | ALTERNATE |
|---|---|
| Air | $O_2$(Oxygen) |

Table 16

**CHOOSING** AN ACTIVITY BUTTON 'PATIENT(SELECTABLE)

When the ventilator is first powered on, you must select an Activity button to begin therapy. The three Activity buttons on the ventilator are programmed to correspond to up to three different prescriptions as directed by a physician.
Low Activity button
Medium Activity button
High Activity button
Choose a Prescription Setting button appropriate for the patient's needs. The Prescription Setting selection can be changed by the patient at any time, if set and activated by a clinician.
NOTE: One, two, or three Prescription Settings may be active, as directed by a physician. For more information, see "Viewing and Editing Prescription Settings".

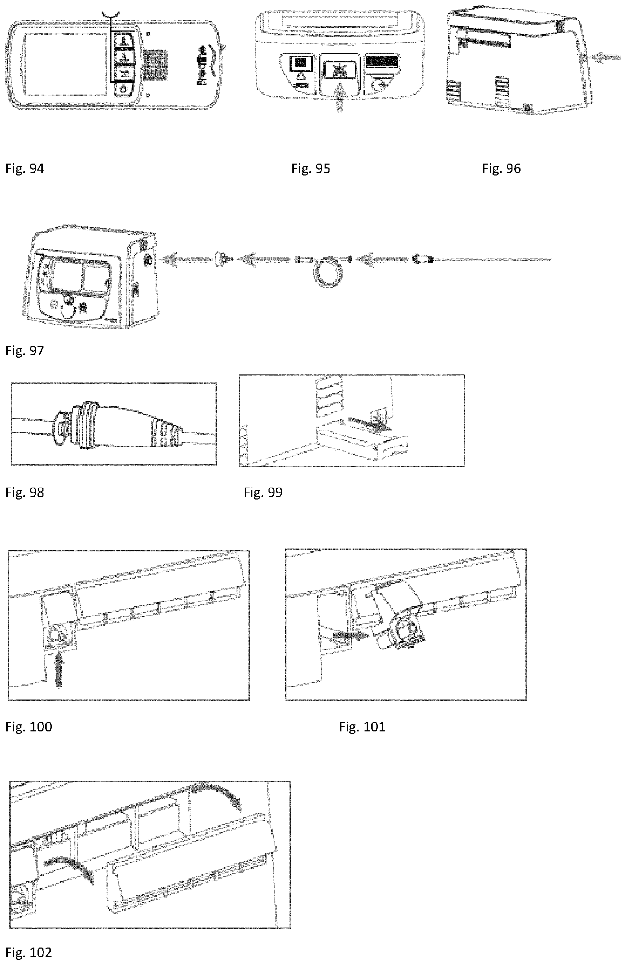

1 Ensure that the ventilator is powered on.
2 Ensure a pressure source is connected to the ventilator and turned on. (Fig. 94)

3 Press and hold an Activity button until you hear a tone that indicates it is active.

4 The touch screen will display the home screen. Confirm the selected Prescription Setting icon is displayed at the bottom of the touch screen and the Prescription Setting icon and volume are displayed at the top of the screen. (The High Activity Prescription Setting is shown for illustrative purposes here.) The ventilator will begin ventilating using the chosen Prescription Setting parameters after one breath.

NOTE: The currently-ventilating Prescription Setting icon and volume are displayed at the top of all screens unless you are in the Clinician's Menu.

"THIS PRESCRIPTION SETTING IS NOT ACTIVE" MESSAGE

If the selected Prescription Setting button is not active, this message will appear on the touch screen. If therapy had already started with a different Prescription Setting, the ventilator will continue delivering therapy using the previous Prescription Setting.

Touch OK and choose another active Prescription Setting button to change the currently-ventilating prescription.

"CONNECT OXYGEN SOURCE" OR "DISCONNECT OXYGEN SOURCE" MESSAGE

After powering on, or during therapy when selecting a Prescription Setting with a different source gas, a message will appear as a reminder to connect or disconnect oxygen as appropriate for the chosen Prescription Setting.

After connecting or disconnecting oxygen per the chosen Prescription Setting, touch OK to begin ventilating with the new Prescription Setting.

For more information, see "Selecting the Source Gas".

ADJUSTING THE TRIGGER SENSITIVITY 'PATIENT(ADJUSTABLE)

Trigger sensitivity determines how easily a patient's inspiratory effort triggers the breath delivery. For shallow breathing, set the trigger sensitivity to a low number. You can choose a setting between 0 and 9. Zero is the most sensitive and 9 is the least sensitive setting.

A Prescription Setting must already be selected (the ventilator must be currently ventilating) to allow changes to the Trigger Sensitivity settings through the patient-accessible Settings menu.

NOTE: Trigger Sensitivity cannot be adjusted by patients when the currently-ventilating Prescription Setting is in

Control Mode (Sensitivity is OFF).

ACCESSING THE TRIGGER SENSITIVITY SCREEN

1 On any screen, touch the Wrench button.

2 On the Menu screen, touch Settings.

3 On the Settings Menu screen, touch Trigger Sensitivity.

NOTE: A grayed-out Trigger Sensitivity button indicates that this feature is not available for one of the following reasons:

- A Prescription Setting has not been chosen (the ventilator is not currently ventilating. For more information, see "Choosing an Activity Button (Patient-Selectable)" on page 78.
- The currently ventilating Prescription Setting is in Control Mode. For more information, see "Setting Ventilation Parameters in Control Ventilation Mode" on page 68.

CHANGING TRIGGER SENSITIVITY

1 While ventilating, on the Trigger Sensitivity screen, touch the Up arrow to increase the value or the Down arrow to decrease it. If you press and hold an arrow, the number automatically increases or decreases.

NOTE: The lower the number, the more sensitive the setting.
2 When you are finished, touch OK.
3 In the message asking if the settings are OK, touch CONFIRM.

NOTE: Changes to settings only take effect when you touch CONFIRM.
This Trigger Sensitivity setting will be saved as part of the currently-ventilating Prescription Setting.

ACCESSING THE UTILITIES MENU

With the Utilities menu, you can change the time and date, brightness of the touch screen, volume of audible alarms, and set alarms to vibrate mode.

1 On any screen, touch the Wrench button.
2 On the Menu screen, touch Settings.
3 On the Settings Menu screen, touch Utilities.

SETTING TIME AND DATE

1 On the Utilities Menu screen, touch Set Time/Date.
2 On the Set Time/Date screen, touch the box you want to change.
3 Touch the Up arrow to increase the value in the box or the Down arrow to decrease it. If you press and hold an arrow, the value automatically increases or decreases.
4 Repeat steps 2 and 3 for each box you want to change, and then touch OK.
5 In the message asking if the settings are OK, touch CONFIRM.

NOTE: Changes to settings only take effect when you touch CONFIRM.

SETTING VIBRATION MODE

The Set Vibration screen lets you change alarm notifications from audible tones to a vibration. However, if a lower medium-priority vibrating alarm occurs and is not resolved in 60 seconds, an audible alarm occurs. For a high priority alarm, an audible tone immediately occurs with a vibration alarm with no delay.

1 On the Utilities Menu screen, touch Set Vibration.
2 On the Set Vibration screen, touch ON or OFF.
3 When you are finished, touch OK.
4 In the message asking if the settings are OK, touch CONFIRM.
NOTE: Changes to settings only take effect when you touch CONFIRM.
5 Check that the Vibrate icon appears, indicating the ventilator is in vibrate mode.

VIBRATION SETTINGS SUMMARY

Table 17

| DEFAULT | ALTERNATE |
|---------|-----------|
| OFF | ON |

Setting Audio Loudness

1 On the Utilities Menu screen, touch Set Loudness.
2 Touch the Up arrow to increase the audio loudness level or the Down arrow to decrease it. If you press and hold an arrow, the number automatically increases or decreases.
You can choose a loudness level between 1 and 5, with 5 being the loudest and 1 the quietest.
3 When you are finished, touch OK.
4 In the message asking if the settings are OK, touch CONFIRM.

NOTE: Changes to settings only take effect when you touch CONFIRM.

WARNING:

Reducing the alarm loudness level to lower than the ambient sound level will impede alarm condition recognition.

LOUDNESS SETTINGS SUMMARY

Table 18

| DEFAULT | MINIMUM | MAXIMUM | INCREMENT |
|---------|---------|---------|-----------|
| 5 | 1 | 5 | 1 |

ADJUSTING SCREEN BRIGHTNESS

1 On the Utilities Menu screen, touch Set Brightness.
2 Touch the Up arrow to increase the brightness or the Down arrow to decrease it. If you press and hold an arrow, the number automatically increases or decreases.
You can choose a brightness level between 1 and 5, with 5 being the brightest and 1 the dimmest.
3 When you are finished, touch OK.
4 In the message asking if the settings are OK, touch CONFIRM.

NOTE: Changes to settings only take effect when you touch CONFIRM.

BRIGHTNESS SETTINGS SUMMARY

Table 19

| DEFAULT | MINIMUM | MAXIMUM | INCREMENT |
|---------|---------|---------|-----------|
| 3 | 1 | 5 | 1 |

VIEWING SOFTWARE VERSION INFORMATION

The Software Version screen displays the software version number, its release date, and the ventilator's total operating time.

1 On any screen, touch the Wrench button.

2 On the Menu screen, touch Information.

3 The screen displays:

- software version,
- serial number, and
- total operating time.

SUMMARY OF FACTORY DEFAULT SETTINGS

The following table lists the factory default settings for the ventilator.

| CLINICIAN'S MENU SETTINGS | DEFAULT VALUE | | |
|---|---|---|---|
| | LOW ACTIVITY | MEDIUM ACTIVITY | HIGH ACTIVITY |
| Ventilation Settings | | | |
| Volume | 100 ml | 150 ml | 190 ml |
| I-Time (Inspiratory Time) | 750 ms | | |
| PEEP (Positive End Expiratory Pressure) | 0 cmH$_2$O | | |
| Sensitivity (Trigger Sensitivity) | 4* | | |
| BR (Breath Rate) | 12 BPM* | | |
| * Therapy mode: Assist/Control ventilation mode | | | |
| Alarm Limits | | | |
| High BR (Breath Rate) alarm limit | 50 BPM | | |
| Low BR (Breath Rate) alarm limit | 5 BPM | | |
| High PIP (Peak Inspiratory Pressure) alarm limit | 20 cmH$_2$O | | |
| Low PIP (Peak Inspiratory Pressure) alarm limit | 1 cmH$_2$O | | |
| High PEEP (Positive End Expiratory Pressure) Pressure alarm limit | +7 cmH$_2$O (above PEEP setting)* | | |
| * High PEEP Pressure alarm limit is automatically set and cannot be adjusted. | | | |
| Breath Timeout | | | |
| Breath Timeout Action | 12 BPM | | |
| Breath Timeout Period | 60 seconds | | |
| Source Gas | | | |
| Source Gas | Air | | |
| UTILITIES MENU SETTINGS | DEFAULT VALUE | | |
| Vibration | OFF | | |
| Audio Loudness | 5 | | |
| Screen Brightness | 3 | | |

Table 20

NOTE: Default values may be reset by entering the above values into the ventilator.

INTRODUCTION TO ALARMS AND ALERTS

There are two types of notifications provided by the Life2000TM Ventilation System:

VENTILATOR ALARMS

Ventilator alarms are visual notifications that appear on the touch screen and are accompanied by distinct sounds or vibration (when set to vibrate).

COMPRESSOR ALERTS

The compressor has audible alerts that are independent of the ventilator. Compressor alerts must be resolved for the compressor alert notifications to be silenced as there is no button to silence alerts originating from the compressor.

VENTILATOR ON-SCREEN ALARM SOUNDS AND MESSAGE DISPLAY

When an alarm notification occurs there is a distinct sound and a display message corresponding to the priority level of the alarm. The priority level of an alarm is indicated by the color and the rate at which the message flashes.

High-Priority Alarm

A red, rapidly flashing alarm message is an alarm that indicates a situation that requires immediate attention.
Sound: Sequence of two sets of five tones

Medium-Priority Alarm

A yellow, steadily flashing alarm message is an alarm that indicates a potentially hazardous situation that must be resolved in a timely manner.
Sound: Sequence of three tones

Low-Priority Alarm

A blue, non-flashing alarm message is an alarm that indicates a problem that is not hazardous but should be resolved.
Sound: Single tone

WARNING:

• Reducing the alarm loudness level to lower than the ambient sound level will impede alarm condition recognition.
• Do not cover the ventilator, touch screen, speaker, or backup alarm buzzer with tape or any other object. Covering the ventilator or any of its parts might cause didculty in hearing alarms and might a]ect ventilator performance.

ACTIVE ALARMS WINDOW

Multiple on-screen alarms may occur at the same time. Touch the Active Alarms button at the top of the touch screen to display a list of active on-screen alarms.
NOTE: The Active Alarms button is only visible during ventilator on-screen alarm notifications.

1 Touch the Active Alarms button to display the alarm list.
2 Alarm icon
3 Use the Scroll Down Arrow to view additional active alarms.
4 Use the Scroll Up Arrow to go to the beginning of the list of active alarms.
1 Alarm message alternates between each occurring alarm.
2 Alarm Silenced icon.
3 Alarm Silenced icon is displayed when all alarms are silenced.

TIP:

The Active Alarms window displays up to three on-screen alarms, from highest to lowest priority (red, yellow, blue). If there are more than three alarms, you can use the Scroll Up and Scroll Down arrows to scroll through the list.

SILENCING AND CLEARING ON@SCREEN ALARMS (Fig 95 and 96)

Alarm notifications that appear on the touch screen originate from the ventilator. Silencing and clearing on-screen alarms is a multi-step process that depends on alarm priority and how many alarms are active.

1 Press the Silence Alarm button to temporarily silence the on-screen alarm for 60 seconds. Pressing the Silence Alarm button silences only one alarm at a time-in audible or vibrating alarm mode. If more than one on-screen alarm occurs, press the Silence Alarm button once for each alarm. If the on-screen alarm is a medium- or high-priority alarm and is not silenced after 60 seconds, the alarm will continue with an additional buzzer.

2 Resolve the condition that triggered the on-screen alarm. For help resolving alarms, see the alarm and troubleshooting tables that follow for possible causes of an alarm and options to resolve it. If a Silence Alarm button is pressed but the condition that triggered the alarm is not resolved, the alarm will sound again after 60 seconds.

3 After resolving a High Temperature, High Circuit Pressure, or High PEEP Pressure high-priority alarm, touch OK in the message that indicates the alarm has been resolved.

Silence Alarm button on ventilator
(when in Extended Range or
Stand-Alone Configuration).
Silence Alarm button on compressor
(when in Stationary Configuration).

VENTILATOR ALARMS

The following tables list high-, medium-, and low-priority alarms. For each alarm, the tables list the notification, the possible causes for the alarm, and possible options for resolving it. The sample screens are for illustrative purposes only.

NOTE: Options for resolving the alarm are based on the current configuration of the ventilation system.

HIGH@PRIORITY ALARMS

| NOTIFICATION | | CAUSE | CHECKS AND POSSIBLE RESOLUTION IN EACH CONFIGURATION | | |
|---|---|---|---|---|---|
| | | | STATIONARY | EXTENDED RANGE | STAND-ALONE |
| High Circuit Pressure (High Circuit Pressure) | 14 | Interface may be pinched or kinked | Check the interface tube: replace it if it is pinched or kinked. Ensure the ventilator is properly docked into the compressor. | Check the interface tube: replace it if it is pinched or kinked. | Check the interface tube: replace it if it is pinched or kinked. |
| High PEEP Pressure | 15 / 20 / 7.5 | Interface may be blocked | Ensure the ventilator is properly docked into the compressor. Inspect and clean the interface per the instructions for cleaning the interface. | Inspect and clean the interface per the instructions for cleaning the interface. | Inspect and clean the interface per the instructions for cleaning the interface. |
| High PIP Pressure | 12 / 13.2 / 3.4 | Peak Inspiratory Pressure (PIP) exceeds the set limit | Ensure the ventilator is properly docked into the compressor. Check the interface or tubing for any obstruction. Check all connectors for possible damage. Re-adjust volume setting for the active prescription. | Check the interface or tubing for any obstruction. Check all connectors for possible damage. Re-adjust volume setting for the active prescription. | Check the interface or tubing for any obstruction. Check all connectors for possible damage. Re-adjust volume setting for the active prescription. |
| High Temperature | 15 / 20 / 7.5 | CPU or battery temperature is above the allowable limit | Check to make sure the compressor is: • Not near a heat source. • In a well-ventilated area. • Not covered or enclosed. • Operating within the given operating environment specifications. If the alarm persists, contact your Breathe Technologies service representative. | Check to make sure the ventilator is: • Not near a heat source. • In a well-ventilated area. • Not covered or enclosed. • Operating within the given operating environment specifications. If the alarm persists, contact your Breathe Technologies service representative. | Check to make sure the ventilator is: • Not near a heat source. • In a well-ventilated area. • Not covered or enclosed. • Operating within the given operating environment specifications. If the alarm persists, contact your Breathe Technologies service representative. |

Table 21

HIGH@PRIORITY ALARMS (CONTINUED)

| NOTIFICATION | CAUSE | CHECKS AND POSSIBLE RESOLUTION IN EACH CONFIGURATION | | |
|---|---|---|---|---|
| | | STATIONARY | EXTENDED RANGE | STAND-ALONE |
| Very Low Battery | Ventilator battery capacity drops below 15% | Ensure the ventilator is properly docked and locked into the compressor and the locked icon is illuminated to indicate the ventilator is charging. Ensure the compressor is powered on. If the battery does not recharge, place the patient on an alternate means of ventilation and contact your Breathe Technologies service representative. | Connect the ventilator to the ventilator battery charger and an AC power source to recharge the battery. OR Dock the ventilator back into the compressor to recharge the battery. Ensure that the locked icon on the compressor illuminates to indicate the ventilator is charging. Ensure the compressor is powered on. If the battery does not recharge, place the patient on an alternate means of ventilation and contact your Breathe Technologies service representative. | Connect the ventilator to the ventilator battery charger and an AC power source to recharge the battery. OR Dock the ventilator back into the compressor to recharge the battery. Ensure that the locked icon on the ventilator is charging. Ensure the compressor is powered on. If the battery does not recharge, place the patient on an alternate means of ventilation and contact your Breathe Technologies service representative. |
| Buzzer sounds constantly for two to five minutes and screen goes back. | The ventilator battery is damaged. | Contact your Breathe Technologies service representative. | Contact your Breathe Technologies service representative. | Contact your Breathe Technologies service representative. |

Table 22

MEDIUM-PRIORITY ALARMS

EP 3 341 062 B1

| NOTIFICATION | CAUSE | CHECKS AND POSSIBLE RESOLUTION IN EACH CONFIGURATION | | |
| --- | --- | --- | --- | --- |
| | | STATIONARY | EXTENDED RANGE | STAND-ALONE |
| Battery Low <br> 12 3.9 1.1 | Ventilator battery capacity drops below 25%. | Ensure the ventilator is properly docked and locked into the compressor and the locked icon is illuminated to indicate the ventilator is charging. <br><br> Ensure the compressor is powered on. <br><br> If the battery does not recharge, contact your Breathe Technologies service representative. | Connect the ventilator to the ventilator battery charger and an AC power source to recharge the battery. <br><br> OR <br><br> Dock the ventilator back into the compressor to recharge the battery. Ensure that the locked icon on the compressor illuminates to indicate the ventilator is charging. Ensure the compressor is powered on. <br><br> If the battery does not recharge, contact your Breathe Technologies service representative. | Connect the ventilator to the ventilator battery charger and an AC power source to recharge the battery. <br><br> OR <br><br> Dock the ventilator back into the compressor to recharge the battery. Ensure that the locked icon on the compressor illuminates to indicate the ventilator is charging. Ensure the compressor is powered on. <br><br> If the battery does not recharge, contact your Breathe Technologies service representative. |
| Breath Timeout <br> 1.4 3.0 | No breath is detected for 20 or 60 seconds, depending on the setting | Patient is not breathing. <br><br> Patient is breathing through the mouth while using the Breathe Pillows interface. <br><br> Breaths are too shallow to trigger ventilation; change the trigger sensitivity to a lower setting (higher sensitivity). <br><br> Ensure patient interface is not leaking at patient side. <br><br> Inspect and clean the interface per the instructions for cleaning the interface. <br><br> Ensure the ventilator is properly docked into the compressor. | Patient is not breathing. <br><br> Check that the interface is connected to the ventilator, not the compressor. <br><br> Patient is breathing through the mouth while using the Breathe Pillows interface. <br><br> Breaths are too shallow to trigger ventilation; change the trigger sensitivity to a lower setting (higher sensitivity). <br><br> Ensure patient interface is not leaking at patient side. <br><br> Inspect and clean the interface per the instructions for cleaning the interface. | Patient is not breathing. <br><br> Patient is breathing through the mouth while using the Breathe Pillows interface. <br><br> Breaths are too shallow to trigger ventilation; change the trigger sensitivity to a lower setting (higher sensitivity). <br><br> Ensure patient interface is not leaking at patient side. <br><br> Inspect and clean the interface per the instructions for cleaning the interface. |
| High Breath Rate <br> 43 1.5 11.3 | Respiratory rate exceeds the set limit. | Patient is breathing faster than the rate set by the clinician. <br><br> Inspect and clean the interface per the instructions for cleaning the interface. <br><br> The ventilator may be false triggering because the trigger sensitivity is set too low (in Assist or Assist/Control ventilation mode). Verify the ventilator is syncing with patient effort and adjust trigger sensitivity higher (lower sensitivity). | Patient is breathing faster than the rate set by the clinician. <br><br> Inspect and clean the interface per the instructions for cleaning the interface. <br><br> The ventilator may be false triggering because the trigger sensitivity is set too low (in Assist or Assist/Control ventilation mode). Verify the ventilator is syncing with patient effort and adjust trigger sensitivity higher (lower sensitivity). | Patient is breathing faster than the rate set by the clinician. <br><br> Inspect and clean the interface per the instructions for cleaning the interface. <br><br> The ventilator may be false triggering because the trigger sensitivity is set too low (in Assist or Assist/Control ventilation mode). Verify the ventilator is syncing with patient effort and adjust trigger sensitivity higher (lower sensitivity). |

Table 23

61

| NOTIFICATION | CAUSE | CHECKS AND POSSIBLE RESOLUTION IN EACH CONFIGURATION | | |
|---|---|---|---|---|
| | | STATIONARY | EXTENDED RANGE | STAND-ALONE |
| High Del. Pressure (High Delivery Pressure) <br> 14 | Interface pressure during delivery exceeds the maximum expected. | Check the interface; replace it if the tubing is torn, bent, or kinked. <br> Ensure the interface tubing is not pinched, crushed, bent, or kinked. <br> Ensure the ventilator is properly docked into the compressor. | Check the interface; replace it if the tubing is torn, bent, or kinked. <br> Ensure the interface tubing is not pinched, crushed, bent, or kinked. | Check the interface; replace it if the tubing is torn, bent, or kinked. <br> Ensure the interface tubing is not pinched, crushed, bent, or kinked. |
| High Gas Pressure <br> 12 | Gas pressure exceeds the allowable limit. | Place the patient on an alternate means of ventilation, or undock the ventilator from the compressor and connect to an alternate pressure source in Stand-Alone configuration. <br> Power off the compressor. <br> Contact your Breathe Technologies service representative. | Place the patient on an alternate means of ventilation, or connect the ventilator to an alternate pressure source in Stand-Alone configuration. <br> Power off the compressor. <br> Contact your Breathe Technologies service representative. | Place the patient on an alternate means of ventilation. <br> Ensure that you are using a 50-PSI (nominal) regulator. <br> If the alarm is not resolved, power off the ventilator and contact your Breathe Technologies service representative. |
| Low Breath Rate <br> 13 | Respiratory rate falls below set limit. | Ensure that the ventilator is properly docked into the compressor. <br> The patient is breathing through the mouth while using the Breathe Pillows interface. <br> Breaths are too shallow to trigger ventilation; change the trigger sensitivity to a lower setting (higher sensitivity). <br> Ensure patient interface is not leaking at patient side. <br> Inspect and clean the interface per the instructions for cleaning the interface. | Check that the interface is connected to the ventilator, not the compressor. <br> Patient is breathing through the mouth while using the Breathe Pillows interface. <br> Breaths are too shallow to trigger ventilation; change the trigger sensitivity to a lower setting (higher sensitivity). <br> Ensure patient interface is not leaking at patient side. <br> Inspect and clean the interface per the instructions for cleaning the interface. | Patient is breathing through the mouth while using the Breathe Pillows interface. <br> Breaths are too shallow to trigger ventilation; change the trigger sensitivity to a lower setting (higher sensitivity). <br> Ensure patient interface is not leaking at patient side. <br> Inspect and clean the interface per the instructions for cleaning the interface. |
| Low Del. Pressure (Low Delivery Pressure) <br> 11 | Interface pressure during delivery fails to exceed the minimum expected. | Ensure the ventilator is properly docked into the compressor. <br> Check the interface connections. <br> Check the interface; replace it if it is leaking. | Check that the interface is connected to the ventilator, not the compressor. <br> Check the interface connections. <br> Check the interface; replace it if it is leaking. | Check the interface connections. <br> Check the interface; replace it if it is leaking. |

Table 24

62

EP 3 341 062 B1

| NOTIFICATION | CAUSE | CHECKS AND POSSIBLE RESOLUTION IN EACH CONFIGURATION | | |
|---|---|---|---|---|
| | | STATIONARY | EXTENDED RANGE | STAND-ALONE |
| Low Gas Pressure <br><br> 18  3.1  6.2 | Gas pressure drops below the allowable limit | Verify that the compressor is powered on. <br><br> Ensure the ventilator is properly docked into the compressor. <br><br> If the alarm is not resolved, place the patient on an alternate means of ventilation, or undock the ventilator from the compressor and connect to an alternate pressure source in Stand-Alone configuration. Contact your Breathe Technologies service representative. | Verify that the compressor is powered on. <br><br> Check all connections for possible leak. <br><br> Ensure that the gas supply hose is not kinked or pinched. <br><br> Dock the ventilator back into the compressor. <br><br> If the alarm is not resolved, place the patient on an alternate means of ventilation, or connect the ventilator to an alternate pressure source in Stand-Alone Configuration. Contact your Breathe Technologies service representative. | Check all connections for possible leak. <br><br> Ensure that the gas supply hose is not kinked or pinched. <br><br> Ensure you are using a 50-PSI (nominal) regulator with a minimum outlet flow of >40 LPM at 41 PSI. <br><br> Ensure the gas source (e.g. cylinder) has a sufficient supply of gas (e.g. check that the cylinder is not empty.) <br><br> If using a cylinder as the gas source, ensure the cylinder valve is fully open. <br><br> Connect the ventilator to the compressor in Extended Range or Stationary configuration or connect the ventilator to another gas source. <br><br> If the alarm is not resolved, place the patient on an alternate means of ventilation and contact your Breathe Technologies service representative. |
| Flashing Low Gas Pressure | The Low Gas Pressure alarm flashes on the screen intermittently | Contact your Breathe Technologies service representative. | Contact your Breathe Technologies service representative. | The cylinder is nearly empty; connect the ventilator to a full cylinder. If the issue persists, contact your Breathe Technologies service representative. |
| Low PIP Pressure <br><br> 13  0.0  4.3 | Peak Inspiratory Pressure (PIP) below set limit | Ensure patient interface is not leaking at patient side. <br><br> Ensure the ventilator is properly docked into the compressor. <br><br> Switch to another active Prescription Setting. <br><br> If symptoms persist, contact your physician. | Ensure patient interface is not leaking at patient side. <br><br> Switch to another active Prescription Setting. <br><br> If symptoms persist, contact your physician. | Ensure patient interface is not leaking at patient side. <br><br> Switch to another active Prescription Setting. <br><br> If symptoms persist, contact your physician. |
| System Fault <br><br> System Fault <br> Press OK to <br> restart system. | Internal fault detected during operation | If a system fault occurs, in the message on the touch screen to reboot, touch OK; the ventilator will turn itself off and then on again. <br><br> If the system fault persists, place the patient on an alternate means of ventilation and contact your Breathe Technologies service representative. | If a system fault occurs, in the message on the touch screen to reboot, touch OK; the ventilator will turn itself off and then on again. <br><br> If the system fault persists, place the patient on an alternate means of ventilation and contact your Breathe Technologies service representative. | If a system fault occurs, in the message on the touch screen to reboot, touch OK; the ventilator will turn itself off and then on again. <br><br> If the system fault persists, place the patient on an alternate means of ventilation and contact your Breathe Technologies service representative. |

Table 25

63

| NOTIFICATION | CAUSE | CHECKS AND POSSIBLE RESOLUTION IN EACH CONFIGURATION | | |
| --- | --- | --- | --- | --- |
| | | STATIONARY | EXTENDED RANGE | STAND-ALONE |
| POST System Fault | System fault is detected during ventilator power on. | Power off the ventilator, and power it on again.<br><br>If the system fault persists, place the patient on an alternate means of ventilation and contact your Breathe Technologies service representative. | Power off the ventilator, and power it on again.<br><br>If the system fault persists, place the patient on an alternate means of ventilation and contact your Breathe Technologies service representative. | Power off the ventilator, and power it on again.<br><br>If the system fault persists, place the patient on an alternate means of ventilation and contact your Breathe Technologies service representative. |

Table 27

64

COMPRESSOR ALERTS

The compressor has alerts that are independent of the ventilator. Compressor alerts must be resolved in order for the compressor alert notifications to be silenced; there is no Silence Alarm button for alerts originating from the compressor.

| NOTIFICATION | CAUSE | CHECKS AND POSSIBLE RESOLUTION IN EACH CONFIGURATION | | |
|---|---|---|---|---|
| | | STATIONARY | EXTENDED RANGE | STAND-ALONE |
| Compressor Low Battery Alert - Intermittent buzzer | Compressor battery capacity drops below 20%. | Connect the compressor to an AC power source. If the battery does not recharge, place the patient on an alternate means of ventilation and contact your Breathe Technologies service representative. | Connect the compressor to an AC power source. If the battery does not recharge, place the patient on an alternate means of ventilation and contact your Breathe Technologies service representative. | N/A |
| Constant Alert (Compressor stops operating) | Multiple causes | Place the patient on an alternate means of ventilation, or undock the ventilator from the compressor and connect to an alternate pressure source in Stand-Alone configuration. If running on battery, check the compressor's battery charge status. If the status is less than two indicator lights, connect the compressor to an AC power source. Power off the compressor and power it on again. If the alert persists, power off the compressor and contact your Breathe Technologies service representative. | Place the patient on an alternate means of ventilation, or connect the ventilator to an alternate pressure source in Stand-Alone configuration. If running on battery, check the compressor's battery charge status. If the status is less than two indicator lights, connect the compressor to an AC power source. Power off the compressor and power it on again. If the alert persists, power off the compressor and contact your Breathe Technologies service representative. | N/A |

Table 28

TROUBLESHOOTING

The following table lists situations that may occur during normal use of the ventilation system that do not have an alarm associated with them. The possible causes and options for resolving these situations are also listed.

| OBSERVATION | CAUSE | CHECKS AND POSSIBLE RESOLUTION IN EACH CONFIGURATION | | |
|---|---|---|---|---|
| | | STATIONARY | EXTENDED RANGE | STAND-ALONE |
| Breath indicator light is not syncing with patient breathing or is missing patient breaths | Patient interface is not connected or is leaking. | Verify that the interface is properly connected to the compressor and is not leaking at patient side. | Verify that the interface is properly connected to the ventilator (not the compressor) and is not leaking at patient side. | Verify that the interface is properly connected to the ventilator and is not leaking at patient side. |
| | A ventilator Prescription Setting button has not been pressed. | Press a Prescription Setting button. | Press a Prescription Setting button. | Press a Prescription Setting button. |
| | Patient's breath is too shallow to trigger breath. | Change the trigger sensitivity setting (higher sensitivity). | Change the trigger sensitivity setting (higher sensitivity). | Change the trigger sensitivity setting to a lower setting (higher sensitivity). |
| | Patient is mouth breathing while using a Breathe Pillows interface or other nasal mask. | Instruct patient to breathe in through their nose (purse lipped breathing is acceptable). | Instruct patient to breathe in through their nose (purse lipped breathing is acceptable). | Instruct patient to breathe in through their nose (purse lipped breathing is acceptable). |
| | Secretions may have built up on the interface, blocking the sense port. | Inspect and clean the interface per the instructions for cleaning the interface. | Inspect and clean the interface per the instructions for cleaning the interface. | Inspect and clean the interface per the instructions for cleaning the interface. |
| | Patient is breathing faster than 40 BPM. | It is normal for the ventilator to limit breath rate to 40 BPM. | It is normal for the ventilator to limit breath rate to 40 BPM. | It is normal for the ventilator to limit breath rate to 40 BPM. |
| No volume output | Compressor and/or ventilator is not on. | Ensure both the compressor and the ventilator are on. | Ensure both the compressor and the ventilator are on. | Turn the ventilator on. |
| | A Prescription Setting button has not been pressed to start therapy. | Press a Prescription Setting button. | Press a Prescription Setting button. | Press a Prescription Setting button. |
| | Patient interface is not connected or is leaking. | Verify that the interface is properly connected to the compressor and is not leaking at patient side. | Verify that the interface is properly connected to the ventilator (not the compressor) and is not leaking at patient side. | Verify that the interface is properly connected to the ventilator and is not leaking at patient side. |
| | Battery is depleted if running on battery. | If not plugged in, recharge the battery by connecting the compressor to an AC power source. | Connect the ventilator to the ventilator battery charger and an AC power source or dock the ventilator back into the compressor and power on the compressor to recharge the battery. | Connect the ventilator to the ventilator battery charger and an AC power source or dock the ventilator back into the compressor and power on the compressor to recharge the battery. |
| | Ventilator or compressor is inoperative. | If there is still no volume output, contact your Breathe Technologies service representative. | If there is still no volume output, contact your Breathe Technologies service representative. | If there is still no volume output, contact your Breathe Technologies service representative. |
| | Oxygen or air hose is disconnected. | | | Reconnect the source gas supply. |
| | Oxygen or air cylinder is empty. | | | Replace the source gas supply. |
| | Incorrect source gas supply hose is being used. | | | Ensure that the correct source gas supply hose (oxygen or air) is connected. |

Table 29

ADDITIONAL TROUBLESHOOTING SITUATIONS (CONTINUED)

| OBSERVATION | CAUSE | CHECKS AND POSSIBLE RESOLUTION IN EACH CONFIGURATION | | |
| --- | --- | --- | --- | --- |
| | | STATIONARY | EXTENDED RANGE | STAND-ALONE |
| Breath indicator light is not flashing and there is no volume output | Ventilator is not on. | Ensure ventilator is on. | Ensure ventilator is on. | Ensure ventilator is on. |
| | A ventilator Prescription Setting button has not been pressed. | Press a Prescription Setting button. | Press a Prescription Setting button. | Press a Prescription Setting button. |
| Ventilator is delivering gas without being triggered by patient effort. | Ventilator is in Control or Assist/Control ventilation mode. | It is normal for the ventilator to deliver therapy based on breath rate and breath timeout settings; adjust settings as required. | It is normal for the ventilator to deliver therapy based on breath rate and breath timeout settings; adjust settings as required. | It is normal for the ventilator to deliver therapy based on breath rate and breath timeout settings; adjust settings as required. |
| | The trigger sensitivity is too sensitive. | Adjust trigger sensitivity to a higher number (lower sensitivity). | Adjust trigger sensitivity to a higher number (lower sensitivity). | Adjust trigger sensitivity to a higher number (lower sensitivity). |
| | Secretions have built up on the interface. | Inspect and clean the interface per the instructions for cleaning the interface. | Inspect and clean the interface per the instructions for cleaning the interface. | Inspect and clean the interface per the instructions for cleaning the interface. |
| | Patient interface or connector is damaged. | Check the patient interface and connector for damage and replace if necessary. | Check the patient interface and connector for damage and replace if necessary. | Check the patient interface and connector for damage and replace if necessary. |
| Ventilator sometimes misses breaths. | Patient is breathing faster than 40 BPM. | It is normal for the ventilator to limit breath rate to less than 40 BPM. | It is normal for the ventilator to limit breath rate to less than 40 BPM. | It is normal for the ventilator to limit breath rate to less than 40 BPM. |
| | Secretions have built up on the interface. | Inspect and clean the interface per the instructions for cleaning the interface. | Inspect and clean the interface per the instructions for cleaning the interface. | Inspect and clean the interface per the instructions for cleaning the interface. |
| | Patient interface is not connected or is leaking. | Verify that the interface is properly connected to the compressor and is not leaking at patient side. | Verify that the interface is properly connected to the ventilator (not the compressor) and is not leaking at patient side. | Verify that the interface is properly connected to the ventilator and is not leaking at patient side. |
| Ventilator is triggering during exhalation. | Secretions have built up on the interface. | Inspect and clean the interface per the instructions for cleaning the interface. | Inspect and clean the interface per the instructions for cleaning the interface. | Inspect and clean the interface per the instructions for cleaning the interface. |
| Therapy delivery is causing coughing or irritation in airway. | Interface is not positioned correctly. | Reposition the interface per the instructions in "Chapter 5: Connecting an Interface". | Reposition the interface per the instructions in "Chapter 5: Connecting an Interface". | Reposition the interface per the instructions in "Chapter 5: Connecting an Interface". |
| | Patient is breathing against ventilator-triggered breaths. | Switch to another active Prescription Setting. If symptoms persist, contact your physician. | Switch to another active Prescription Setting. If symptoms persist, contact your physician. | Switch to another active Prescription Setting. If symptoms persist, contact your physician. |
| Ventilator battery does not last as long as expected after a charge. | Ventilator battery is not charged completely. | Recharge the ventilator battery. Check that the ventilator is correctly docked in the compressor and make sure that the ventilator charging indicator light (the locked icon) on the compressor is illuminated when the compressor is on. | Connect the ventilator to the ventilator battery charger and an AC power source or dock the ventilator back into the compressor and power on the compressor to recharge the battery. | Connect the ventilator to the ventilator battery charger and an AC power source or dock the ventilator back into the compressor to recharge the battery. |
| | Ventilator battery life is nearing its end. | Contact your Breathe Technologies service representative. | Contact your Breathe Technologies service representative. | Contact your Breathe Technologies service representative. |

Table 30

ADDITIONAL TROUBLESHOOTING SITUATIONS (CONTINUED)

EP 3 341 062 B1

| OBSERVATION | CAUSE | CHECKS AND POSSIBLE RESOLUTION IN EACH CONFIGURATION | | |
| | | STATIONARY | EXTENDED RANGE | STAND-ALONE |
|---|---|---|---|---|
| An on-screen alarm flashes on the screen intermittently | Low gas pressure | Contact your Breathe Technologies service representative. | Contact your Breathe Technologies service representative. | The cylinder is nearly empty; connect the ventilator to a full cylinder. If the issue persists, contact your Breathe Technologies service representative. |
| Ventilator does not turn on | Ventilator is not correctly docked in the compressor. | Check that the ventilator is correctly docked in the compressor and make sure that the ventilator charging indicator light (the locked icon) on the compressor is illuminated when the compressor is on. | N/A | N/A |
| | Ventilator battery is completely discharged. | Connect the compressor to an AC power source. If the issue is not resolved, contact your Breathe Technologies service representative. | Connect the ventilator to the ventilator battery charger and an AC power source or dock the ventilator back into the compressor and power on the compressor to recharge the battery. If the issue is not resolved, contact your Breathe Technologies service representative. | Connect the ventilator to the ventilator battery charger and an AC power source or dock the ventilator back into the compressor and power on the compressor to recharge the battery. If the issue is not resolved, contact your Breathe Technologies service representative. |
| The oxygen or air hose does not connect to the gas source. | An incorrect source gas supply hose is being used. | N/A | Ensure you are using the provided air hose or another Breathe Technologies approved air hose to connect the ventilator to the compressor. | Ensure you are using the correct Breathe Technologies approved gas supply hose (oxygen or air). |
| | An incompatible regulator is being used. | | N/A | Ensure the gas regulator is 42-87 PSI with a standard DISS fitting. |
| | | | If issues persist, contact your Breathe Technologies service representative. | If issues persist, contact your Breathe Technologies service representative. |
| Cylinder does not last as long as expected | User breath rate is higher than expected. | N/A | N/A | Refer to "Cylinder Duration Information" on page 54. |
| | Prescription selected requires higher volumes of gas. | | | Obtain a new or larger cylinder. |
| | Cylinder was not full at the beginning of ventilation. | | | |
| | The gas regulator is not properly connected to the gas cylinder. | | | Reconnect the gas regulator to the gas cylinder and verify there are no leaks. |
| | Flow regulator is on. | | | Ensure the flow valve is off or set to 0. |
| | The gas regulator may have a leak. | | | The regulator sealing washer that connects to the gas cylinder may be worn or damaged. Contact your Breathe Technologies service representative. |

69

Table 31

CLEANING BEFORE FIRST USE

It is not necessary to clean or sterilize the Life2000TM before the first use.

DAILY CHECKS

Look at the ventilation system components daily. If any of the following conditions are discovered, discontinue use of the ventilation system:

- Check for cracks in the casing.
- Check for loose or damaged buttons, connectors, or other control and alarm components.
- Check the interface and the oxygen or air hose (if applicable) for leaks and loose or damaged cabling or connectors.

Daily, or more often if necessary, check and empty the compressor's water tray and replace if necessary. For more information, see "Checking and Replacing the Water Tray".

Essential Performance:

- Absence of system fault alarms
- Unintended change of settings and modes
- Absence of false alarms
- No interruption of operation without alarms.

For instructions on servicing or replacing damaged ventilation system components, contact your Breathe Technologies service representative.

ENVIRONMENTAL SPECIFICATIONS

Do not use the ventilation system if the ambient temperature is greater than 40°C (104°F) or less than 5°C (41°F).
Store the ventilation system in ambient temperatures less than 60°C (140°F) and greater than -20°C (-4°F).

WARNING:

The backside of the ventilator enclosure may reach 49°C in a 40°C environment.

CLEANING AND DISINFECTING THE VENTILATION SYSTEM

- Using a clean cloth, clean and disinfect the external surfaces of the ventilation system with 70% isopropyl alcohol or PDI Sani-Cloth wipes as necessary and between uses.
  NOTE: If using PDI Sani-Cloth, allow for the manufacturer's suggested wait time before you wipe off the residue.
- Wipe the surface of the ventilation system with clean dry cloth to remove any residual cleaner.
- Do not clean the ventilation system with petrochemical or oil-based materials.
- Clean the touch screen with a soft microfiber cloth and disinfect with PDI Sani-Cloth wipes as necessary. After allowing for the manufacturer's suggested wait time, wipe the screen with clean cloth to remove any residual cleaner.

CAUTION:

- 70% isopropyl alcohol may damage the touch screen. When cleaning external surfaces of the ventilation system with 70% isopropyl alcohol, avoid contact with the touch screen.
- Keep in a clean environment to protect the ventilation system from ingress of dust, lint, and pests.
- Do not leave exposed to the sun or other sources of radiant heat, it may overheat.
- Do not allow children or pets to access the ventilation system, it may become damaged.

ALARM CHECKS

Confirm that when the ventilator is turned on, it makes audible tones. If tones are not heard, the ventilator should be returned to your Breathe Technologies service representative.

CLEANING FOR SINGLE@PATIENT USE

VENTILATION SYSTEM

1 Once a week, or more often if necessary, follow the instructions found in the " Cleaning and Disinfecting the Ventilation System" section of this chapter.
2 Daily, or more often if necessary, check and empty the compressor's water tray and replace if necessary. For more information, see "Checking and Replacing the Water Tray".
3 Every three to six months, or more often if necessary, check the compressor's air inlet filter and replace if necessary. For more information, see "Checking and Replacing the Air Inlet Filter".
4 Every three to six months, or more often if necessary, check the compressor's cooling air filter and replace if necessary. For more information, see "Checking and Replacing the Cooling Air Filter".

PILLOWS INTERFACE AND UNIVERSAL CIRCUITTM INTERFACE

Once a week, or more often if necessary, follow the instructions found in the "Cleaning the Breathe Interfaces" section of this chapter.

THIRD@PARTY MASKS

Follow the manufacturer's suggested cleaning instructions.

CLEANING FOR MULTI@PATIENT USE

WARNING:

To prevent risk of cross-contamination, clean and disinfect the ventilation system before using it on a new patient, and use a new Pillows Interface or Universal CircuitTM interface. For the third-party mask, refer to the user guide provided by the manufacturer.
In addition to the cleaning and maintenance instructions for single-patient use, you must perform the following before the ventilation system is provided to a new patient.

VENTILATION SYSTEM

1 Follow the instructions in "Cleaning and Disinfecting the Ventilation System".
2 Replace the compressor's water tray. For more information, see "Checking and Replacing the Water Tray".
3 Replace the compressor's air inlet filter. For more information, see "Checking and Replacing the Air Inlet Filter" on page 122.
4 Replace the compressor's cooling air filter. For more information, see "Checking and Replacing the Cooling Air Filter" on page 123.

PILLOWS INTERFACE

Replace between patients.

UNIVERSAL CIRCUITTM INTERFACE

Replace between patients.

THIRD@PARTY MASKS

Refer to the user guide provided with the mask in use.

CLEANING THE BREATHE INTERFACES

NOTE: Below instructions are for cleaning Breathe interfaces. If using third-party patient interfaces, please refer to the manufacturer's suggested cleaning instructions.

- If mucous accumulates on the patient interfaces, use a clean cloth to remove it.
- If dirt is visible on the outside of the interfaces, use a clean cloth and mild detergent such as dish-washing soap to remove it.

WARNING:

Do not subject Breathe interfaces to heat sterilization, hot water pasteurization, autoclaving, radiation sterilization, ethylene oxide gas sterilization, or attempt to clean them in a dishwasher or microwave oven. Doing any of these may damage the interfaces and impair gas delivery.

PERIODICALLY CLEAN THE BREATHE INTERFACES FOLLOWING THESE STEPS:

1 Place the patient on an alternate means of ventilation.
2 Power off the ventilator and compressor.
3 Disconnect the interface from the ventilation system.
4 Submerge the patient side of the Pillows Interface or the patient side of the Universal CircuitTM interface in a clean container of mixed warm water suitable for drinking and a mild detergent (e.g., dish washing soap) and agitate the patient side of the interface to clean it. Rinse the patient side of the interface thoroughly with warm water.
5 Perform a purge immediately after the rinse to completely dry the interface and to clear any excess water that may impede air flow. For purging instructions, see the section "Purging the Pillows Interface and Universal CircuitTM Interface" that immediately follows.
6 Hang the Pillows Interface or the Universal CircuitTM interface to completely dry away from direct sunlight.
7 Before reusing the interface, perform a second purge to clear any excess water that may impede air flow.

For purging instructions, see the section "Purging the Pillows Interface and Universal CircuitTM Interface" that immediately follows.

PURGING THE PILLOWS INTERFACE AND UNIVERSAL CIRCUITTM INTERFACE

After cleaning and completely drying the interface or when you suspect dust or debris has entered the air-flow passage, you can purge the interface with the purge tube connector and purge tube. NOTE: The ventilator is not required for the purging process.

1 Place the patient on an alternate means of ventilation.
(see Fig. 97)
2 Connect the purge tube connector to the DISS 1240 outlet connection on the compressor by twisting on.
3 Connect the larger end of the purge tube to the barbed outlet of the purge tube connector by twisting on.
4 Power on the compressor.
5 Firmly press and hold the smaller end of the purge tube over one of the interface ports that connects the interface to the compressor (Fig. 98). Take care not to slide the tube over the O-ring of the port. Hold the purge tube over the interface port until all the water is purged from the tube.
6 Repeat step 4 for the other interface port.
7 Power off the compressor.
8 Twist to remove the purge tube from the purge tube connector, and store the purge tube and purge tube connector for future use.

PREVENTIVE MAINTENANCE

The ventilation system can only be serviced or repaired by Breathe Technologies or an authorized service center. Contact your Breathe Technologies service representative to make arrangements for annual preventive maintenance, service, and component replacement. Trained personnel and authorized service centers are provided with the proper documentation to maintain the ventilation system.

When shipping the ventilation system, use proper packaging for protection.

NOTE: The compressor contains a lithium ion battery. Before shipping the compressor, contact your transportation carrier for information.

TIP:

The ventilation system is shipped in specially designed, protective boxes. Do not throw away the boxes; keep them for future transportation needs.

CHECKING AND REPLACING THE WATER TRAY

The water tray collects water condensate from the humidity in the air.

Check the water tray daily, or more frequently if needed. The frequency that the water tray needs to be checked and emptied will depend on the amount of humidity in the ambient air. The more humidity that is in the air, the more frequently the water tray will need to be checked, emptied, and replaced.

NOTE: The compressor is shipped with a water tray already installed.

1 Place the patient on an alternate means of ventilation.

2 If operating in Stationary or Extended Range Configuration, power off the ventilator.

3 Power off the compressor.

4 To check the water tray, carefully slide the water tray out from the back of the compressor, attempting to keep the water tray level (see Fig. 99).

5 If condensation has collected in the water tray, completely remove the water tray from the compressor and empty the water thoroughly. Squeeze the attached water tray sponge to remove as much water as possible (some remaining dampness is normal).

NOTE: Do not try to remove the sponge from the water tray.

6 Ensure that the overflow hole is not blocked.

7 Inspect the water tray and replace it if it has any damage or if the sponge shows signs of degradation, contamination, or odor.

If replacing the water tray, obtain a replacement water tray from Breathe Technologies. For more information about purchasing replacement water trays, refer to "Accessories and Replacement Parts".

8 Insert the water tray into the compressor until it clicks into place.

NOTE: Dispose of used water trays appropriately.

WARNING:

- Do not power on or use the compressor without the filters and water tray properly installed.
- Do not insert foreign objects into any part of the ventilation system.

CAUTION: Use only a Breathe Technologies approved water tray with the ventilator.

BATTERY REPLACEMENT

Contact your Breathe Technologies service representative to make arrangements for replacing the ventilator or compressor battery if battery runtime degrades to an unacceptable level.

CAUTION: No user serviceable components are inside the device.

CHECKING AND REPLACING THE AIR INLET FILTER

The air inlet filter is used to filter ambient air and oxygen (if connected) entering through the low flow oxygen connection on the back of the compressor.

The compressor air inlet filter needs to be changed every three to six months, or as needed.

NOTE: The compressor is shipped with an air inlet filter already installed.

1 Place the patient on an alternate means of ventilation.

2 If operating in Stationary or Extended Range Configuration, power off the ventilator.

3 Power off the compressor.

4 To inspect the filter, locate the filter cover on the back of the unit. Gently press up on the small tab at the bottom of the filter cover to remove the filter cover and filter (Fig. 100 and 101).

5 Remove the used filter from the filter cover and inspect the filter.

Replace the filter if it is blocked by dirt or dust or is otherwise contaminated. If replacing the filter, obtain a replacement filter from Breathe Technologies. For more information about purchasing replacement filters, refer to "Accessories and Replacement Parts.

If the filter is clean, return it to the filter cover.

6 Close the filter cover.

NOTES:

- Reusing contaminated filters is not recommended.
- Dispose of used filters appropriately.
- The filter might need to be changed more frequently in particulate-filled environments.

WARNING:

- Do not power on or use the compressor without the filters and water tray properly installed.
- Do not insert foreign objects into any part of the ventilation system.

CAUTION: Use only a Breathe Technologies approved filter with the compressor.

CHECKING AND REPLACING THE COOLING AIR FILTER

The cooling air filter is used to filter air that enters the compressor to cool it during operation.

The compressor cooling air filter needs to be changed every three to six months, or as needed.

NOTE: The compressor is shipped with a cooling air filter already installed.

1 Place the patient on an alternate means of ventilation.

2 If operating in Stationary or Extended Range Configuration, power off the ventilator.

3 Power off the compressor.

4 To inspect the filter, locate the filter cover on the back of the unit. Remove the filter cover by gently tilting the top of the filter cover out (Fig. 102).

5 Remove the used filter from the filter cover and inspect the filter.

Replace the filter if it is blocked by dirt or dust or is otherwise contaminated. If replacing the filter, obtain a replacement filter from Breathe Technologies. For more information about purchasing replacement filters, refer to "Accessories and Replacement Parts"

If the filter is clean, return it to the filter cover.

6 Close the filter cover.

NOTES:

- Reusing contaminated filters is not recommended.
- Dispose of used filters appropriately.
- The filter might need to be changed more frequently in particulate-filled environments.

WARNING:

- Do not power on or use the compressor without the filters and water tray properly installed.
- Do not insert foreign objects into any part of the ventilation system.

CAUTION: Use only a Breathe Technologies approved filter with the compressor.

TESTING VENTILATOR ALARMS

This section gives instructions for testing ventilator alarms. Procedures described in this section are only to be performed by trained personnel.
Connect the ventilator to an AC power source or ensure the battery has sufficient charge before beginning testing.
If any test fails, contact your Breathe Technologies service representative.

RECOMMENDATIONS FOR FREQUENCY OF TESTING

In a multi-patient setting or when necessary, the ventilator must be tested before it is assigned to a new patient.
Perform a visual check of the equipment and test alarms. If the device is used in a clinical setting, refer to PL-20-0011 Life2000TM Performance Verification Testing for more information.

VERIFYING POWER@ON SELF@TEST ALARMS

1 Press the Power button to turn on the ventilator.
2 Verify that there are audible tones while powering on the ventilator.

VERIFYING BACKUP ALARM BUZZER

With the ventilator on and a Prescription Setting button pressed:

1 Wait for a Breath Timeout alarm (20 or 60 seconds, depending on the Breath Timeout setting).
2 After an additional 60 seconds, the alarm should sound with an additional buzzer that indicates the alarm has not been silenced.

CHECKING ALARM CONDITIONS

Do not test while the ventilator is being used on a patient.
These testing procedures require clinical settings to be changed. If necessary, record clinical settings before beginning.
For each test verify both corresponding alarm notifications occur and are correct:

1 The visual alarm appears on the touch screen, and
2 The audio alarm is audible, or the ventilator vibrates if in vibration mode.

NOTE: Other alarms and/or multiple alarms may occur during the testing procedure. If a different alarm occurs from the one you're testing for, use the Active Alarms button on the touch screen to display the alarm list. The alarm you are testing for may be listed in the list of additional alarms. For more information, see "Chapter 7: Alarms, Alerts, and Troubleshooting".
As long as the alarm you are testing for is listed, the test may be considered complete. If the alarm is not listed, turn off the ventilator, and turn it back on. Check that the test settings are correct and repeat the test.

EQUIPMENT REQUIRED FOR TESTING

1 Life2000TM Ventilator
2 An adjustable gas source connected to an adjustable regulator (to test the High Gas Pressure alarm, both must be able to reach 95 PSI). Either oxygen or air may be used for testing.
NOTE: The Life2000TM Compressor is not an adjustable air source.
3 An interface (either the Universal CircuitTM interface or the Pillows Interface may be used for testing).

NOTE: The interface will be handled during testing. Thoroughly clean the testing interface before using it on patient or purchase the Life2000TM Performance Verification Testing Kit. For information about ordering accessories and replacement parts, see "Accessories and Replacement Parts".

TESTING SETUP

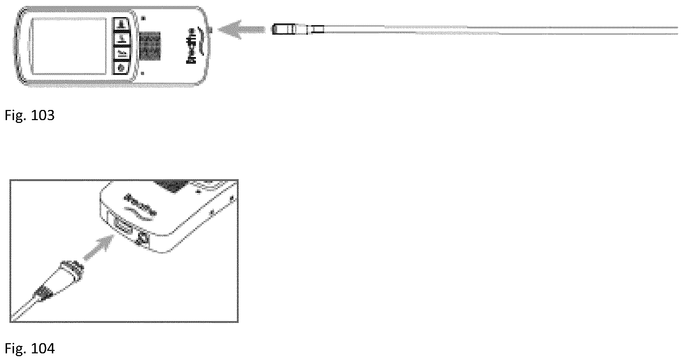

1 Undock the ventilator from the compressor. For more information, see ""Undocking the Ventilator from the Compressor".
2 Power on the ventilator. If necessary, record clinical settings before changing settings.
Using the Clinician's Menu, activate only the Low Activity Prescription Setting. Set the Ventilation Settings to:
Touch OK and then CONFIRM.
Set the Alarm Limits to:
Touch OK and then CONFIRM.
Set the Breath Timeout to:
Touch OK and then CONFIRM.
Set the Source Gas to whatever source gas is being used for testing.
3 Connect the adjustable source gas to the ventilator using an adjustable regulator set to 50 PSI (Fig. 103). Turn on the source gas to 50 PSI nominal. For more information, see "Connecting to a Cylinder" on page 51.
4 Connect the interface (see Fig 104). For more information, see "Chapter 5: Connecting an Interface".

NOTE: The interface does not need to be worn during testing.
Place the ventilator on a flat surface where the interface connection will not be jostled. Set the interface aside, with nasal pillows and tubing free of anything that might impede air flow.

1. TESTING THE HIGH GAS PRESSURE ALARM (MEDIUM PRIORITY)

1 Begin by ventilating using the Low Activity Prescription Setting. Allow the ventilator to ventilate for a few breaths.
2 With the source gas supply on and connected to the ventilator and an adjustable regulator, set the regulator to 95 PSI.
3 The top of the touch screen should display the High Gas Pressure alarm and you should hear a sequence of three tones indicating a medium-priority alarm or vibrate.
NOTES:

- If the Low PIP Pressure alarm is displayed before the High Gas Pressure alarm, increase the volume in the Ventilation Settings until the Low PIP Pressure alarm clears. Changes to the Ventilation Settings take effect only after selecting the OK and CONFIRM buttons.
- If a low- or medium-priority alarm occurs while the ventilator is in vibration mode, and the alarm is not resolved within 60 seconds, an audible alarm occurs.

4 After verifying the correct alarm notifications, adjust the regulator to 50 PSI.

The High Gas Pressure alarm will resolve itself once the regulator has been adjusted to 50 PSI.

## 2. TESTING THE LOW GAS PRESSURE ALARM (MEDIUM PRIORITY)

1 Begin by ventilating using the Low Activity setting. Allow the ventilator to ventilate for a few breaths.
2 Disconnect the source gas supply hose from the ventilator connection by pulling back on the knurled ring until the hose detaches.
3 The top of the touch screen should display the Low Gas Pressure alarm, and you should hear a sequence of three tones indicating a medium-priority alarm or vibrate. NOTE: If a low- or medium-priority alarm occurs while the ventilator is in vibration mode, and the alarm is not resolved within 60 seconds, an audible alarm occurs.
4 After verifying the correct alarm notifications, reattach the source gas supply hose to the ventilator (see Fig. 103).

The Low Gas Pressure alarm will resolve itself once the gas supply is correctly reattached.

## 3. TESTING THE HIGH DELIVERY PRESSURE ALARM (MEDIUM PRIORITY)

1 Begin by ventilating using the Low Activity setting. Allow the ventilator to ventilate for a few breaths.
2 When as soon as the breath indicator light shows a breath being delivered by the ventilator, quickly pinch the interface tubing near the ventilator connection.
3 The top of the touch screen should display the High Delivery Pressure (High Del. Pressure) alarm, and you should hear a sequence of three tones indicating a medium-priority alarm or vibrate.
NOTE: If a low- or medium-priority alarm occurs while the ventilator is in vibration mode, and the alarm is not resolved within 60 seconds, an audible alarm occurs.
4 After verifying the correct alarm notifications, release the interface tubing.

The High Delivery Pressure alarm will resolve itself once the interface has been released.

## 4. TESTING THE LOW PIP PRESSURE ALARM (MEDIUM PRIORITY) AND LOW DELIVERY PRESSURE ALARM (MEDIUM PRIORITY)

1 Begin by ventilating using the Low Activity setting. Continue to use the current Ventilation Settings. Allow the ventilator to ventilate for a few breaths.
2 Disconnect the interface from the ventilator.
3 The top of the touch screen should display the Low PIP Pressure alarm, and you should hear a sequence of three tones indicating a medium-priority alarm or vibrate.
4 Use the active alarms button at the top of the touch screen to display the alarm list; the Low Del. Pressure alarm should be listed in the alarm list.
NOTE: If a low- or medium-priority alarm occurs while the ventilator is in vibration mode, and the alarm is not resolved within 60 seconds, an audible alarm occurs.
5 After verifying the correct alarm notifications, reconnect the interface to the ventilator.
The alarms should resolve themselves once the interface is correctly reattached
6 Close the active alarm list by touching the Close button.

## 5. TESTING THE HIGH PIP PRESSURE ALARM (HIGH PRIORITY)

1 Begin by ventilating using the Low Activity setting. Continue to use the current Ventilation Settings. Allow the ventilator to ventilate for a few breaths.
2 Using the Clinician's Settings Menu, navigate to Alarm Limits and change the High PIP alarm limit to 10 cmH2O. Select OK and CONFIRM.
3 If testing with the Universal CircuitTM interface, completely plug the large opening on the patient side of the interface using the palm of your hand. Or, if testing with the Pillows Interface, use your thumbs to completely plug the nasal pillows until the ventilator alarms.

3 The top of the touch screen should display the High PIP Pressure alarm, and you should hear a sequence of two sets of five tones indicating a high priority alarm.
NOTE: For a high-priority alarm, an audible tone immediately occurs with a vibration alarm with no delay.
4 After verifying the correct alarm notifications for the High PIP Pressure alarm, release the interface and allow the alarm to resolve itself.
5 Using the Clinician's Settings Menu, navigate to Alarm Limits and return the High PIP alarm limit to 10 cmH2O.

Select OK and CONFIRM.

6. TESTING THE BREATH TIMEOUT ALARM (MEDIUM PRIORITY) AND HIGH CIRCUIT PRESSURE ALARM (HIGH PRIORITY)

1 Begin by ventilating using the Low Activity setting. Allow the ventilator to ventilate for a few breaths. Ventilation Settings
2 Using the Clinician's Settings Menu, navigate to Alarm Limits and change the BR setting to 0/min.
3 Wait up to 20 seconds to allow the Breath Timeout to trigger. The top of the touch screen should display the Breath Timeout alarm, and you should hear a sequence of three tones indicating a medium-priority alarm or vibrate.
NOTES:

- The High Del. Pressure alarm may appear before the Breath Timeout alarm.
- If a low- or medium-priority alarm occurs while the ventilator is in vibration mode, and the alarm is not resolved within 60 seconds, an audible alarm occurs.

4 After verifying the correct alarm notifications for the Breath Timeout alarm, kink the interface tubing near the connection to the ventilator.
5 Within a few seconds the top of the touch screen should display the High Circuit Pressure (High Crct Pressure), and you should hear a sequence of two sets of five tones indicating a high priority alarm.
NOTE: For a high-priority alarm, an audible tone immediately occurs with a vibration alarm with no delay.
6 After verifying the correct alarm notifications for the High Circuit Pressure alarm, release the interface tubing and allow the High Circuit Pressure alarm to resolve itself.
Touch OK in the message that indicates the alarm has been resolved.
Ventilation Settings
8 Using the Clinician's Settings Menu, navigate to Ventilation Settings and return the BR setting to 12/min.

Select OK and CONFIRM.
The Breath Timeout alarm will resolve itself once the setting is changed.

7. TESTING THE HIGH BREATH RATE ALARM (MEDIUM PRIORITY)

1 Begin by ventilating using the Low Activity setting. Continue to use the current Ventilation Settings. Allow the ventilator to ventilate for a few breaths.
2 Using the Clinician's Settings Menu, navigate to Alarm Limits and change the High BR alarm limit to 20/min. Select OK and CONFIRM.
3 Repeatedly pinch the interface tubing near the ventilator to impede, but not completely stop, airflow. Pinch more than once every three seconds to alarm.
4 The top of the touch screen should display the High Breath Rate alarm, and you should hear a sequence of three tones indicating a medium-priority alarm or vibrate.
NOTE: If a low- or medium-priority alarm occurs while the ventilator is in vibration mode, and the alarm is not resolved within 60 seconds, an audible alarm occurs.
5 After verifying the correct alarm notifications, release the interface tubing. The High Breath Rate alarm will resolve itself within a few breaths.
6 Using the Clinician's Settings Menu, navigate to Alarm Limits and return the High BR alarm limit to 40/min.

Select OK and CONFIRM.

8. TESTING THE LOW BREATH RATE ALARM (MEDIUM PRIORITY)

1 Begin by ventilating using the Low Activity setting. Continue to use the current Ventilation Settings. Allow the ventilator to ventilate for a few breaths.
2 Using the Clinician's Settings Menu, navigate to Alarm Limits and change the Low BR alarm limit to 20/min. Select OK and CONFIRM.
3 The top of the touch screen should display the Low Breath Rate alarm, and you should hear a sequence of three tones indicating a medium-priority alarm or vibrate.
NOTE: If a low- or medium-priority alarm occurs while the ventilator is in vibration mode, and the alarm is not resolved within 60 seconds, an audible alarm occurs.
4 After verifying the correct alarm notifications, release the interface tubing.
5 Using the Clinician's Settings Menu, navigate to Alarm Limits and return the Low BR alarm limit to 0/min.

Select OK and CONFIRM.
The Low BR alarm will resolve itself when the Low BR alarm limit is returned to 0/Min.

9. TESTING THE BATTERY LOW ALARM (MEDIUM-PRIORITY) AND VERY LOW BATTERY ALARM (HIGH-PRIORITY)

1 If the ventilator is connected to an AC power source, disconnect the ventilator from the power source so it is running on its internal battery.
2 Ventilate by using the High Activity Setting.
3 Allow the ventilator to ventilate until the Battery Low Alarm occurs (25% battery charge).
4 The top of the touch screen should display the Battery Low alarm and you should hear a sequence of three tones indicating a medium-priority alarm or vibrate.
NOTE: If a low- or medium-priority alarm occurs while the ventilator is in vibration mode, and the alarm is not resolved within 60 seconds, an audible alarm occurs.
5 After verifying the correct alarm notifications for the Battery Low alarm, allow the ventilator to continue ventilating at the High Activity setting. The ventilator will continue to alarm for the Battery Low alarm until the Very Low Battery alarm occurs (15% battery charge).
6 When the Very Low Battery alarm occurs, the screen should display the Very Low Battery alarm, and you should hear a sequence of two sets of five tones indicating a high priority alarm.
NOTE: For a high-priority alarm, an audible tone immediately occurs with a vibration alarm with no delay.
7 After verifying the correct alarm notifications for the Very Low Battery alarm, connect the ventilator to an AC power source and allow the internal battery to recharge.

AFTER CHECKING ALARM CONDITIONS

After testing has been completed successfully, program clinical settings for patient use and make sure that the ventilator has sufficient charge before using on a patient.

BATTERY INFORMATION

The Life2000TM Ventilation System uses lithium ion batteries with the following specifications.

VENTILATOR BATTERY SPECIFICATIONS

| SPECIFICATION | DESCRIPTION |
| --- | --- |
| Type | Lithium Ion, rechargeable, not user replaceable. |
| Ampere/hour rating | 1800 mAh |
| Maximum current | 450 mA |
| Operating Voltage | 7.4 V internal |
| Duration | Approximately 4 hours |
| Ventilator | If the battery performance degrades to unacceptable levels before the scheduled 2.5 year replacement, contact your Breathe Technologies service representative. |

Table 32

TIPS:

- A fully charged battery in good condition is designed to operate for four hours of typical use, but exact operating time depends on patient breath rate.
- If the battery has no charge, it takes approximately three to four hours to fully recharge.
- The ventilator can be used while charging.
- The ventilator sounds an alarm and displays an alarm message when the battery has less than 25% of its charge and then again when it has less than 15%.

COMPRESSOR BATTERY SPECIFICATIONS

| SPECIFICATION | DESCRIPTION |
| --- | --- |
| Type | Lithium Ion, rechargeable, not user replaceable. |
| Ampere/hour rating | 10200 mAh |
| Maximum current | 10 A |
| Operating Voltage | 28.8 V internal |
| Duration | Up to 2 hours |
| Compressor | If the battery performance degrades to unacceptable levels, contact your Breathe Technologies service representative. |

Table 33

NOTE: The compressor contains a lithium ion battery. Before shipping or traveling with the compressor, contact your transportation carrier for information.

TIPS:

- A fully charged battery in good condition is designed to operate for up to two hours of typical use, but exact operating time depends on usage conditions.
- If the battery has no charge, it takes approximately three to four hours to fully recharge.
- A fully charged battery in good condition lasts up to two hours.
- The compressor can be used while charging.
- The compressor sounds an alarm when the battery charge drops below 20% 138 139

VENTILATOR BATTERY CHARGER AND POWER CORD SPECIFICATIONS VENTILATOR BATTERY CHARGER SPECIFICATIONS

| CATEGORY | SPECIFICATION |
|---|---|
| Input AC voltage | 100-240 VAC |
| Input AC frequency | 50-60 Hz |
| Input AC current | 0.3 A max. |
| Output DC voltage | 8.4 VDC |
| Output DC current | 1.3 A |
| Insulation class | Class II |
| Electrical safety approvals | UL 60601-1, EN 60950, EN 60601-1, EN 60335-2-29 |
| Dimensions | 3.55" x 1.77" x 1.26" (90 x 45 x 32 mm) |
| Weight | 0.25 lb. (115 g) |

Table 34

VENTILATOR POWER CORD SPECIFICATIONS

| CATEGORY | SPECIFICATION |
|---|---|
| Input AC voltage | 100-240 VAC |
| Input AC frequency | 50-60 Hz |
| Input AC current | 5 A max. at 115 VAC<br>2.5 A max. at 230 VAC |
| Output DC voltage | 24 VDC |
| Output DC current | 12.5 A |
| Insulation class | Class II |
| Electrical safety approvals | ANSI/AAMI ES60601-1, cUL ES60601-1, TUV EN60601-1 3rd edition |
| Dimensions | 7.8" x 4" x 2" (198 x 102 x 51 mm) |
| Weight | 3 lb. (1.36 kg) |

Table 36

COMPRESSOR POWER CORD SPECIFICATIONS

6 ft. IEC 320-C13 compliant cord

OXYGEN MONITOR

WARNING:

To ensure accuracy of oxygen administration and to monitor for the presence of contamination (incorrect gas connected), use an external oxygen monitor to verify the oxygen concentration in the delivered gas.
Recommended oxygen monitor: TeledyneTM MX300, or similar.
To set high and low alarms for oxygen concentration and for installation information, refer to the Quick Start Guide for Model AX300 Portable Oxygen Analyzer and MX300 Portable Oxygen Monitor by Teledyne.
The Tee Adapter will be connected between the Universal CircuitTM interface and the patient mask.
NOTE: The clinician shall ensure that the patient is getting sufficient oxygen.

EXHALATION VOLUME MONITOR

WARNING:

To monitor minute volume, use an external exhaled volume monitor.

Recommended flow monitors: OhmedaR 5420, RespironicsR NM3TM, Datex-OhmedaR CardiocapTM/5, or similar.

For installation information, refer to the manufacturer's instructions.

The inline adapter will be connected between the Universal CircuitTM interface and the patient mask.

140 141

VENTILATOR ALTITUDE VOLUME ADJUSTMENT TABLE

The volume output by the ventilator is not automatically adjusted for altitude. The following table shows the actual volume output for several volume settings at different altitudes. Check the performance of the ventilator for adequate therapy delivery in the environment(s) in which it will be used and adjust the volume to compensate for altitude when necessary.

| Atmospheric Pressure | | Approximate Altitude | | VOLUME SETTING ON LIFE2000™ VENTILATION SYSTEM | | | | | |
|---|---|---|---|---|---|---|---|---|---|
| | | | | 90 | 150 | 250 | 350 | 500 | 750 |
| HPA | FEET | METERS | | ML VOLUME OUTPUT | | | | | |
| 1100 | -2290 | -700 | | 84 | 140 | 234 | 327 | 468 | 702 |
| 1075 | -1640 | -500 | | 86 | 143 | 238 | 334 | 477 | 715 |
| 1050 | -990 | -300 | | 87 | 146 | 243 | 340 | 486 | 729 |
| 1025 | -320 | -100 | | 89 | 149 | 248 | 347 | 495 | 743 |
| 1000 | 370 | 110 | | 91 | 152 | 253 | 354 | 505 | 758 |
| 975 | 1060 | 320 | | 93 | 155 | 258 | 361 | 516 | 773 |
| 950 | 1770 | 540 | | 95 | 158 | 263 | 369 | 527 | 790 |
| 925 | 2500 | 760 | | 97 | 161 | 269 | 377 | 538 | 807 |
| 900 | 3240 | 990 | | 99 | 165 | 275 | 385 | 550 | 826 |
| 875 | 4000 | 1220 | | 101 | 169 | 282 | 394 | 563 | 845 |
| 850 | 4780 | 1460 | | 104 | 173 | 288 | 404 | 577 | 865 |
| 825 | 5580 | 1700 | | 106 | 177 | 295 | 413 | 591 | 886 |
| 800 | 6400 | 1950 | | 109 | 182 | 303 | 424 | 606 | 908 |
| 775 | 7230 | 2200 | | 112 | 186 | 310 | 435 | 621 | 931 |
| 750 | 8090 | 2470 | | 115 | 191 | 319 | 447 | 638 | 957 |
| 725 | 8980 | 2740 | | 118 | 197 | 328 | 459 | 656 | 984 |
| 700 | 9880 | 3010 | | 121 | 202 | 337 | 472 | 675 | 1012 |
| 675 | 10820 | 3300 | | 125 | 209 | 348 | 487 | 695 | 1043 |
| 650 | 11780 | 3590 | | 129 | 215 | 358 | 501 | 716 | 1075 |
| 625 | 12780 | 3890 | | 133 | 222 | 370 | 517 | 739 | 1109 |
| 600 | 13800 | 4210 | | 138 | 229 | 382 | 535 | 765 | 1147 |

Table 37

Accessories and replacement parts

| PART NUMBER | PRODUCT DESCRIPTION |
|---|---|
| Interfaces (for single patient use only) | |
| BT-60-0010 | Universal Circuit™ Interface |
| BT-60-0001 | Pillows Interface, Extra Small |
| BT-60-0002 | Pillows Interface, Small |
| BT-60-0003 | Pillows Interface, Medium |
| BT-60-0004 | Pillows Interface, Large |
| Source Gas Supply Hoses | |
| BT-55-0001 | Oxygen Hose, 18" |
| BT-55-0002 | Oxygen Hose, 36" |
| BT-55-0003 | Oxygen Hose, 72" Replacement |
| BT-55-0004 | Oxygen Hose, 120" |
| BT-55-0005 | Oxygen Hose, 20' |
| BT-55-0006 | Oxygen Hose, 50' Replacement |
| BT-55-0007 | Air Hose, 18" |
| BT-55-0008 | Air Hose, 36" |
| BT-55-0009 | Air Hose, 72" |
| BT-55-0010 | Air Hose, 120" |
| BT-55-0011 | Air Hose, 20' |
| BT-55-0012 | Air Hose, 50' |
| Power Supply and Charger | |
| MS-03-1506 | External Power Supply Replacement |
| MS-03-1511 | AC Power Cord Replacement |
| MS-02-0176 | Ventilator Battery Charger Replacement |
| MS-03-0019 | Ventilator Charger Input Cordset Replacement |
| Cleaning and Purging | |
| BT-80-0001 | Air Inlet Filter Replacement |
| BT-80-0002 | Cooling Air Filter Replacement |
| BT-80-0003 | Water Tray Replacement |
| BT-55-0025 | Purge Tube Connector Replacement |
| BT-00-0001 | Purge Tube Replacement |
| Other | |
| MS-03-0772 | Belt Clip Replacement |
| BT-00-0017 | Pole Mount |
| BT-00-0014 | Regulator Replacement |
| BT-20-0003 | Performance Verification Testing Kit |

Table38

The Life2000TM Ventilation System utilizes an electromechanical pneumatic system under the control of a microprocessor to deliver patient ventilation. The following description and diagram illustrate the major components of the ventilation system.

OPERATION SUMMARY

The ventilation system uses ambient air and optional supplemental low-pressure oxygen (see diagram below).
Air and optional supplemental oxygen enter through an inlet filter. The system pressurizes the air and optional supplemental oxygen in the compressor as shown in the diagram below. The compressed gas is then delivered to the safety valve and then to the flow valve, which controls all inspiratory gas flow to the patient. A flow sensor is also provided to measure delivered flow to the patient, and the closed-loop control system ensures the delivery of the required flow to the patient. A pressure sensor is also used to measure the pressure at the patient connection to ensure patient safety. The electromechanical pneumatic system also controls PEEP during exhalation.

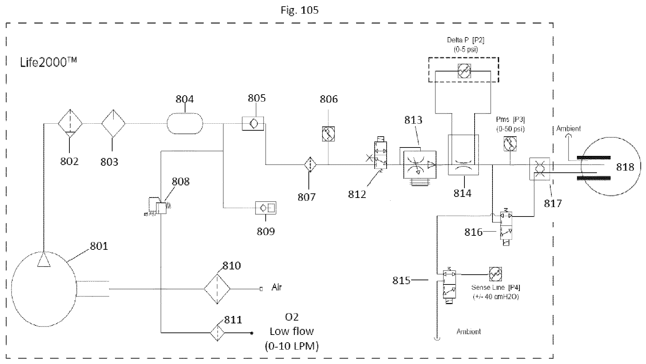

Fig. 105 shows a pneumatic diagram of the LIFE2000™ ventilation system. The reference numerals refer to a compressor 801, a filter/water separator 802, a dryer 803, a 1L accumulator 804, a connector 805, a Psource_gas [P1] (0-100psi) 806, a sintered bronze filter 807, a pressure relief (NC)(65 PSI) 808, a DISS 1240 809, an inlet filter 810, a 5 μ filter 811, a safety valve (normally closed) 812, a pressure compensated proportional valve 813, a Delta P flow sensor 814, a sense line Autozero valve 815, a purge valve 816, a single connector to internal manifold 817, and a patient 818.

The Breathe Technologies Life2000TM Ventilation System is classified per IEC 60601-1 as portable, Class II, Type
BF, Drip-proof (IPX1), continuous operation.

PERFORMANCE SPECIFICATIONS

| PARAMETER | DESCRIPTION |
| --- | --- |
| General Information | |
| Available ventilation modes | Volume ventilation with Control, Assist/Control, and Assist ventilation modes |
| Connection to patient | Breathe Pillows Interface or Universal Circuit™ Interface |
| Features And Description | |
| Frequency | Ventilator can cycle at ≤40 BPM |
| | Accuracy: ±10% or 1 BPM, whichever is greater |
| Tidal Volume | Up to 2,000 ml |
| Volume output range | Range: 50 ml to 750 ml |
| | Resolution: 10 ml |
| | Accuracy: ±10% or 10 ml, whichever is greater, measured at ATP (ambient temperature and pressure) |
| Volume output settings | 3 programmable Prescription Settings can provide different volume outputs as prescribed by the physician |
| | Each setting is clinician definable between 50 ml to 750 ml |
| Volume output adjustment | Volume output may be adjusted by patient as prescribed by physician using the available programmable prescription settings. |
| Inspiratory Time (I-Time) | Range: 150 ms - 3,000 ms |
| | Resolution: 50 ms |
| | Accuracy: ±50 ms |
| Delivered peak gas flow rate from ventilator | 8 LPM minimum |
| | 40 LPM maximum |
| Volume control | Closed loop proportional valve system |
| PEEP | Range: 0–10 cmH₂O |
| | Resolution: 1 cmH₂O |
| | Accuracy: ±2 cmH₂O |
| PIP Monitor | 0–40 cmH₂O |
| Means of triggering | The breath delivery is triggered depending on the breath type—mandatory or assisted—and ventilation mode. The sensitivity setting, adjustable in increments of 1, ranges from 0 to 9, with 0 being the most sensitive and 9 being the least sensitive. |
| Inspiratory trigger delay time | Volume delivery is initiated based on the breath type and ventilation mode. |
| Available waveforms | Square waveform |

Table 39

Performance Specifications

| PARAMETER | DESCRIPTION |
|---|---|
| Means of initiating and terminating inspiratory phases | Control ventilation mode: The inspiratory phase is started and terminated by the ventilator based on set inspiratory time and respiratory rate.<br><br>Assist/Control ventilation mode: The inspiratory phase is started based on the patient's breath triggering or by the ventilator based on set inspiratory time and terminated by the set inspiratory time.<br><br>Assist ventilation mode: The inspiratory phase is started based on the patient's breath triggering and terminated by the set inspiratory time. |
| Backup ventilation parameters | If a **Breath Timeout** alarm is triggered, the ventilator delivers the set volume at a continuous flow rate of 3 LPM or 12 BPM, preset by a clinician. When a patient's breath is detected again, the ventilator delivers the set volume based on **Ventilation Settings** parameters. |
| Other Operating Requirements And Features | |
| Inspiratory pressure relief | Pressure is relieved through the patient's mouth or through the patient interface. |
| Expiratory resistance at patient connection | Breathe Pillows Interface:<br>Small: 1.2 cmH$_2$O at 30 LPM, 4.7 cmH$_2$O at 60 LPM<br>Medium: 1.0 cmH$_2$O at 30 LPM, 3.9 cmH$_2$O at 60 LPM<br>Large: 0.8 cmH$_2$O at 30 LPM, 2.2 cmH$_2$O at 60 LPM<br><br>Universal Circuit™ Interface:<br>0.4 cmH$_2$O at 30 LPM, 1.4 cmH$_2$O at 60 LPM |
| Inspiratory resistance at patient connection | Breathe Pillows Interface:<br>Small: 1.3 cmH$_2$O at 30 LPM, 5.1 cmH$_2$O at 60 LPM<br>Medium: 1.1 cmH$_2$O at 30 LPM, 4.5 cmH$_2$O at 60 LPM<br>Large: 1.0 cmH$_2$O at 30 LPM, 4.2 cmH$_2$O at 60 LPM<br><br>Universal Circuit™ Interface:<br>0.5 cmH$_2$O at 30 LPM |
| Breath sensing line purge flow | A flow of gas is delivered through the sensing lumen to keep the sensing line patent. |
| Fail safe mechanisms | Safety valve prevents overpressure condition in lung, mitigating breath stacking |
| Alternate input pressure source requirements | 41 PSI to 87 PSI, oxygen or air source gas<br>Compatible sources:<br>• Compressed oxygen or air gas cylinders<br>• Compressed oxygen or air wall sources<br>• Any other medical grade oxygen or air sources meeting the following criteria: 41 PSI to 87 PSI and ≥ 40 LPM flow continuous output @ 41 PSI |
| Range, resolution, and accuracy of respiratory rate monitor | Range: 1 BPM to 120 BPM<br>Resolution: 1 BPM<br>Accuracy: ±10% or 1 BPM, whichever is greater, up to 50 BPM |

Table 40

| PARAMETER | DESCRIPTION |
|---|---|
| Range, resolution, and accuracy of oxygen or air minute volume display | Range: 0 LPM to 20 LPM.<br>Resolution: 0.1 LPM.<br>Accuracy: ±10% or ±(10 ml x average breath rate), whichever is greater.<br>NOTE: The displayed air or oxygen minute volume is the product of the average breath rate times the average measured air or oxygen volume settings.<br>During continuous delivery, the ventilator displays an oxygen or air minute volume of 3.0 LPM. |
| Oxygen source time (stationary oxygen) | Indefinite |
| Usage types | Stationary use: place on table or flat surface.<br>Extended Range use with oxygen hose.<br>Stand-alone use with cylinder or alternate gas source. |
| **Ventilator Battery Specifications** | |
| Type | Lithium Ion, rechargeable, not user replaceable. |
| Ampere/hour rating | 1800 mAh |
| Maximum current | 450 mA |
| Operating Voltage | 7.4 V internal |
| Duration | Approximately 4 hours |
| Ventilator | If the battery performance degrades to unacceptable levels before the scheduled 2.5 year replacement, contact your Breathe Technologies service representative. |
| **Compressor Battery Specifications** | |
| Type | Lithium Ion, rechargeable, not user replaceable. |
| Ampere/hour rating | 10200 mAh |
| Maximum current | 10 A |
| Operating Voltage | 28.8 V internal |
| Duration | Up to 2 hours |
| Compressor | If the battery performance degrades to unacceptable levels, contact your Breathe Technologies service representative. |
| **Special Features** | |
| Alarm vibrate | Instead of an audio alarm, the user can enable the alarm to vibrate for medium- and low-level alarms. |
| Touch screen flip | User can flip the screen orientation 180°. |
| Universal Circuit™ Interface | Used to connect commercially available non-invasive masks (full face, nasal, and pillows) or tracheostomy tubes for invasive ventilation |
| **Acoustic** | |
| Ventilator | |
| Compressor | |

Table 41

| PARAMETER | DESCRIPTION |
|---|---|
| Ventilator Alarm Notifications | |
| High Priority Alarm | At Maximum loudness setting: 73 db(A)<br>At Minimum loudness setting: 60 db(A) |
| Medium Priority Alarm | At Maximum loudness setting: 72 db(A)<br>At Minimum loudness setting: 59 db(A) |
| buzzer | db(A) tbd |
| Ventilator Alarms | |
| High Circuit Pressure | Interface may be pinched or kinked. |
| High PEEP Pressure | Interface may be blocked. |
| High PIP Pressure | Peak Inspiratory Pressure (PIP) exceeds the set limit. |
| High Temperature | CPU or battery temperature is above the allowable limit. |
| Very Low Battery | Ventilator battery capacity drops below 15%. |
| Battery Low | Ventilator battery capacity drops below 25%. |
| Breath Timeout | No breath is detected for 20 seconds or 60 seconds, depending on the setting. |
| High Breath Rate | Respiratory rate exceeds the set limit. |
| High Delivery Pressure | Interface pressure during delivery exceeds the maximum expected. |
| High Gas Pressure | Source gas pressure exceeds the allowable limit (95 PSI). |
| Low Breath Rate | Respiratory rate falls below the set limit. |
| Low Delivery Pressure | Interface pressure during delivery fails to exceed the minimum expected. |
| Low Gas Pressure | Source gas pressure drops below the allowable limit (35 PSI). |
| Low PIP Pressure | PIP below set limit |
| System Fault | Internal fault detected during operation. |
| POST System Fault | System fault detected during ventilator power on. |
| Compressor Alerts | |
|  |  |
|  |  |
| Compressor Alert Notifications | |
|  | db(A) tbd |
|  | db(A) tbd |

Table 42

| PARAMETER | DESCRIPTION |
|---|---|
| Monitors and indicators | |
| Ventilator user interface | Touch screen LCD for settings, monitors, and alarms. Mechanical buttons for on/off, alarm silence, and prescription settings |
| Pressure monitoring | Ventilator output pressure and airway pressure are monitored continuously. Airway pressure is monitored using a lumen in the gas delivery circuit wall, which terminates in a sensing port on the distal end of the circuit. |
| Volume Setting displays | Delivered volume setting is displayed. |
| Ventilator breath indicator | An LED visual indicator indicating an inspiration. |
| Compressor battery indicator | When the compressor is powered on, the internal battery level is displayed using indicator lights in the battery charge scale. An alarm occurs when battery capacity is below 20%. |
| Ventilator battery indicator | Indicates percentage of battery charge remaining. Always displayed on touch screen. Alarm occurs when capacity is below 25% and again at 15%. |
| Ventilator AC Battery Charger Specifications | |
| Input AC voltage | 100-240 VAC |
| Input AC frequency | 50-60 Hz |
| Input AC current | 0.3 A max |
| Output DC voltage | 8.4 VDC |
| Output DC current | 1.3 A |
| Insulation class | Class II |
| Electrical safety approvals | UL 60601-1, EN 60950, EN 60601-1, EN 60335-2-29 |
| Dimensions | 3.55" x 1.77" x 1.26" (90 x 45 x 32 mm) |
| Weight | 0.25 lb. (115 g) |
| Compressor Power Cord Specifications | |
| Cord length | 6 ft. |
| Compliance | IEC 320-E7 |
| Compressor Power Supply Specifications | |
| Input AC voltage | 100-240 VAC |
| Input AC frequency | 50-60 Hz |
| Input AC current | 5 A max. at 115 VAC 2.5 A max. at 230 VAC |
| Output DC voltage | 24 VDC |
| Output DC current | 12.5 A |
| Insulation class | Class II |
| Electrical safety approvals | ANSI/AAMI ES60601-1, cUL ES60601-1, TUV EN60601-1 3rd edition |
| Dimensions | 7.8" x 4" x 2" (198 x 102 x 51 mm) |
| Weight | 3 lb. (1.36 kg) |

Table 43

| PARAMETER | DESCRIPTION |
|---|---|
| Compressor Power Cord Specifications | |
| Cord length | 6 ft. |
| Compliance | IEC 320-C13 |
| Compressor Output (at sea level and normal ambient conditions) | |
| Pressure | 58 PSI ±15% |
| Flow | 17–21 LPM |
| Storage and Transport Environment | |
| Storage & Transport Temperature | -20°C to +60°C (-4°F to +140°F) |
| Storage & Transport Humidity | 10% to 95% non-condensing |
| Storage & Transport Altitude | 1100 hPa to 624 hPa or -2290 ft. to +8000 ft. (-700 m to +2439 m) |
| Operating Environment | |
| Operating Temperature | +5°C to +40°C (+41°F to +104°F) |
| Operating Humidity | 10% to 95% non-condensing |
| Operating Altitude | 1100 hPa to 624 hPa or -2290 ft to +8000 ft (-700 m to +2439 m) |
| NOTE: The performance of the compressor may degrade in high temperature, high humidity, or high altitude environments. If degradation is seen, switch to an alternate means of ventilation. | |
| Physical Features | |
| Ventilator weight | Approximately 1 lb. |
| Ventilator size | 3 ⅛" x 1 ¼" x 7 ½" |
| Compressor weight | Approximately 15.5 lb. |
| Compressor size | 9" x 12" x 9" (H x W x D) |
| Phthalates | The ventilation system does not contain phthalates. |
| Latex | The ventilation system is not manufactured with natural rubber latex. |
| Expected Service Life | |
| Expected Service Life | The Life2000™ Ventilation System has a five year design life when operated in accordance with the *Instructions For Use* provided. Do not use the ventilation system past its expected service date. The battery shall be replaced if performance degrades to an unacceptable level during the 2 year warranty period. |
| Service Interval | |
| Service Interval | One year |

Table 44

IEC COMPLIANCE: EN/IEC 60601-1-2:2001 (Medical electrical equipment Part 1-2: General requirements for safety-Collateral standard: Electromagnetic compatibility-Requirements and tests) defines the RF emissions limits and minimum RF immunity requirements for different types of medical electrical equipment. The following tables include the RF emissions limits and RF immunity requirements for, and results of tests upon, the Breathe

Technologies Life2000TM Ventilation System.

The Breathe Technologies Life2000TM Ventilation System needs special precautions regarding electromagnetic compatibility (EMC) and needs to be installed and put into service according to the EMC information provided in this manual.

The use of accessories, transducers and cables other than those specified by Breathe Technologies may result in increased emissions or decreased immunity of the Breathe Technologies Life2000TM Ventilation System.

| GUIDANCE AND MANUFACTURER'S DECLARATION: ELECTROMAGNETIC EMISSIONS | | |
|---|---|---|
| The Breathe Technologies Life2000™ Ventilation System is intended for use in the electromagnetic environment specified below. The customer or user of the ventilation system should ensure that it is used in such an environment. | | |
| Emission Test | Compliance | Electromagnetic Environment - Guidance |
| RF emissions CISPR 11 | Group 1 | The Breathe Technologies Life2000™ Ventilation System uses RF energy only for its internal function. Therefore, its RF emissions are very low and are not likely to cause any interference in nearby electronic equipment. |
| RF emissions CISPR 11 | Class B* | The Breathe Technologies Life2000™ Ventilation System is suitable for use in all establishments, including domestic establishments and those directly connected to the public low-voltage power supply network that supplies buildings used for domestic purposes. |
| Harmonic emissions IEC 61000-3-2 | Complies** | |
| Voltage fluctuations/flicker emissions IEC 61000-3-3 | Complies** | |
| * The ventilation system complies with Class B requirements. | | |
| ** The ventilation system does not produce a minimum of 75 watts and does not produce flicker | | |

Table 45

| GUIDANCE AND MANUFACTURER'S DECLARATION: ELECTROMAGNETIC IMMUNITY | | | |
|---|---|---|---|
| The Breathe Technologies Life2000™ Ventilation System is intended for use in the electromagnetic environment specified below. The customer or user of the ventilation system should ensure that it is used in such an environment. | | | |
| Immunity Test | IEC 60601 Test Level | Compliance Level | Electromagnetic Environment - Guidance |
| Electrostatic discharge (ESD) IEC 61000-4-2 | ±6 kV contact ±8 kV air | **VENTILATOR** ±6 kV contact ±15 kV air **COMPRESSOR** ±6 kV contact ±8 kV air | Floors should be wood, concrete or ceramic tile. If floors are covered with synthetic material, the relative humidity should be at least 30%. |
| Electrical fast Transient/burst IEC 61000-4-4 | ±2 kV for power supply lines ±1 kV for input/output lines | ±2 kV for power supply lines ±1 kV for input/output lines | Mains power quality should be that of a typical commercial or hospital environment. |
| Surge IEC 61000-4-5 | ±1 kV line(s) to line(s) ±2 kV line(s) to earth | ±1 kV line(s) to line(s) N/A | Mains power quality should be that of a typical commercial or hospital environment. |
| Voltage dips, short interruptions and voltage variations on power supply input lines IEC 61000-4-11 | <5% $U_T$ ( 95% dip in $U_T$ for 0.5 cycle) | <11.5 V (>95% dip in 230 V) and <6 V (>95% dip in 120 V) for 0.5 cycle | Mains power quality should be that of a typical commercial or hospital environment. |
| | 40% $U_T$ ( 60% dip in $U_T$ for 5 cycles) | 92 V (60% dip in 230 V) and 48 V (60% dip in 120 V) for 5 cycles | |
| | 70% $U_T$ ( 30% dip in $U_T$ for 25 cycles) | 161 V (30% dip in 230 V) and 84 V (30% dip in 120 V) for 25 cycles | |
| | <5% $U_T$ (> 95% dip in $U_T$ for 5 seconds) | <11.5 V (>95% dip in 230 V) and <6 V (>95% dip in 120 V) for 5 seconds | |
| Power frequency (50/60 Hz) magnetic field IEC 61000-4-8 | 3 A/m | 3 A/m | Power frequency magnetic fields should be at levels characteristic of a typical location in a typical commercial or hospital environment. |

Table 46

| GUIDANCE AND MANUFACTURER'S DECLARATION: ELECTROMAGNETIC IMMUNITY | | | |
|---|---|---|---|
| The Breathe Technologies Life2000™ Ventilation System is intended for use in the electromagnetic environment specified below. The customer or user of the ventilation system should ensure that it is used in such an environment. | | | |
| BREATHE TECHNOLOGIES LIFE2000™ VENTILATOR | | | |
| Immunity Test | IEC 60601 Test Level | Compliance Level | Electromagnetic Environment - Guidance |
| Conducted RF IEC 61000-4-6 | 3 Vrms 150 kHz to 80 MHz | 10 Vrms 150 kHz to 80 MHz | Portable and mobile RF communications equipment should be used no closer to any part of the Breathe Technologies Life2000™ Ventilator, including cables, than the recommended separations distance calculated from the equation applicable to the frequency of the transmitter. Recommended separation distance: $d = 1.17 \sqrt{P}$ 150kHz to 80MHz $d = 0.35 \sqrt{P}$ 80MHz to 800 MHz $d = 0.70 \sqrt{P}$ 800 MHz to 2.5GHz |
| Radiated RF IEC 61000-4-3 | 3 V/m 80 MHz to 2.5 GHz | 10 V/m 26 MHz to 1 GHz | Where P is the maximum output power rating of the transmitter in watts (W) according to the transmitter manufacturer and d is the recommended separation distance in meters (m). Field strengths from fixed RF transmitters, as determined by an electromagnetic site survey* should be less than the compliance level in each frequency range. Interference may occur in the vicinity of equipment marked with the following symbol: |
| NOTE 1: $U_n$ is the AC mains voltage prior to application of the test level. NOTE 2: At 80 MHz, the higher frequency range applies. NOTE 3: These guidelines may not apply in all situations. Electromagnetic propagation is affected by absorption and reflection from structures, objects and people. | | | |
| * Field strengths from fixed transmitters, such as base stations for radio (cellular/cordless) telephones and land mobile radios, amateur radio, AM and FM radio broadcast and TB broadcast cannot be predicted theoretically with accuracy. To assess the electromagnetic environment due to fixed RF transmitters, and electromagnetic site survey should be considered. If the measured field strength in the location in which the ventilation system is used exceeds the applicable RF compliance level above, the ventilation system should be observed to verify normal operation. If abnormal performance is observed, additional measures may be necessary, such as reorienting or relocating the ventilation system. | | | |

Table 47

## GUIDANCE AND MANUFACTURER'S DECLARATION: ELECTROMAGNETIC IMMUNITY

The Breathe Technologies Life2000™ Ventilation System is intended for use in the electromagnetic environment specified below. The customer or user of the ventilation system should ensure that it is used in such an environment.

### BREATHE TECHNOLOGIES LIFE2000™ VENTILATOR

| Immunity Test | IEC 60601 Test Level | Compliance Level | Electromagnetic Environment - Guidance |
|---|---|---|---|
| Conducted RF IEC 61000-4-6 | 3 Vrms 150 kHz to 80 MHz | 10 Vrms 150 kHz to 80 MHz | Portable and mobile RF communications equipment should be used no closer to any part of the Breathe Technologies Life2000™ Ventilator, including cables, than the recommended separations distance calculated from the equation applicable to the frequency of the transmitter. Recommended separation distance: $d = 1.17 \sqrt{P}$ 150kHz to 80MHz $d = 0.35 \sqrt{P}$ 80MHz to 800 MHz $d = 0.70 \sqrt{P}$ 800 MHz to 2.5GHz |
| Radiated RF IEC 61000-4-3 | 3 V/m 80 MHz to 2.5 GHz | 10 V/m 26 MHz to 1 GHz | Where P is the maximum output power rating of the transmitter in watts (W) according to the transmitter manufacturer and d is the recommended separation distance in meters (m). Field strengths from fixed RF transmitters, as determined by an electromagnetic site survey* should be less than the compliance level in each frequency range. Interference may occur in the vicinity of equipment marked with the following symbol: |

NOTE 1: U₁ is the AC mains voltage prior to application of the test level.
NOTE 2: At 80 MHz, the higher frequency range applies.
NOTE 3: These guidelines may not apply in all situations. Electromagnetic propagation is affected by absorption and reflection from structures, objects and people.

* Field strengths from fixed transmitters, such as base stations for radio (cellular/cordless) telephones and land mobile radios, amateur radio, AM and FM radio broadcast and TB broadcast cannot be predicted theoretically with accuracy. To assess the electromagnetic environment due to fixed RF transmitters, and electromagnetic site survey should be considered. If the measured field strength in the location in which the ventilation system is used exceeds the applicable RF compliance level above, the ventilation system should be observed to verify normal operation. If abnormal performance is observed, additional measures may be necessary, such as reorienting or relocating the ventilation system.

Table 48

| RECOMMENDED SEPARATION DISTANCES BETWEEN PORTABLE AND MOBILE RF COMMUNICATION EQUIPMENT AND THE BREATHE TECHNOLOGIES LIFE2000™ VENTILATION SYSTEM | | |
|---|---|---|
| The Breathe Technologies Life2000™ Ventilation System is intended for use in an electromagnetic environment in which radiated RF disturbances are controlled. The clinician can help prevent electromagnetic interferences by maintaining a minimum distance between portable and mobile RF communications equipment (transmitters) and the ventilation system as recommended in this table, according to the maximum output power of the communications equipment. | | |
| BREATHE TECHNOLOGIES LIFE2000™ VENTILATOR | | |
| Rated maximum output power of transmitter (W) | Separation distance according to frequency of transmitter (m) | | |
| | 150 kHz to 80 MHz $d = 1.17 \sqrt{P}$ | 80 MHz to 800 MHz $d = 0.35 \sqrt{P}$ | 800 MHz to 2.5 GHz $d = 0.7 \sqrt{P}$ |
| 0.01 | 0.17 | 0.035 | 0.070 |
| 0.1 | 0.37 | 0.11 | 0.22 |
| 1 | 1.17 | 0.35 | 0.70 |
| 10 | 3.69 | 1.1 | 2.2 |
| 100 | 11.70 | 3.5 | 7.0 |
| BREATHE TECHNOLOGIES LIFE2000™ COMPRESSOR | | |
| Rated maximum output power of transmitter (W) | Separation distance according to frequency of transmitter (m) | | |
| | 150 kHz to 80 MHz $d = 1.2 \sqrt{P}$ | 80 MHz to 800 MHz $d = 1.2 \sqrt{P}$ | 800 MHz to 2.5 GHz $d = 3.5 \sqrt{P}$ |
| 0.01 | 0.12 | 0.12 | 0.23 |
| 0.1 | 0.38 | 0.38 | 0.73 |
| 1 | 1.2 | 1.2 | 3.5 |
| 10 | 3.8 | 3.8 | 7.3 |
| 100 | 12 | 12 | 23 |
| NOTE 1: At 80 MHz and 800 MHz, the separation distance for the higher frequency range applies. NOTE 2: These guidelines may not apply in all situations. Electromagnetic propagation is affected by absorption and reflection from structures, objects and people. For transmitters rated at a maximum output power not listed above, the recommended separations distance (d) in meters (m) can be estimated using the equation applicable to the frequency of the transmitter, where P is the maximum output power rating of the transmitter in watts (W) according to the transmitter manufacturer. | | |

Table 49

| ICON | WHERE USED | MEANING |
|---|---|---|
| ⊕ | Ventilator | Indicates communication port. This port is only used by the manufacturer. |
| △ | Ventilator | Indicates power-in port. |
| ▬ ▬ ▬ | Ventilator and compressor | Indicates direct current. |
| 🔔 | Ventilator and compressor | The Silence Alarm button silences a vibration or audible alarm for 60 seconds. |
| 🔔 | Ventilator touch screen | Displayed in the **Active Alarms** window of the touch screen if an active alarm has not been silenced. |
| 🔕 | Ventilator touch screen | Displayed in the **Active Alarms** window of the touch screen if an active alarm has been silenced.<br>Displayed on the bottom of touch screen if all active alarms have been silenced. |
| 🔧 | Ventilator touch screen | Displayed on touch screen. Touching the **Wrench** button displays the **Menu** screen. |
| FLIP | Ventilator touch screen | Displayed on touch screen. Touching the **Flip** button rotates the touch screen 180°. |
| ≈ | Ventilator touch screen | Displayed on touch screen if ventilator is in vibrate mode. |
| EC REP | Ventilator label and compressor label | Authorized representative for Breathe Technologies |
| REF | Ventilator label and compressor label | Indicates catalog number and denotes the location of the part number |
| SN | Ventilator label and compressor label | Indicates serial number and denotes the location of the serial number |
| IP22 | Ventilator label | Indicates ingress protection levels of 2 for solid particle protection and 2 for liquid ingress protection. |
| IP12 | Compressor label | Indicates ingress protection levels of 1 for solid particle protection and 2 for liquid ingress protection. |

Table 50

| ICON | WHERE USED | MEANING |
|---|---|---|
| | Ventilator touch screen | Displayed on touch screen if ventilator battery status is unknown or charge is critically low (< 5%). |
| | Ventilator touch screen | Displayed on touch screen if ventilator battery has approximately 5-14% of its charge remaining. |
| | Ventilator touch screen | Displayed touch screen if ventilator battery has approximately 15–35% of its charge remaining. |
| | Ventilator touch screen | Displayed on touch screen if ventilator battery has approximately 36–56% of its charge remaining. |
| | Ventilator touch screen | Displayed on touch screen if ventilator battery has approximately 57–79% of its charge remaining. |
| | Ventilator touch screen | Displayed on touch screen if ventilator battery charge has approximately 80–100% of its charge remaining. |
| | Ventilator touch screen | Displayed on touch screen if battery is charging. |
| | Ventilator label and compressor label | Indicates BF type equipment. Device isolates the patient from any live voltage in the equipment. |
| | Ventilator label, compressor label, ventilator battery charger label, and compressor external power supply | Indicates a Class II device. Device is double insulated and does not require a safety connection to electrical earth (US: ground). |
| | Ventilator label and compressor label | Consult *Instructions for Use*. |
| | Ventilator label and compressor label | Indicates product emits non-ionizing radiation. |
| | Ventilator label | Indicates product is a Bluetooth wireless compatible device that emits radio frequency waves. |
| | Compressor label | Keep dry. Indicates that the equipment needs to be protected from moisture. |

Table 52

| ICON | WHERE USED | MEANING |
|------|-----------|---------|
| | Ventilator label, compressor label, compressor external power supply | Indicates disposal of device must conform to WEEE Directive (Waste in Electrical and Electronic Equipment) 2011/65/EU. |
| | Ventilator battery charger label | This symbol is used to support the Battery Directive 2006/66/EC. |
| CE | Ventilator battery charger label and compressor external power supply | Indicates the battery charger meets European Economic Area standards for use. |
| | Ventilator label and compressor label | Indicates manufacturer and denotes manufacturer name and address. |
| | Ventilator label and compressor label | Indicates the date of manufacture |
| | Ventilator battery charger label and compressor external power supply | Indicates product meets US standards for use with medical electrical equipment. |
| | Ventilator battery charger label | Consult *Instructions for Use*. |
| | Ventilator label, compressor label, and documentation | Documentation includes important information that must be read before using device. |
| | Ventilator battery charger label | Indicates **indoor use only** and denotes that it should only be used indoors |
| | Ventilator battery charger label | Indicates battery charge. The black shaded area represents the amount of charge within the battery. |
| FC | Compressor external power supply | Certifies that the electromagnetic interference from the device is under limits approved by the Federal Communications Commission |
| VI | Compressor external power supply | Indicates compliance with the latest DOE Efficiency Level VI requirements for average efficiency and standby power |

Table 53

| ICON | WHERE USED | MEANING |
|---|---|---|
| RoHS | Compressor external power supply | |
| (unlock icon) | Compressor | Indicates the unlocking knob is in the unlocked position. |
| (lock icon) | Compressor | When lit, indicates the locking knob is in the locked position. |
| (battery icon) | Compressor | Battery Charge Status button |
| (power icon) | Compressor | Power on/off button |
| (power icon) | Ventilator | Power on/off button |
| (activity icon) | Ventilator and ventilator touch screen | On ventilator, the **Low Activity Prescription Setting** button delivers prescription parameters set by a clinician in the **Clinician's Settings** menu. Displayed on the **Prescription Settings** screen as a label for the **Low Activity Prescription Setting** parameters set by a clinician. Displayed on the touch screen if the ventilator is set to **Low Activity Prescription Setting**. |
| (activity icon) | Ventilator and ventilator touch screen | On ventilator, the **Medium Activity Prescription Setting** button delivers prescription parameters set by a clinician in the **Clinician's Settings** menu. Displayed on the **Prescription Settings** screen as a label for the **Medium Activity Prescription Setting** parameters set by a clinician. Displayed on the touch screen if the ventilator is set to **Medium Activity Prescription Setting**. |
| (activity icon) | Ventilator and ventilator touch screen | On ventilator, the **High Activity Prescription Setting** button delivers prescription parameters set by a clinician in the **Clinician's Settings** menu. Displayed on the **Prescription Settings** screen as a label for the **High Activity Prescription Setting** parameters set by a clinician. Displayed on the touch screen if the ventilator is set to **High Activity Prescription Setting**. |

Table 54

**Claims**

1. A ventilatory support apparatus (10) transitionable between a stationary configuration and an extended range configuration, the ventilatory support apparatus (10) comprising:

   a compressor unit (14), the compressor unit (14) comprising:

   a compressor (83) for providing compressed gas;
   a ventilator dock (42) having a ventilator dock compressed gas output port (43) and a ventilator dock ventilation gas inlet port (45);
   a compressor unit ventilation gas output port (60); and
   a compressor unit compressed gas output port (58);

   a ventilator (12) for providing ventilation gas, the ventilator (12) being configured for removable docking at the ventilator dock (42), the ventilator comprising:

a ventilator ventilation gas output port (38); and
a ventilator compressed gas inlet port (40); and

a patient interface (80) for receiving ventilation gas and delivering ventilation gas to a patient, the patient interface (80) having a patient interface gas inlet port (81) transitionable between placement in fluid communication with the compressor unit ventilation gas output port (60) and placement in fluid communication with the ventilator ventilation gas output port (38);

wherein, when the ventilatory support apparatus (10) is transitioned to the stationary configuration, the ventilator (12) is docked at the ventilator dock (42), the ventilator compressed gas inlet port (40) is in fluid communication with the ventilator dock compressed gas output port (43), the ventilator ventilation gas output port (38) is in fluid communication with the ventilator dock ventilation gas inlet port (45), and the patient interface gas inlet port (81) is in fluid communication with the compressor ventilation gas output port (60), such that, in use, compressed gas is provided by the compressor unit (14) to the ventilator (12) and ventilation gas is returned to the compressor unit (14) for subsequent output from the compressor unit (14) to the patient interface; and

wherein, when the ventilatory support apparatus (10) is transitioned to the extended range configuration, the ventilator (12) is not docked at the ventilator dock (42), the ventilator compressed gas inlet port (40) is in fluid communication with the compressor unit compressed gas output port (58) via a compressed gas hose (84), and the patient interface gas inlet port (81) is in fluid communication with the ventilator ventilation gas output port (38), such that, in use, compressed gas is provided by the compressor unit (14) to the ventilator (12) and ventilation gas is provided by the ventilator (12) to the patient interface without being returned to the compressor unit (14).

2. The ventilatory support apparatus (10) according to claim 1, further wherein the ventilatory support apparatus (10) is transitionable between the stationary configuration, the extended range configuration, and a stand-alone configuration, wherein, when the ventilatory support apparatus (10) is transitioned to the stand-alone configuration, the ventilator (12) is not docked at the ventilator dock (42), the ventilator compressed gas inlet port (40) is in fluid communication with an external compressed gas source, and the patient interface gas inlet port (81) is in fluid communication with the ventilator ventilation gas output port (38), such that, in use, compressed gas is provided by the external compressed gas source to the ventilator (12) and ventilation gas is provided by the ventilator (12) to the patient interface without passing through the compressor unit (14).

3. The ventilatory support apparatus (10) of Claim 2, wherein the compressor unit (14) further comprises a low flow gas input port.

4. The ventilatory support apparatus (10) of Claim 3, wherein the low flow gas input port comprises a low flow oxygen input port.

5. The ventilatory support apparatus (10) of Claim 1 or 2, wherein the compressor (83) is configured to compress ambient air.

6. The ventilatory support apparatus (10) of Claim 5, wherein the compressor unit (14) further comprises one or more ambient air apertures for introducing ambient air to the compressor (83).

7. The ventilatory support apparatus (10) of Claim 6, wherein the one or more ambient air apertures further comprises a filter.

8. The ventilatory support apparatus (10) of Claim 1 or 2, wherein the compressor unit compressed gas output port (58) comprises a Diameter Index Safety System coupling.

9. The ventilatory support apparatus (10) of Claim 1 or 2, wherein the ventilator (12) is electrically powered and includes a rechargeable battery.

10. The ventilatory support apparatus (10) of Claim 9, wherein the ventilator dock (42) is configured to provide electrical power for powering the ventilator (12) and for recharging the rechargeable battery when the ventilator (12) is docked at the ventilator dock (42).

11. The ventilatory support apparatus (10) of Claim 1 or 2, wherein the ventilator (12) further comprises a user interface and a wireless transmitter, wherein the compressor unit (14) further comprises a wireless receiver, and wherein the compressor (83) is controllable by signal transmission from the wireless transmitter to the wireless receiver initiated by user input at the user interface.

12. A method for transitioning a modular ventilatory support apparatus (10) from one to another of a stationary configuration, an extended range configuration, and a stand-alone configuration, the method comprising:

providing a modular ventilatory support apparatus (10) comprising:

a compressor unit (14) comprising: a compressor (83) for providing compressed gas; a compressor unit ventilation gas output port (60); a compressor unit compressed gas output port (58); and a ventilator dock (42), the ventilator dock (42) having a ventilator dock compressed gas output port (43) and a ventilator dock ventilation gas inlet port (45);

a ventilator (12) configured for removable docking at the ventilator dock (42), the ventilator (12) comprising: a user interface; a ventilator ventilation gas output port (38); and a ventilator compressed gas inlet port (40); and

a patient interface (80) having a patient interface gas inlet port (81) transitionable between placement in fluid communication with the compressor unit ventilation gas output port (60) and placement in fluid communication with the ventilator ventilation gas output port (38); and

transitioning the modular ventilatory support apparatus (10) from one to another of a stationary configuration, an extended range configuration, and a stand-alone configuration;

wherein the modular ventilatory support apparatus (10) is transitioned to the stationary configuration when the ventilator (12) is docked at the ventilator dock (42) with the ventilator compressed gas inlet port (40) placed in fluid communication with the ventilator dock compressed gas output port (43) and with the ventilator ventilation gas output port (38) placed in fluid communication with the ventilator dock ventilation gas inlet port (45), and the patient interface gas inlet port (81) is placed in fluid communication with the compressor ventilation gas output port (60), such that, in use, compressed gas is provided by the compressor unit (14) to the ventilator (12) and ventilation gas is returned to the compressor unit (14) for subsequent output from the compressor unit (14) to the patient interface (80);

wherein the modular ventilatory support apparatus (10) is transitioned to the extended range configuration when the ventilator (12) is not docked at the ventilation dock (42), the ventilator compressed gas inlet port (40) is placed in fluid communication with the compressor unit compressed gas output port (58) via a compressed gas hose (84), and the patient interface gas inlet port (81) is placed in fluid communication with the ventilator ventilation gas output port (38), such that, in use, compressed gas is provided by the compressor unit (14) to the ventilator (12) and ventilation gas is provided by the ventilator (12) to the patient interface (80) without being returned to the compressor unit (14); and

wherein the modular ventilatory support apparatus (10) is transitioned to the stand-alone configuration when the ventilator (12) is not docked at the ventilation dock, the ventilator compressed gas inlet port (40) is placed in fluid communication with an external compressed gas source, and the patient interface gas inlet port (81) is placed in fluid communication with the ventilator ventilation gas output port (38), such that compressed gas is provided by the external compressed gas source to the ventilator (12) and ventilation gas is provided by the ventilator (12) to the patient interface (80) without passing through the compressor unit (14).

13. The method of Claim 12, wherein the compressor (83) is configured to compress ambient air.

14. The method of Claim 12, wherein the compressor unit (14) comprises a low flow gas input port.

**Patentansprüche**

1. Atemhilfevorrichtung (10), die zwischen einer feststehenden Konfiguration und einer Konfiguration mit vergrößerter Reichweite überführt werden kann, wobei die Atemhilfevorrichtung (10) umfasst:

eine Kompressoreinheit (14), wobei die Kompressoreinheit (14) umfasst:

einen Kompressor (83) zum Bereitstellen von Druckgas;
ein Beatmungsgerätdock (42) mit einem Beatmungsgerätdock-Druckgas-Ausgangsanschluss (43) und mit einem Beatmungsgerätdock-Beatmungsgas-Einlassanschluss (45);
einen Kompressoreinheits-Beatmungsgas-Ausgangsanschluss (60); und
einen Kompressoreinheits-Druckgas-Ausgangsanschluss (58);

ein Beatmungsgerät (12) zum Bereitstellen von Beatmungsgas, wobei das Beatmungsgerät (12) für das lösbare Andocken an dem Beatmungsgerätdock (42) konfiguriert ist, wobei das Beatmungsgerät umfasst:

einen Beatmungsgerät-Beatmungsgas-Ausgangsanschluss (38); und
einen Beatmungsgerät-Druckgas-Einlassanschluss (40); und

eine Patientenschnittstelle (80) zum Empfangen von Beatmungsgas und zum Zuführen von Beatmungsgas zu einem Patienten, wobei die Patientenschnittstelle (80) einen Patientenschnittstellengas-Einlassanschluss (81) aufweist, der zwischen der Anordnung in Fluidverbindung mit dem Kompressoreinheits-Beatmungsgas-Ausgangsanschluss (60) und der Anordnung in Fluidverbindung mit dem Beatmungsgerät-Beatmungsgas-Ausgangsanschluss (38) überführt werden kann;
wobei das Beatmungsgerät (12) bei dem Beatmungsgerätdock (42) angedockt ist, der Beatmungsgerät-Druckgas-Einlassanschluss (40) mit dem Beatmungsgerätdock-Druckgas-Ausgangsanschluss (43) in Fluidverbindung steht, der Beatmungsgerät-Beatmungsgas-Ausgangsanschluss (38) mit dem Beatmungsgerätdock-Beatmungsgas-Einlassanschluss (45) in Fluidverbindung steht und der Patientenschnittstellengas-Einlassanschluss (81) mit dem Kompressor-Beatmungsgas-Ausgangsanschluss (60) in Fluidverbindung steht, wenn die Atemhilfevorrichtung (10) in die feststehende Konfiguration überführt ist, so dass in Verwendung durch die Kompressoreinheit (14) Druckgas für das Beatmungsgerät (12) bereitgestellt wird und Beatmungsgas zur nachfolgenden Ausgabe von der Kompressoreinheit (14) an die Patientenschnittstelle zu der Kompressoreinheit (14) zurückgeführt wird; und
wobei das Beatmungsgerät (12) nicht bei dem Beatmungsgerätdock (42) angedockt ist, der Beatmungsgerät-Druckgas-Einlassanschluss (40) über einen Druckgasschlauch (84) mit dem Kompressoreinheits-Druckgas-Ausgangsanschluss (58) in Fluidverbindung steht und der Patientenschnittstellengas-Einlassanschluss (81) mit dem Beatmungsgerät-Beatmungsgas-Ausgangsanschluss (38) in Fluidverbindung steht, so dass in Verwendung durch die Kompressoreinheit (14) Druckgas für das Beatmungsgerät (12) bereitgestellt wird und Beatmungsgas durch das Beatmungsgerät (12) für die Patientenschnittstelle bereitgestellt wird, ohne zu der Kompressoreinheit (14) zurückgeführt zu werden, wenn die Atemhilfevorrichtung (10) in die Konfiguration mit vergrößerter Reichweite überführt ist.

2. Atemhilfevorrichtung (10) gemäß Anspruch 1, wobei die Atemhilfevorrichtung (10) ferner zwischen der feststehenden Konfiguration, der Konfiguration mit vergrößerter Reichweite und einer selbstständigen Konfiguration überführt werden kann,
wobei das Beatmungsgerät (12) nicht bei dem Beatmungsgerätdock (42) angedockt ist, der Beatmungsgerät-Druckgas-Eingangsanschluss (40) mit einer externen Druckgasquelle in Fluidverbindung steht und der Patientenschnittstellengas-Einlassanschluss (81) mit dem Beatmungsgerät-Beatmungsgas-Ausgangsanschluss (38) in Fluidverbindung steht, wenn die Atemhilfevorrichtung (10) in die selbstständige Konfiguration überführt ist, so dass in Verwendung Druckgas durch die externe Druckgasquelle für das Beatmungsgerät (12) bereitgestellt wird und Beatmungsgas durch das Beatmungsgerät (12) für die Patientenschnittstelle bereitgestellt wird, ohne durch die Kompressoreinheit (14) zu gehen.

3. Atemhilfevorrichtung (10) gemäß Anspruch 2, wobei die Kompressoreinheit (14) ferner einen Gaseingangsanschluss mit geringem Durchfluss umfasst.

4. Atemhilfevorrichtung (10) gemäß Anspruch 3, wobei der Gaseingangsanschluss mit geringem Durchfluss einen Sauerstoffeingangsanschluss mit geringem Durchfluss umfasst.

5. Atemhilfevorrichtung (10) gemäß Anspruch 1 oder 2, wobei der Kompressor (83) dafür konfiguriert ist, Umgebungsluft zu verdichten.

6. Atemhilfevorrichtung (10) gemäß Anspruch 5, wobei die Kompressoreinheit (14) ferner eine oder mehrere Umgebungsluftöffnungen zum Einleiten von Umgebungsluft in den Kompressor (83) umfasst.

7. Atemhilfevorrichtung (10) gemäß Anspruch 6, wobei die eine oder die mehreren Umgebungsluftöffnungen ferner einen Filter umfassen.

8. Atemhilfevorrichtung (10) gemäß Anspruch 1 oder 2, wobei der Kompressoreinheits-Druckgas-Ausgangsanschluss (58) eine Diameter-Index-Safety-System-Kupplung umfasst.

9. Atemhilfevorrichtung (10) gemäß Anspruch 1 oder 2, wobei das Beatmungsgerät (12) elektrisch mit Leistung versorgt wird und eine wieder aufladbare Batterie enthält.

10. Atemhilfevorrichtung (10) gemäß Anspruch 9, wobei das Beatmungsgerätdock (42) dafür konfiguriert ist, elektrische Leistung für die Leistungsversorgung des Beatmungsgeräts (12) und zum Wiederaufladen der wieder aufladbaren Batterie bereitzustellen, wenn das Beatmungsgerät (12) bei dem Beatmungsgerätdock (42) angedockt ist.

11. Atemhilfevorrichtung (10) gemäß Anspruch 1 oder 2, wobei das Beatmungsgerät (12) ferner eine Benutzerschnittstelle und einen drahtlosen Sender umfasst, wobei die Kompressoreinheit (14) ferner einen drahtlosen Empfänger umfasst und wobei der Kompressor (83) durch Signalübertragung von dem drahtlosen Sender an den drahtlosen Empfänger, die durch Benutzereingabe bei der Benutzerschnittstelle initiiert wird, steuerbar ist.

12. Verfahren zum Überführen einer modularen Atemhilfevorrichtung (10) von einer in eine andere einer feststehenden Konfiguration, einer Konfiguration mit vergrößerter Reichweite und einer selbstständigen Konfiguration, wobei das Verfahren umfasst:

Bereitstellen einer modularen Atemhilfevorrichtung (10), die umfasst:

eine Kompressoreinheit (14), die umfasst:

einen Kompressor (83) zum Bereitstellen von Druckgas;
eine Kompressoreinheits-Beatmungsgas-Ausgangsanschluss (60);
einen Kompressoreinheits-Druckgas-Ausgangsanschluss (58); und
ein Beatmungsgerätdock (42), wobei das Beatmungsgerätdock (42) einen Beatmungsgerätdock-Druckgas-Ausgangsanschluss (43) und einen Beatmungsgerätdock-Beatmungsgas-Einlassanschluss (45) aufweist;

ein Beatmungsgerät (12), das für das lösbare Andocken an dem Beatmungsgerätdock (42) konfiguriert ist, wobei das Beatmungsgerät (12) umfasst:

eine Benutzerschnittstelle;
einen Beatmungsgerät-Beatmungsgas-Ausgangsanschluss (38); und
einen Beatmungsgerät-Druckgas-Einlassanschluss (40); und

eine Patientenschnittstelle (80) mit einem Patientenschnittstellengas-Einlassanschluss (81), der zwischen der Anordnung in Fluidverbindung mit dem Kompressoreinheits-Beatmungsgas-Ausgangsanschluss (60) und der Anordnung in Fluidverbindung mit dem Beatmungsgerät-Beatmungsgas-Ausgangsanschluss (38) überführt werden kann; und

Überführen der modularen Atemhilfevorrichtung (10) von einer in eine andere einer feststehenden Konfiguration, einer Konfiguration mit vergrößerter Reichweite und einer selbstständigen Konfiguration;
wobei die modulare Atemhilfevorrichtung (10) in die feststehende Konfiguration überführt wird, wenn das Beatmungsgerät (12) bei dem Beatmungsgerätdock (42) angedockt wird, wobei der Beatmungsgerät-Druckgas-Einlassanschluss (40) in Fluidverbindung mit dem Beatmungsgerätdock-Druckgas-Ausgangsanschluss (43) angeordnet wird und wobei der Beatmungsgerät-Beatmungsgas-Ausgangsanschluss (38) in Fluidverbindung mit dem Beatmungsgerätdock-Beatmungsgas-Einlassanschluss (45) angeordnet wird und wobei der Patientenschnittstellengas-Einlassanschluss (81) in Fluidverbindung mit dem Kompressor-Beatmungsgas-Ausgangsanschluss (60) angeordnet wird, so dass in Verwendung durch die Kompressoreinheit (14) Druckgas für das Beatmungsgerät (12) bereitgestellt wird und Beatmungsgas zur nachfolgenden Ausgabe von der Kompressoreinheit (14) an die Patientenschnittstelle (80) zu der Kompressoreinheit (14) zurückgeführt wird;
wobei die modulare Atemhilfevorrichtung (10) in die Konfiguration mit vergrößerter Reichweite überführt wird, wenn das Beatmungsgerät (12) nicht bei dem Beatmungsgerätdock (42) angedockt wird, der Beatmungsgerät-Druckgas-Einlassanschluss (40) über einen Druckgasschlauch (84) in Fluidverbindung mit dem Kompressoreinheits-Druckgas-Ausgangsanschluss (58) angeordnet wird und der Patientenschnittstellengas-Einlassanschluss (81) in Fluidverbindung mit dem Beatmungsgerät-Beatmungsgas-Ausgangsanschluss (38) angeordnet wird, so dass in Verwendung durch die Kompressoreinheit (14) Druckgas für das Beatmungsgerät (12) bereitgestellt wird und Beatmungsgas durch das Beatmungsgerät (12) für die Patientenschnittstelle (80) bereitgestellt wird, ohne zu der Kompressoreinheit (14) zurückgeführt zu werden; und

wobei die modulare Atemhilfevorrichtung (10) in die selbstständige Konfiguration überführt wird, wenn das Beatmungsgerät (12) nicht bei dem Beatmungsgerätdock angedockt wird, der Beatmungsgerät-Druckgas-Eingangsanschluss (40) in Fluidverbindung mit einer externen Druckgasquelle angeordnet wird und der Patientenschnittstellengas-Einlassanschluss (81) in Fluidverbindung mit dem Beatmungsgerät-Beatmungsgas-Ausgangsanschluss (38) angeordnet wird, so dass Druckgas durch die externe Druckgasquelle für das Beatmungsgerät (12) bereitgestellt wird und Beatmungsgas durch das Beatmungsgerät (12) für die Patientenschnittstelle (80) bereitgestellt wird, ohne durch die Kompressoreinheit (14) zu gehen.

13. Verfahren gemäß Anspruch 12, wobei der Kompressor (83) zum Verdichten von Umgebungsluft konfiguriert ist.

14. Verfahren gemäß Anspruch 12, wobei die Kompressoreinheit (14) einen Gaseingangsanschluss mit geringem Durchfluss umfasst.


**Revendications**

1. Dispositif de support ventilatoire (10) qui peut passer entre une configuration fixe et une configuration à portée étendue, le dispositif de support ventilatoire (10) comprenant :
une unité de compresseur (14), l'unité de compresseur (14) comprenant :

un compresseur (83) pour fournir un gaz comprimé ;
une attache de ventilateur (42) ayant un orifice de sortie de gaz comprimé d'attache de ventilateur (43) et un orifice d'admission de gaz de ventilation d'attache de ventilateur (45) ;
un orifice de sortie de gaz de ventilation d'unité de compresseur (60) ; et
un orifice de sortie de gaz comprimé d'unité de compresseur (58) ;
un ventilateur (12) pour fournir un gaz de ventilation, le ventilateur (12) étant configuré pour une attache amovible à l'attache de ventilateur (42), le ventilateur comprenant :

un orifice de sortie de gaz de ventilation de ventilateur (38) ; et
un orifice d'admission de gaz comprimé de ventilateur (40) ; et
une interface patient (80) pour recevoir un gaz de ventilation et délivrer un gaz de ventilation à un patient, l'interface patient (80) ayant un orifice d'admission de gaz d'interface patient (81) qui peut passer entre un positionnement en communication fluidique avec l'orifice de sortie de gaz de ventilation d'unité de compresseur (60) et un positionnement en communication fluidique avec l'orifice de sortie de gaz de ventilation de ventilateur (38) ;
où, quand le dispositif de support ventilatoire (10) est passé à une configuration fixe, le ventilateur (12) est attaché à l'attache de ventilateur (42), l'orifice d'admission de gaz comprimé de ventilateur (40) est en communication fluidique avec l'orifice de sortie de gaz comprimé d'attache de ventilateur (43), l'orifice de sortie de gaz de ventilation de ventilateur (38) est en communication fluidique avec l'orifice d'admission de gaz de ventilation d'attache de ventilateur (45), et l'orifice d'admission de gaz d'interface patient (81) est en communication fluidique avec l'orifice de sortie de gaz de ventilation de compresseur (60), de manière à ce que, pendant l'utilisation,
le gaz comprimé est fourni par l'unité de compresseur (14) au ventilateur (12) et le gaz de ventilation est renvoyé à l'unité de compresseur (14) pour une sortie ultérieure de l'unité de compresseur (14) à l'interface patient ; et
où, quand le dispositif de support ventilatoire (10) est passé à la configuration à portée étendue, le ventilateur (12) n'est pas attaché à l'attache de ventilateur (42), l'orifice d'admission de gaz comprimé de ventilateur (40) est en communication fluidique avec l'orifice de sortie de gaz comprimé de l'unité de compresseur (58) à travers un tuyau de gaz comprimé (84),
et l'orifice d'admission d'interface patient (81) est en communication fluidique avec l'orifice de sortie de gaz de ventilation de ventilateur (38), de manière à ce que, pendant l'utilisation, le gaz comprimé est fourni par l'unité de compresseur (14) au ventilateur (12) et le gaz de ventilation est fourni par le ventilateur (12) à l'interface patient sans être renvoyé à l'unité de compresseur (14).

2. Dispositif de support ventilatoire (10) selon la revendication 1, où en outre le dispositif de support ventilatoire (10) peut passer entre la configuration fixe, la configuration à portée étendue, et une configuration autonome,
où, quand le dispositif de support ventilatoire (10) est passé en configuration autonome, le ventilateur (12) n'est pas attaché à l'attache de ventilateur (42), l'orifice d'admission de gaz comprimé de ventilateur (40) est en communication fluidique avec une source de gaz comprimé externe, et l'orifice d'admission de gaz d'interface patient (81) est en communication fluidique avec l'orifice de sortie de gaz de ventilation de ventilateur (38), de manière à ce que, pendant l'utilisation,
le gaz comprimé est fourni par la source de gaz comprimé externe au ventilateur (12) et le gaz de ventilation est fourni par le ventilateur (12) à l'interface patient sans passer par l'unité de compresseur (14).

3. Dispositif de support ventilatoire (10) de la revendication 2, où l'unité de compresseur (14) comprend en outre un orifice d'entrée de gaz à bas débit.

4. Dispositif de support ventilatoire (10) de la revendication 3, où l'orifice d'entrée de gaz à bas débit comprend un orifice d'entrée d'oxygène à bas débit.

5. Dispositif de support ventilatoire (10) de la revendication 1 ou 2, où le compresseur (83) est configuré pour comprimer l'air ambiant.

6. Dispositif de support ventilatoire (10) de la revendication 5, où l'unité de compresseur (14) comprend en outre une ou plusieurs ouvertures d'air ambiant pour introduire l'air ambiant dans le compresseur (83).

7. Dispositif de support ventilatoire (10) de la revendication 6, où l'une ou plusieurs ouvertures d'air ambiant comprennent en outre un filtre.

8. Dispositif de support ventilatoire (10) de la revendication 1 ou 2, où l'orifice de sortie de gaz comprimé d'unité de compresseur (58) comprend un couplage de système de sécurité à diamètres indexés (DISS).

9. Dispositif de support ventilatoire (10) de la revendication 1 ou 2, où le ventilateur (12) est électriquement alimenté et inclut une batterie rechargeable.

10. Dispositif de support ventilatoire (10) de la revendication 9, où l'attache de ventilateur (42) est configurée pour fournir l'énergie électrique pour alimenter le ventilateur (12) et pour recharger la batterie rechargeable quand le ventilateur (12) est attaché à l'attache de ventilateur (42).

11. Dispositif de support ventilatoire (10) de la revendication 1 ou 2, où le ventilateur (12) comprend en outre une interface utilisateur et un émetteur sans fil, où l'unité de compresseur (14) comprend en outre un récepteur sans fil, et où le compresseur (83) est contrôlable par une transmission de signal de l'émetteur sans fil au récepteur sans fil amorcée par une entrée utilisateur à l'interface utilisateur.

12. Procédé pour faire passer un dispositif de support ventilatoire modulaire (10) d'une configuration à une autre entre une configuration fixe, une configuration à portée étendue et une configuration autonome, le procédé comprenant :
la fourniture d'un dispositif de support ventilatoire modulaire (10) comprenant :
une unité de compresseur (14) comprenant :

un compresseur (83) pour fournir un gaz comprimé ;
un orifice de sortie de gaz de ventilation d'unité de compresseur (60) ;
un orifice de sortie de gaz comprimé d'unité de compresseur (58) ; et
une attache de ventilateur (42), l'attache de ventilateur (42) ayant un orifice de sortie de gaz comprimé d'attache de ventilateur (43) et un orifice d'admission de gaz de ventilation d'attache de ventilateur (45) ;
un ventilateur (12) configuré pour l'attache amovible à l'attache de ventilateur (42), le ventilateur (12) comprenant : une interface utilisateur ;
un orifice de sortie de gaz de ventilation de ventilateur (38) ;
et un orifice d'admission de gaz comprimé de ventilateur (40) ; et
une interface patient (80) ayant un orifice d'admission de gaz d'interface patient (81) qui peut passer entre un positionnement en communication fluidique avec l'orifice de sortie de gaz de ventilation d'unité de compresseur (60) et un positionnement en communication fluidique avec l'orifice de sortie de gaz de ventilation de ventilateur (38) ; et
le passage du dispositif de support ventilatoire modulaire (10) d'une configuration à une autre entre une configuration fixe, une configuration à portée étendue et une configuration autonome ;

où le dispositif de support ventilatoire modulaire (10) est passé à la configuration fixe quand le ventilateur (12) est attaché à l'attache de ventilateur (42) avec l'orifice d'admission de gaz comprimé de ventilateur (40) positionné en communication fluidique avec l'orifice de sortie de gaz comprimé d'attache de ventilateur (43) et avec l'orifice de sortie de gaz de ventilation de ventilateur (38) positionné en communication fluidique avec l'orifice d'admission de gaz de ventilation d'attache de ventilateur (45) et l'orifice d'admission de gaz d'interface patient (81) est positionné en communication fluidique avec l'orifice de sortie de gaz de ventilation de compresseur (60), de manière à ce que, pendant l'utilisation,

le gaz comprimé est fourni par l'unité de compresseur (14) au ventilateur (12) et le gaz de ventilation est renvoyé à l'unité de compresseur (14) pour une sortie ultérieure de l'unité de compresseur (14) à l'interface patient (80) ;

où le dispositif de support ventilatoire modulaire (10) est passé à la configuration à portée étendue quand le ventilateur (12) n'est pas attaché à l'attache de ventilation (42), l'orifice d'admission de gaz comprimé de ventilateur (40) est positionné en communication fluidique avec l'orifice de sortie de gaz comprimé d'unité de compresseur (58) à travers un tuyau de gaz comprimé (84),

et l'orifice d'admission de gaz d'interface patient (81) est positionné en communication fluidique avec l'orifice de sortie de gaz de ventilation de ventilateur (38), de manière à ce que, pendant l'utilisation,

le gaz comprimé est fourni par l'unité de compresseur (14) au ventilateur (12) et le gaz de ventilation est fourni par le ventilateur (12) à l'interface patient (80) sans être renvoyé à l'unité de compresseur (14) ; et

où le dispositif de support ventilatoire modulaire (10) est passé à la configuration autonome quand le ventilateur (12) n'est pas attaché à l'attache de ventilation, l'orifice d'admission de gaz comprimé de ventilateur (40) est positionné en communication fluidique avec une source de gaz comprimé externe, et l'orifice d'admission de gaz d'interface patient (81) est positionné en communication fluidique avec l'orifice de sortie de gaz de ventilation de ventilateur (38), de manière à ce que le gaz comprimé est fourni par la source de gaz comprimé externe au ventilateur (12) et le gaz de ventilation est fourni par le ventilateur (12) à l'interface patient (80) sans passer à travers l'unité de compresseur (14).

13. Procédé de la revendication 12, où le compresseur (83) est configuré pour comprimer l'air ambiant.

14. Procédé de la revendication 12, où l'unité de compresseur (14) comprend un orifice d'entrée de gaz à bas débit.

**FIG.1**

**FIG. 2**

**FIG. 3**

**FIG. 4**

**FIG. 5**

**FIG. 6**

**FIG. 7**

**FIG. 8**

**FIG. 9A**

**FIG. 9B**

**FIG. 9C**

**FIG. 9D**

**FIG. 10**

**FIG. 11**

**FIG. 12**

**FIG. 13A**

**FIG. 13B**

**FIG. 14**

**FIG. 15**

**FIG. 16**

FIG. 17A

FIG. 17B

FIG. 18

**FIG. 19A**

Pole Mount

94

**FIG. 19B**

92

94

96

98

**FIG. 19C**

30

12

92

94

**FIG. 20A**

100

102

**FIG. 20B**

102

84

100

12

40

84

**FIG. 20C**

Fig. 21

Fig. 22

Fig. 23

Fig. 24                                    Fig. 25

Fig. 26

Fig. 27                                    Fig. 28

Fig. 29                              Fig. 30

Fig.31

Fig. 32

Fig. 33

Fig. 34

Fig. 35                    Fig. 36                    Fig. 37

| BATTERY CHARGE SCALE | | | | | | |
|---|---|---|---|---|---|---|
| APPROX. CHARGE AMOUNT | 0% | 1–20%* | 21–50% | 51–70% | 71–90% | 91–100% |

Fig. 38

| BATTERY CHARGE SCALE | | | | | | |
|---|---|---|---|---|---|---|
| APPROX. CHARGE AMOUNT | 0–20% | 21–50% | 51–70% | 71–90% | 91–99% | 100% |

Fig. 39

Fig. 40                    Fig. 41

Fig. 42

Fig. 43

Fig. 44

Fig. 45

Fig. 46

Fig. 47

Fig. 48

EP 3 341 062 B1

Fig. 49

Fig. 50

Fig. 51

| BATTERY CHARGE SCALE | | | | | | |
|---|---|---|---|---|---|---|
| APPROX. CHARGE AMOUNT | 0% | 1–20%* | 21–50% | 51–70% | 71–90% | 91–100% |

Fig. 52

| BATTERY CHARGE SCALE | | | | | | |
|---|---|---|---|---|---|---|
| APPROX. CHARGE AMOUNT | 0–20% | 21–50% | 51–70% | 71–90% | 91–99% | 100% |

Fig. 53

EP 3 341 062 B1

Fig. 54

| BATTERY CHARGE ICON | | | | | | |
|---|---|---|---|---|---|---|
| APPROX. CHARGE AMOUNT | Charging | < 5% | < 15% | 15–35% | 36–56% | 57–79% | 80–100% |
| APPROX. TIME REMAINING | N/A | Critically low. Recharge immediately. | Less than 0.5 hour.* Recharge immediately. | 0.5–1.5 hours* | 1.5–2.5 hours | 2.5–3 hours | 3–4 hours |

Fig. 55

Fig. 56

Fig. 57

123

Fig. 58

Fig. 59

Fig. 60

Fig. 61

Fig. 62

Fig. 63

Fig. 64

Fig. 65

| BATTERY CHARGE ICON | | | | | | | |
|---|---|---|---|---|---|---|---|
| APPROX. CHARGE AMOUNT | Charging | < 5% | <15% | 15–35% | 36–56% | 57–79% | 80–100% |
| APPROX. TIME REMAINING | N/A | Critically low. Recharge immediately. | Less than 0.5 hour.* Recharge immediately. | 0.5–1.5 hours* | 1.5–2.5 hours | 2.5–3 hours | 3–4 hours |

Fig. 66

Fig. 67

Fig. 68

Fig. 69

Fig. 70

Fig. 71

Fig. 72

Fig. 73

Fig. 74

Fig. 75

Fig. 76

Fig. 77

Fig. 78

Fig. 79

Fig. 80

Fig. 81

Fig. 82

Fig. 83

Fig. 84

Fig. 85

Fig. 86

Fig. 87

Fig. 88

Fig. 89

Fig. 90

Fig. 91

Fig. 92

Fig. 93

Fig. 94

Fig. 95

Fig. 96

Fig. 97

Fig. 98

Fig. 99

Fig. 100

Fig. 101

Fig. 102

Fig. 103

Fig. 104

Fig. 105

Life2000™

Delta P [P2]
(0-5 psi)

804   805   806   813

Pms [P3]
(0-50 psi)

Ambient

818

802   803

808

807   812   814

817

809

801

816

810

Air

815   Sense Line [P4]
(+/- 40 cmH2O)

811

O2
Low flow
(0-10 LPM)

Ambient

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 105169537 A
- US 5335651 A1
- US 6158430 A
- US 6095138 A
- US 7533670 B
- US 8381729 B
- US 8418694 B
- US 8567399 B
- US 8770193 B
- US 8776793 B
- US 8895108 B
- US 9399109 B
- US 524983
- US 20130333702
- US 566902
- US 20140034055
- US 841189
- US 20140261426
- US 849443
- US 20140283834
- US 927016
- US 20140373842
- US 935362
- US 20150011905
- US 020729
- US 20150073291
- US 104842
- US 20150165143
- US 181431
- US 20150231349
- US 181435
- US 20150231350
- US 482444
- US 20150068528
- US 482445
- US 20150068529
- US 020032
- US 20150068519
- US 8839791 B
- US 8844533 B
- US 9038634 B
- US 9038635 B
- US 9132250 B
- US 9180270 B
- US 9227034 B
- US 9327092 B